(19) Europäisches Patentamt
European Patent Office
Office européen des brevets

(11) EP 4 454 662 A1

(12) EUROPEAN PATENT APPLICATION
published in accordance with Art. 153(4) EPC

(43) Date of publication:
**30.10.2024 Bulletin 2024/44**

(21) Application number: **22910172.0**

(22) Date of filing: **23.12.2022**

(51) International Patent Classification (IPC):
***A61K 39/395*** *(2006.01)* ***C07K 16/28*** *(2006.01)*

(52) Cooperative Patent Classification (CPC):
**A61K 47/6857; A61K 47/6803;** A61K 2039/505; C07K 16/28; C07K 2317/24; C07K 2317/33; C07K 2317/565; C07K 2317/77; C07K 2317/92

(86) International application number:
**PCT/CN2022/141269**

(87) International publication number:
**WO 2023/116861 (29.06.2023 Gazette 2023/26)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **23.12.2021 CN 202111592539**

(71) Applicants:
- **Jiangsu Hengrui Pharmaceuticals Co., Ltd.**
**Lianyungang, Jiangsu 222047 (CN)**
- **Shanghai Hengrui Pharmaceutical Co., Ltd.**
**Shanghai 200245 (CN)**

(72) Inventors:
- **YE, Xin**
**Shanghai 200245 (CN)**
- **YAO, Qingqing**
**Shanghai 200245 (CN)**
- **JIN, Xinsheng**
**Shanghai 200245 (CN)**
- **YING, Hua**
**Shanghai 200245 (CN)**
- **TAO, Weikang**
**Shanghai 200245 (CN)**

(74) Representative: **Dragotti & Associati S.R.L.**
**Via Nino Bixio, 7**
**20129 Milano (IT)**

Remarks:
The complete document including Reference Table(s) and the Sequence Listing(s) can be downloaded from the EPO website

# (54) ANTI-DLL3 ANTIBODY AND PHARMACEUTICAL USE THEREOF, AND ANTIBODY-DRUG CONJUGATE CONTAINING ANTI-DLL3 ANTIBODY

(57) An anti-DLL3 antibody and a pharmaceutical use thereof, and an antibody-drug conjugate containing the anti-DLL3 antibody. The present disclosure relates to an anti-DLL3 antibody-ecteinascidin drug conjugate represented by general formula (Pc-L-D), wherein Pc is the anti-DLL3 antibody.

EP 4 454 662 A1

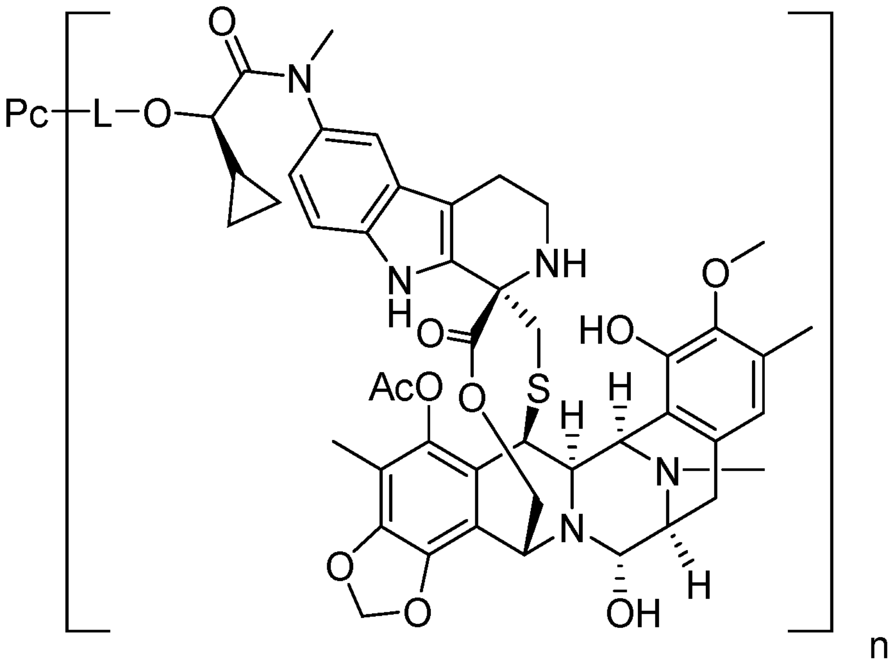

Pc-L-D

## Description

The present application claims priority to the Chinese Patent Application (CN202111592539.7) filed on Dec. 23, 2021.

## TECHNICAL FIELD

The present disclosure relates to an anti-DLL3 antibody, a drug conjugate thereof, and a preparation method therefor. Also, the anti-DLL3 antibody or the ADC thereof described in the present disclosure may be used in the preparation of a medicament for treating a disease or disorder.

## BACKGROUND

The statements herein merely provide background information related to the present disclosure and may not necessarily constitute the prior art.

Small cell lung cancer (SCLC) is a relatively malignant type of lung cancer, accounting for 10%-15% of all lung cancer cases. Tumors of small cell lung cancer grow fast and easily metastasize, with a five-year survival rate below 7%. Platinum/etoposide combination chemotherapy is often used to treat small cell lung cancer. Patients with small cell lung cancer initially respond well to chemotherapy but are very likely to develop resistance and relapse. In recent years, immunotherapies such as PD-L1 antibodies and PD1 antibodies have shown some efficacy in patients with small cell lung cancer, with an effective rate of about 15%. There are still no specific targeted therapy drugs developed for this disease.

DLL3 is a ligand that inhibits Notch. Under normal conditions, DLL3 is located on the Golgi apparatus, but in cancer cells (such as cells of small cell lung cancer), DLL3 can move to the cell surface and bind to Notch in a cis manner, impeding cell-to-cell binding and the endocytosis of Notch in target cells, thereby inhibiting the Notch signaling pathway and promoting tumor cell growth. DLL3 is primarily expressed in nerve or neuroendocrine tumors, including SCLC, large cell neuroendocrine cancer, gastrointestinal neuroendocrine tumors, small cell bladder cancer, glioblastoma multiforme, metastatic castration prostate cancer, melanoma, and the like. Particularly, over 80% of SCLC cases involve positive DLL3 expression, while it is not expressed in normal lung tissue or paracancerous tissue. This differential expression makes DLL3 a very potential therapeutic target for SCLC treatment.

Ecteinascidin derivatives, such as lurbinectedin, are inhibitors of RNA polymerase II that bind covalently to the minor groove of DNA double helices, selectively inhibiting the transcription process mediated by RNA polymerase II activated in a trans manner, while affecting neither RNA polymerase I or mitochondrial RNA polymerase activity nor the normal mRNA transcription process. RNA polymerase II is often over-activated in the transcription process of tumor cells. Lurbinectedin can cause abnormality and apoptosis in the mitosis process of tumor cells, eventually reducing cell proliferation. Tumor cells rely on a rapid transcription process for proliferation. These tumor cells are particularly sensitive to lurbinectedin, and they include SCLC, BRCA1/2 mutant breast cancer, platinum-resistant ovarian cancer, and sarcomas caused by chromosomal translocations and the like.

Antibody-drug conjugates (ADCs) are formed by linking monoclonal antibodies or antibody fragments to biologically active drugs by linkers. They fully combine the specificity of antibodies in binding to antigens on the surfaces of normal cells and tumor cells and the high efficiency of drugs (such as cytotoxic agents), while avoiding such shortcomings as the low efficacy of antibodies and the serious toxic and side effects of drugs. Antibody-drug conjugates can kill tumor cells more precisely and have less effect on normal cells than conventional chemotherapeutic drugs.

## SUMMARY

The present disclosure relates to an anti-DLL3 antibody, an ADC thereof, and pharmaceutical use thereof. More specifically, the present disclosure provides a variety of anti-DLL3 antibodies with brand-new sequences, as well as ADCs thereof formed by conjugation to cytotoxic substances (e.g., ecteinascidin derivatives).

In some embodiments, the present disclosure provides an anti-DLL3 antibody comprising a heavy chain variable region and a light chain variable region, wherein:

i) the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 57; and the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27, wherein SEQ ID NO: 57 is represented by PLYX$_1$YGRSYNX$_2$VAY, wherein X$_1$ is Y or H; X$_2$ is A or G; or

ii) the heavy chain variable region comprises: a HCDR1 comprising the amino acid sequence of SEQ ID NO: 16; a HCDR2 comprising the amino acid sequence of SEQ ID NO: 17; and a HCDR3 comprising the amino acid sequence of SEQ ID NO: 18; and the light chain variable region comprises: a LCDR1 comprising the amino acid sequence of SEQ ID NO: 19; a LCDR2 comprising the amino acid sequence of SEQ ID NO: 20; and a LCDR3 comprising the amino acid sequence of SEQ ID NO: 21.

In some embodiments, in the aforementioned anti-DLL3 antibody:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 24, 30, or 31; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27.

In some embodiments, in the aforementioned anti-DLL3 antibody:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 24; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27.

In some embodiments, in the aforementioned anti-DLL3 antibody:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 30; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27.

In some embodiments, in the aforementioned anti-DLL3 antibody:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 31; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27.

In some embodiments, in the aforementioned anti-DLL3 antibody:

i) the heavy chain variable region comprises a HCDR1, a HCDR2, and a HCDR3 set forth in SEQ ID NO: 22, SEQ ID NO: 23, and SEQ ID NO: 57, respectively, and the light chain variable region comprises a LCDR1, a LCDR2, and a LCDR3 set forth in SEQ ID NO: 25, SEQ ID NO: 26, and SEQ ID NO: 27, respectively;
wherein, SEQ ID NO: 57 is represented by PLYX$_1$YGRSYNX$_2$VAY, wherein X$_1$ is Y or H; X$_2$ is A or G; or
ii) the heavy chain variable region comprises a HCDR1, a HCDR2, and a HCDR3 set forth in SEQ ID NO: 16, SEQ ID NO: 17, and SEQ ID NO: 18, respectively, and the light chain variable region comprises a LCDR1, a LCDR2, and a LCDR3 set forth in SEQ ID NO: 19, SEQ ID NO: 20, and SEQ ID NO: 21, respectively. In some embodiments, the aforementioned anti-DLL3 antibody is a murine antibody, a chimeric antibody, or a humanized antibody. In some embodiments, the aforementioned anti-DLL3 antibody is preferably a humanized antibody.

In some embodiments, the aforementioned anti-DLL3 antibody comprises human immunoglobulin framework regions (FRs).
In one embodiment, in the aforementioned anti-DLL3 antibody:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 57, wherein SEQ ID NO: 57 is represented by PLY$X_1$YGRSYN$X_2$VAY, wherein $X_1$ is Y or H; $X_2$ is A or G; and the FRs of the heavy chain variable region comprise one or more back mutations selected from the group consisting of 1E, 49A, and 94S; and

the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27; and

the FRs of the light chain variable region comprise a 43I back mutation; the back mutation sites described above are in accordance with the Kabat numbering scheme.

In one embodiment, the anti-DLL3 antibody comprises a heavy chain variable region and a light chain variable region, wherein:

the heavy chain variable region comprises: a HCDR1 comprising the amino acid sequence of SEQ ID NO: 16; a HCDR2 comprising the amino acid sequence of SEQ ID NO: 17; and a HCDR3 comprising the amino acid sequence of SEQ ID NO: 18; and

the FRs of the heavy chain variable region comprise one or more back mutations selected from the group consisting of 1E, 27Y, 30T, 38K, 43K, 48I, 67A, 68A, 69L, 71V, 73K, 75S, 76N, and 93A; and

the light chain variable region comprises: a LCDR1 comprising the amino acid sequence of SEQ ID NO: 19; a LCDR2 comprising the amino acid sequence of SEQ ID NO: 20; and a LCDR3 comprising the amino acid sequence of SEQ ID NO: 21; and

the FRs of the light chain variable region comprise one or more back mutations selected from the group consisting of 36L, 43S, 44F, 46G, 69A, 71Y, and 85D. The back mutation sites described above are in accordance with the Kabat numbering scheme.

In one embodiment, the anti-DLL3 antibody comprises a heavy chain variable region and a light chain variable region, wherein:

the heavy chain variable region comprises: a HCDR1 comprising the amino acid sequence of SEQ ID NO: 16; a HCDR2 comprising the amino acid sequence of SEQ ID NO: 17; and a HCDR3 comprising the amino acid sequence of SEQ ID NO: 18; and the FRs of the heavy chain variable region comprise 1E, 68A, 69L, 71V, 73K, 75S, and 76N back mutations; and

the light chain variable region comprises: a LCDR1 comprising the amino acid sequence of SEQ ID NO: 19; a LCDR2 comprising the amino acid sequence of SEQ ID NO: 20; and a LCDR3 comprising the amino acid sequence of SEQ ID NO: 21; and the FRs of the light chain variable region comprise 36L, 46G, 69A, 71Y, and 85D back mutations, and the back mutation sites described above are in accordance with the Kabat numbering scheme.

In one specific embodiment, the anti-DLL3 antibody comprises a heavy chain variable region and a light chain variable region, wherein:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 24; and the FRs of the heavy chain variable region comprise 1E and 94S back mutations; and

the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27, and

the back mutation sites described above are in accordance with the Kabat numbering scheme.

In one embodiment, the anti-DLL3 antibody comprises a heavy chain variable region and a light chain variable region, wherein:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 24, 30,

or 31; and the FRs of the heavy chain variable region comprise one or more back mutations selected from the group consisting of 1E, 49A, and 94S; and

the light chain variable region comprises: a LCDR1 comprising the amino acid sequence of SEQ ID NO: 25; a LCDR2

comprising the amino acid sequence of SEQ ID NO: 26; and a LCDR3 comprising the amino acid sequence of SEQ ID NO: 27; and
the FRs of the light chain variable region comprise a 43I back mutation. The back mutation sites described above are in accordance with the Kabat numbering scheme.

In one embodiment, the anti-DLL3 antibody comprises a heavy chain variable region and a light chain variable region, wherein:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 24; and
the FRs of the heavy chain variable region comprise one or more back mutations selected from the group consisting of 1E, 49A, and 94S; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27; and
the FRs of the light chain variable region comprise a 43I back mutation. The back mutation sites described above are in accordance with the Kabat numbering scheme.

In one embodiment, the anti-DLL3 antibody comprises a heavy chain variable region and a light chain variable region, wherein:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 30; and the FRs of the heavy chain variable region comprise one or more back mutations selected from the group consisting of 1E, 49A, and 94S; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27; and
the FRs of the light chain variable region comprise a 43I back mutation. The back mutation sites described above are in accordance with the Kabat numbering scheme.

In one embodiment, the anti-DLL3 antibody comprises a heavy chain variable region and a light chain variable region, wherein:

the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 31; and
the FRs of the heavy chain variable region comprise one or more back mutations selected from the group consisting of 1E, 49A, and 94S; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27; and
the FRs of the light chain variable region comprise a 43I back mutation. The back mutation sites described above are in accordance with the Kabat numbering scheme.

In some embodiments, in the aforementioned anti-DLL3 antibody:

i) the heavy chain variable region comprises a sequence having at least 90%, 91%, 92%, 93%, 94%, 95%, 96%, 97%, 98%, or 99% identity to SEQ ID NO: 14, 50, 51, 52, 53, or 54; and/or
the light chain variable region comprises a light chain variable region having at least 90%, 91%, 92%, 93%, 94%, 95%, 96%, 97%, 98%, or 99% identity to SEQ ID NO: 15, 55, or 56; or
ii) the heavy chain variable region comprises a sequence having at least 90%, 95%, 96%, 97%, 98%, or 99% identity to SEQ ID NO: 12, 32, 33, 34, 35, 36, 37, 38, 39, 40, 41, 42, 43, or 44; and/or
the light chain variable region comprises a light chain variable region having at least 90%, 91%, 92%, 93%, 94%, 95%, 96%, 97%, 98%, or 99% identity to SEQ ID NO: 13, 45, 46, 47, 48, or 49.

In some embodiments, in the aforementioned anti-DLL3 antibody:

i) the heavy chain variable region comprises a sequence having at least 90%, 91%, 92%, 93%, 94%, 95%, 96%, 97%, 98%, or 99% identity to SEQ ID NO: 14; and/or
the light chain variable region comprises a light chain variable region having at least 90%, 91%, 92%, 93%, 94%, 95%, 96%, 97%, 98%, or 99% identity to SEQ ID NO: 15; or
ii) the heavy chain variable region comprises a sequence having at least 90%, 91%, 92%, 93%, 94%, 95%, 96%, 97%, 98%, or 99% identity to SEQ ID NO: 50, 51, 52, 53, or 54; and/or
the light chain variable region comprises a sequence having at least 90%, 91%, 92%, 93%, 94%, 95%, 96%, 97%, 98%, or 99% identity to SEQ ID NO: 55 or 56; or
iii) the heavy chain variable region comprises a sequence having at least 90%, 91%, 92%, 93%, 94%, 95%, 96%, 97%, 98%, or 99% identity to SEQ ID NO: 12; and/or
the light chain variable region comprises a sequence having at least 90%, 91%, 92%, 93%, 94%, 95%, 96%, 97%, 98%, or 99% identity to SEQ ID NO: 13; or
iv) the heavy chain variable region comprises a sequence having at least 90%, 91%, 92%, 93%, 94%, 95%, 96%, 97%, 98%, or 99% identity to SEQ ID NO: 32, 33, 34, 35, 36, 37, 38, 39, 40, 41, 42, 43, or 44; and/or
the light chain variable region comprises a sequence having at least 90%, 91%, 92%, 93%, 94%, 95%, 96%, 97%, 98%, or 99% identity to SEQ ID NO: 45, 46, 47, 48, or 49.

In some embodiments, in the aforementioned anti-DLL3 antibody:

i) the heavy chain variable region comprises a sequence having at least 90%, 91%, 92%, 93%, 94%, 95%, 96%, 97%, 98%, or 99% identity to SEQ ID NO: 50; and/or
the light chain variable region comprises a sequence having at least 90%, 91%, 92%, 93%, 94%, 95%, 96%, 97%, 98%, or 99% identity to SEQ ID NO: 55; or
ii) the heavy chain variable region comprises a sequence having at least 90%, 91%, 92%, 93%, 94%, 95%, 96%, 97%, 98%, or 99% identity to SEQ ID NO: 43; and/or
the light chain variable region comprises a sequence having at least 90%, 91%, 92%, 93%, 94%, 95%, 96%, 97%, 98%, or 99% identity to SEQ ID NO: 48.

In some embodiments, in the aforementioned anti-DLL3 antibody:

i) the heavy chain variable region comprises the amino acid sequence of SEQ ID NO: 14, and/or the light chain variable region comprises the amino acid sequence of SEQ ID NO: 15; or
ii) the heavy chain variable region comprises an amino acid sequence selected from the group consisting of any one of SEQ ID NOs: 50, 51, 52, 53, and 54, and/or the light chain variable region comprises the amino acid sequence of 55 or 56; or
iii) the heavy chain variable region comprises the amino acid sequence of SEQ ID NO: 12, and/or the light chain variable region comprises the amino acid sequence of SEQ ID NO: 13; or
iv) the heavy chain variable region comprises an amino acid sequence selected from the group consisting of any one of SEQ ID NOs: 43, 32, 33, 34, 35, 36, 37, 38, 39, 40, 41, 42, and 44, and/or the light chain variable region comprises an amino acid sequence selected from the group consisting of any one of SEQ ID NOs: 48, 45, 46, 47, and 49.

In some embodiments, in the aforementioned anti-DLL3 antibody:

the heavy chain variable region comprises the amino acid sequence of SEQ ID NO: 50, and the light chain variable region comprises the amino acid sequence of SEQ ID NO: 55;
the heavy chain variable region comprises the amino acid sequence of SEQ ID NO: 43, and the light chain variable region comprises the amino acid sequence of SEQ ID NO: 48.

In some embodiments, the aforementioned anti-DLL3 antibody is an antibody fragment, wherein the antibody fragment is a Fab, Fab', $F(ab')_2$, Fab'-SH, Fd, Fv, scFv, dsFv, diabody, or domain antibody.
In some embodiments, the aforementioned anti-DLL3 antibody comprises a light chain constant region and a heavy chain constant region.
In some embodiments, the aforementioned anti-DLL3 antibody comprises a constant region of IgG1, IgG2, IgG3, or IgG4.
In some embodiments, the aforementioned anti-DLL3 antibody comprises a constant region of a $\lambda$ chain or a $\kappa$ chain.
In some embodiments, the aforementioned anti-DLL3 antibody comprises the heavy chain constant region of

IgG1 and the light chain constant region of a κ chain.

In some embodiments, in the aforementioned anti-DLL3 antibody, the heavy chain constant region comprises the sequence of SEQ ID NO: 28, and/or the light chain constant region comprises the sequence of SEQ ID NO: 29.

In some embodiments, the aforementioned anti-DLL3 antibody comprises a heavy chain and a light chain, wherein:

the heavy chain comprises a sequence having at least 85%, 86%, 87%, 88%, 89%, 90%, 91%, 92%, 93%, 94%, 95%, 96%, 97%, 98%, or 99% identity to SEQ ID NO: 60, and/or the light chain comprises a sequence having at least 85%, 86%, 87%, 88%, 89%, 90%, 91%, 92%, 93%, 94%, 95%, 96%, 97%, 98%, or 99% identity to SEQ ID NO: 61; or

the heavy chain comprises a sequence having at least 85%, 86%, 87%, 88%, 89%, 90%, 91%, 92%, 93%, 94%, 95%, 96%, 97%, 98%, or 99% identity to SEQ ID NO: 58, and/or the light chain comprises a sequence having at least 85%, 86%, 87%, 88%, 89%, 90%, 91%, 92%, 93%, 94%, 95%, 96%, 97%, 98%, or 99% identity to SEQ ID NO: 59.

In some embodiments, in the aforementioned anti-DLL3 antibody, the heavy chain comprises the sequence set forth in SEQ ID NO: 60, and/or the light chain comprises the sequence set forth in SEQ ID NO: 61; or
the heavy chain comprises the sequence set forth in SEQ ID NO: 58, and/or the light chain comprises the sequence set forth in SEQ ID NO: 59.

In another aspect, the present disclosure provides an isolated anti-DLL3 antibody that competes for binding to DLL3 or an epitope thereof with the antibody according to any one of the foregoing.

In some embodiments, the aforementioned anti-DLL3 antibody has at least one of the following properties:

a) the anti-DLL3 antibody binding to human DLL3 or an epitope thereof with a KD value of ≤3 nM, ≤2 nM, ≤1 nM, ≤0.9 nM, ≤0.8 nM, ≤0.7 nM, ≤0.6 nM, ≤0.5 nM, or ≤0.4 nM, as determined by Biacore;

b) the anti-DLL3 antibody binding to DLL3-expressing H1184 cells with an EC50 of ≤3 nM, an EC50 of ≤2 nM, an EC50 of ≤1 nM, an EC50 of ≤0.5 nM, an EC50 of ≤0.2 nM, an EC50 of ≤0.1 nM, an EC50 of ≤0.09 nM, an EC50 of ≤0.08 nM, an EC50 of ≤0.07 nM, an EC50 of ≤0.06 nM, as determined by FACS; and

c) the anti-DLL3 antibody being capable of being endocytosed by a DLL3-expressing cell; and

d) the anti-DLL3 antibody binding to DLL3 or an epitope thereof with an EC50 of ≤0.1 nM (≤0.09 nM, ≤0.08 nM, ≤0.07 nM, ≤0.06 nM, ≤0.05 nM, or ≤0.04 nM), as determined by ELISA; and

e) the anti-DLL3 antibody recognizing a different DLL3 epitope than a positive antibody (e.g., BI-764532) does.

In another aspect, the present disclosure provides an isolated nucleic acid encoding the anti-DLL3 antibody according to any one of the foregoing.

In another aspect, the present disclosure provides a vector comprising the aforementioned isolated nucleic acid.

In yet another aspect, the present disclosure provides a host cell comprising the aforementioned vector or expressing the anti-DLL3 antibody according to any one of the foregoing.

In one aspect, the present disclosure provides a method for preparing an antibody that binds to DLL3, comprising culturing the aforementioned host cell under conditions suitable for expression of the antibody.

In yet another aspect, the present disclosure provides an antibody-drug conjugate or a pharmaceutically acceptable salt thereof, comprising the anti-DLL3 antibody according to any one of the foregoing.

In yet another aspect, the present disclosure provides an antibody-drug conjugate or a pharmaceutically acceptable salt thereof, comprising the anti-DLL3 antibody according to any one of the foregoing and a drug, wherein the drug is conjugated to the anti-DLL3 antibody.

In some embodiments, in the aforementioned antibody-drug conjugate or the pharmaceutically acceptable salt thereof, the drug is selected from the group consisting of one or more of a cytotoxic agent, a radiolabel, a fluorophore, a chromophore, an imaging agent, an immunomodulator, an inhibitor of angiogenesis, an inhibitor of cell proliferation, a pro-apoptotic agent, and a lytic enzyme.

In some embodiments, the drug is ecteinascidin Et-743 (trabectedin) or a derivative thereof. In some embodiments, the drug is an ecteinascidin derivative, such as lurbinectedin.

In some embodiments, the aforementioned antibody-drug conjugate or the pharmaceutically acceptable salt thereof has a structure represented by general formula (Pc-L-Da):

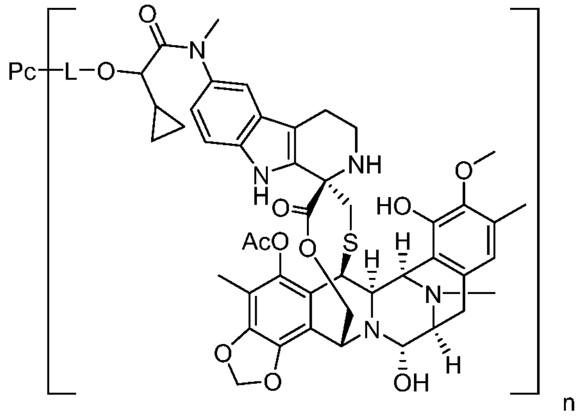

Pc-L-Da

wherein: Pc is the anti-DLL3 antibody according to any one of the foregoing; L is a linker; n is 1 to 10 (including integers and decimals); preferably, n is 3 to 5.

In some embodiments, the aforementioned antibody-drug conjugate or the pharmaceutically acceptable salt thereof is an antibody-drug conjugate represented by general formula (Pc-L-D) or a pharmaceutically acceptable salt thereof:

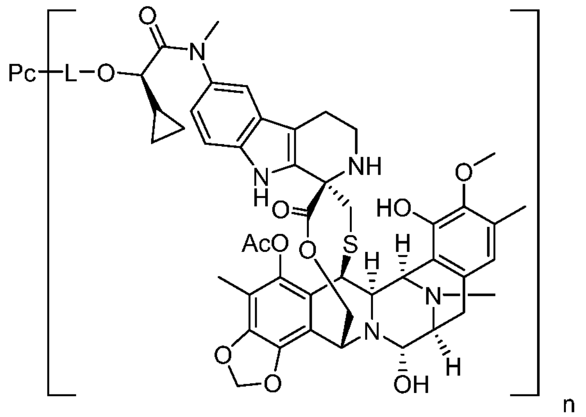

Pc-L-D

wherein: Pc is the anti-DLL3 antibody according to any one of the foregoing; L is a linker; n is 1 to 10; preferably, n is 3 to 5.

In some embodiments, in the aforementioned antibody-drug conjugate represented by general formula (Pc-L-Da) or (Pc-L-D) or the pharmaceutically acceptable salt thereof, n is the average number of drug moieties per antibody, which may be an integer or a decimal. In some embodiments, n is an average of 1-10, or 1-9, or 2-10, or 2-9, or 1-8, or 2-8, or 2-7, or 2-4, or 1-4, or 1-3, or 3-8, or 3-7, or 3-6, or 3-5, or 4-7, or 4-6, or 4-5; in some embodiments, n is 1, 2, 3, 4, 5, 6, 7, 8, 9, or 10.

In some embodiments, in the antibody-drug conjugate represented by general formula (Pc-L-Da) or (Pc-L-D) or the pharmaceutically acceptable salt thereof according to any one of the foregoing, the linker -L- is $-L^1-L^2-L^3-L^4-$, wherein:

$L^1$ is selected from the group consisting of -(succinimid-3-yl-N)-W-C(O)-, $-CH_2-C(O)-NR^3-W-C(O)-$, and -C(O)-W-C(O)-, wherein W is selected from the group consisting of $C_{1-6}$ alkylene and $C_{1-6}$ alkylene-$C_{3-6}$ cycloalkyl, wherein the $C_{1-6}$ alkylene and $C_{1-6}$ alkylene-$C_{3-6}$ cycloalkyl are each independently optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)_pCH_2CH_2C(O)-$, $-NR^4(CH_2CH_2O)_pCH_2C(O)-$, $-S(CH_2)_pC(O)-$, and a chemical bond, wherein p is an integer from 1 to 20;

$L^3$ is a peptide residue consisting of 2 to 7 amino acid residues, wherein the amino acids are selected from the group consisting of phenylalanine (F), glycine (G), valine (V), lysine (K), citrulline, serine (S), glutamic acid (E), and aspartic acid (D), and are optionally further substituted with one or more substituents selected from the group

consisting of halogen, hydroxy, cyano, amino, alkyl, chloroalkyl, deuterated alkyl, alkoxy, and cycloalkyl;

$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;

$R^3$, $R^4$, and $R^5$ are identical or different and are each independently selected from the group consisting of a hydrogen atom, alkyl, haloalkyl, deuterated alkyl, and hydroxyalkyl;

$R^6$ and $R^7$ are identical or different and are each independently selected from the group consisting of a hydrogen atom, halogen, alkyl, haloalkyl, deuterated alkyl, and hydroxyalkyl.

In some embodiments, in the antibody-drug conjugate represented by general formula (Pc-L-Da) or (Pc-L-D) or the pharmaceutically acceptable salt thereof according to any one of the foregoing, the linker -L- is $-L^1-L^2-L^3-L^4-$, wherein:

$L^1$ is

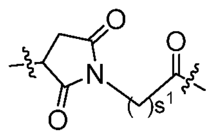

,

and $s^1$ is an integer from 2 to 8, including, but not limited to, 2, 3, 4, 5, 6, 7, and 8;

$L^2$ is a chemical bond;

$L^3$ is a tetrapeptide residue; preferably, $L^3$ is a tetrapeptide residue comprising glycine-glycine-phenylalanine-glycine;

$L^4$ is $-NH(CH_2)t-$, and t is 1 or 2;

wherein the $L^1$ end is attached to Pc.

In some embodiments, in the antibody-drug conjugate represented by general formula (Pc-L-Da) or (Pc-L-D) or the pharmaceutically acceptable salt thereof according to any one of the foregoing, -L- is:

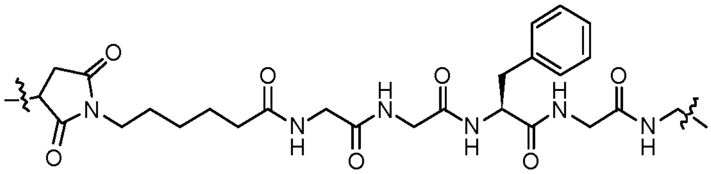

.

In some embodiments, in the antibody-drug conjugate represented by general formula (Pc-L-Da) or (Pc-L-D) or the pharmaceutically acceptable salt thereof according to any one of the foregoing, the antibody-drug conjugate has a structure selected from the group consisting of the following:

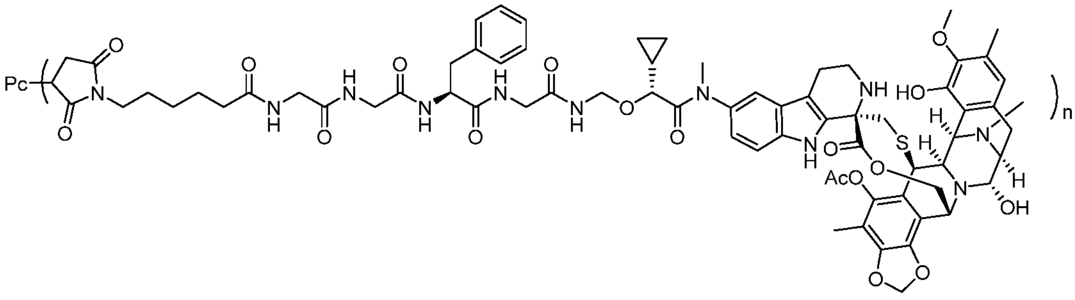

,

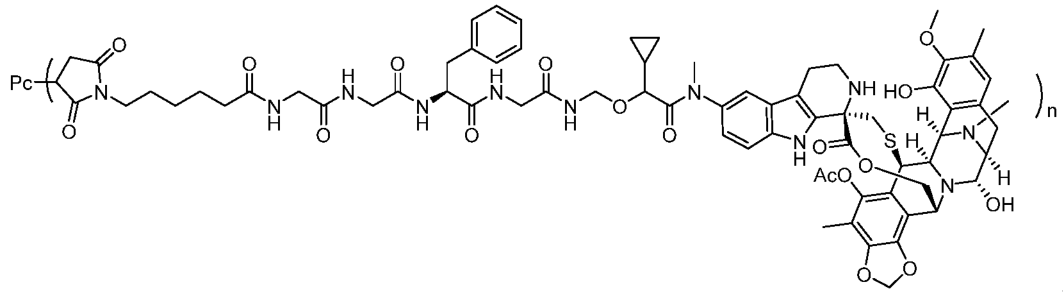

,

or

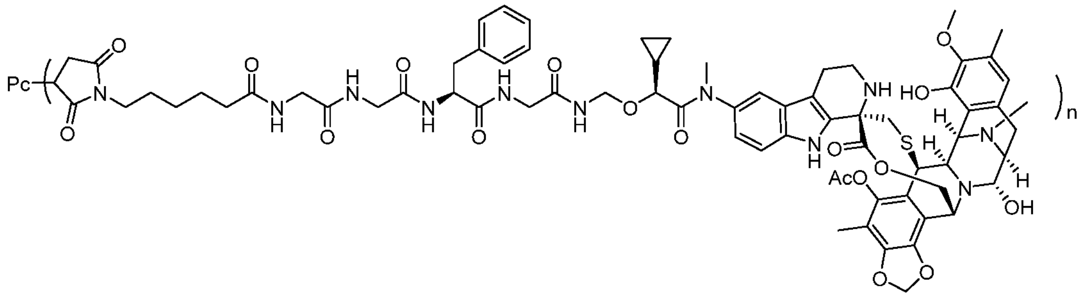

,

wherein: Pc is the anti-DLL3 antibody according to any one of the foregoing; n is 1 to 10.

In some embodiments, in the antibody-drug conjugate represented by general formula (Pc-L-Da) or (Pc-L-D) or the pharmaceutically acceptable salt thereof according to any one of the foregoing, the antibody-drug conjugate has the following structure:

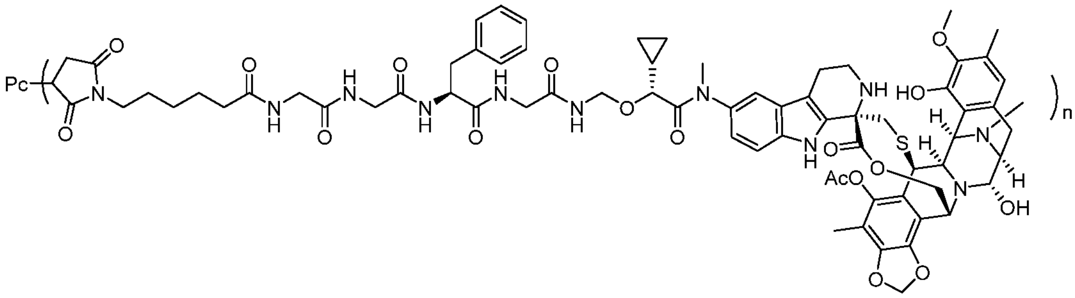

wherein:

n is 1 to 10;

Pc is the anti-DLL3 antibody according to any one of the foregoing; preferably, Pc is an anti-DLL3 antibody comprising:

a heavy chain set forth in SEQ ID NO: 60 and a light chain set forth in SEQ ID NO: 61; or
a heavy chain set forth in SEQ ID NO: 58 and a light chain set forth in SEQ ID NO: 59.

In some embodiments, in the antibody-drug conjugate represented by general formula (Pc-L-Da) or (Pc-L-D) or the pharmaceutically acceptable salt thereof according to any one of the foregoing, the antibody-drug conjugate has the following structure:

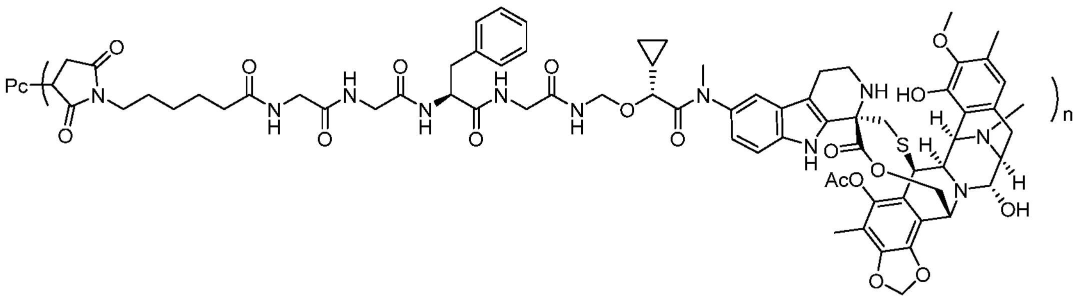

wherein:
Pc is the anti-DLL3 antibody according to any one of the foregoing; preferably, Pc is an anti-DLL3 antibody comprising:

a heavy chain set forth in SEQ ID NO: 60 and a light chain set forth in SEQ ID NO: 61; or
a heavy chain set forth in SEQ ID NO: 58 and a light chain set forth in SEQ ID NO: 59;
n is 1 to 8.

In some embodiments, in the antibody-drug conjugate represented by general formula (Pc-L-Da) or (Pc-L-D) or the pharmaceutically acceptable salt thereof according to any one of the foregoing, the antibody-drug conjugate has the following structure:

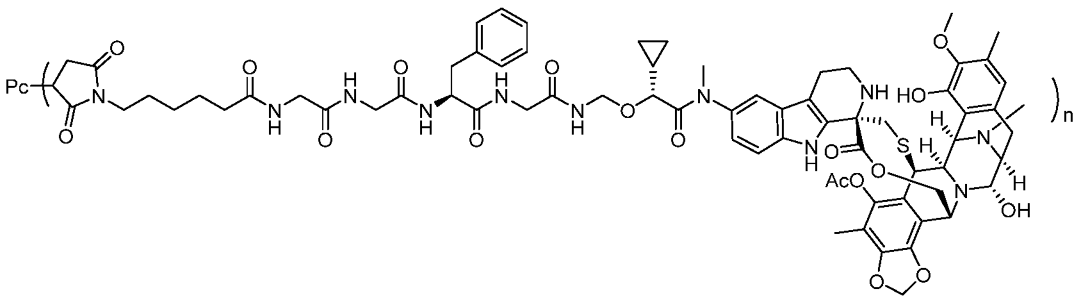

,

wherein:
Pc is the anti-DLL3 antibody according to any one of the foregoing; preferably, Pc is an anti-DLL3 antibody comprising:

a heavy chain set forth in SEQ ID NO: 60 and a light chain set forth in SEQ ID NO: 61; or
a heavy chain set forth in SEQ ID NO: 58 and a light chain set forth in SEQ ID NO: 59;
n is 3 to 5.

In some embodiments, in the antibody-drug conjugate represented by general formula (Pc-L-Da) or (Pc-L-D) or the pharmaceutically acceptable salt thereof according to any one of the foregoing, the antibody-drug conjugate has the following structure:

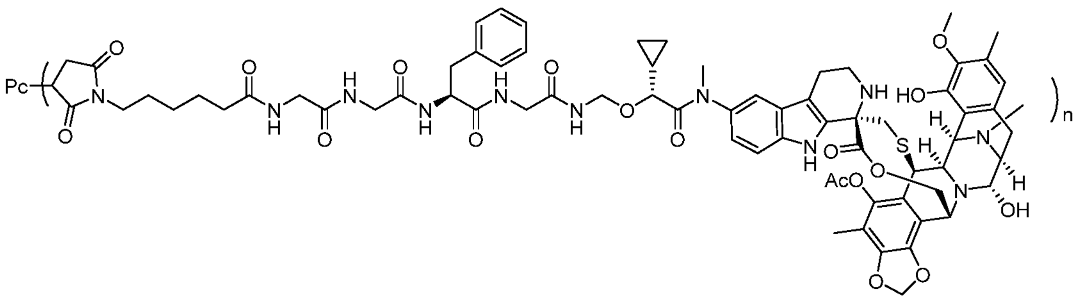

wherein:
Pc is the anti-DLL3 antibody according to any one of the foregoing; preferably, Pc is an anti-DLL3 antibody comprising:

a heavy chain set forth in SEQ ID NO: 60 and a light chain set forth in SEQ ID NO: 61; or
a heavy chain set forth in SEQ ID NO: 58 and a light chain set forth in SEQ ID NO: 59;
n is 3.73 to 4.27.

In some embodiments, in the antibody-drug conjugate represented by general formula (Pc-L-Da) or (Pc-L-D) or the pharmaceutically acceptable salt thereof according to any one of the foregoing, the antibody-drug conjugate has the following structure:

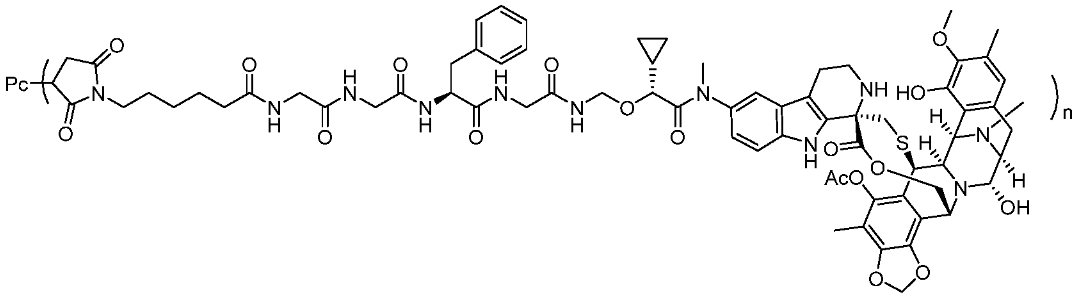

wherein:
Pc is the anti-DLL3 antibody according to any one of the foregoing; preferably, Pc is an anti-DLL3 antibody comprising:

a heavy chain set forth in SEQ ID NO: 60 and a light chain set forth in SEQ ID NO: 61; or
a heavy chain set forth in SEQ ID NO: 58 and a light chain set forth in SEQ ID NO: 59;
n is 3.72 to 4.88.

The present disclosure further provides a method for preparing an antibody-drug conjugate, comprising reducing the anti-DLL3 antibody according to any one of the foregoing and conducting a conjugation reaction with a compound represented by (L-D) or (L-Da) to give the antibody-drug conjugate according to the present disclosure. In another aspect, the present disclosure provides a pharmaceutical composition comprising the anti-DLL3 antibody according to any one of the foregoing, the antibody-drug conjugate or the pharmaceutically acceptable salt thereof according to any one of the foregoing, and one or more pharmaceutically acceptable excipients, diluents, or carriers. In some embodiments, a unit dose of the pharmaceutical composition comprises 0.1-3000 mg or 1-1000 mg of the aforementioned anti-DLL3 antibody or the aforementioned antibody-drug conjugate.
In another aspect, the present disclosure provides use of the anti-DLL3 antibody according to any one of the foregoing, or the antibody-drug conjugate or the pharmaceutically acceptable salt thereof according to any one of the foregoing, or a pharmaceutical composition comprising same as a medicament.
In another aspect, the present disclosure provides use of the anti-DLL3 antibody according to any one of the foregoing, or the antibody-drug conjugate or the pharmaceutically acceptable salt thereof according to any one of the foregoing, or the aforementioned pharmaceutical composition in the preparation of a medicament for treating a DLL3-mediated disease or disorder. In some embodiments, the DLL3-mediated disease or disorder is a tumor or cancer. In some embodiments, the DLL3-mediated disease or disorder is a DLL3-expressing disease or disorder.
In another aspect, the present disclosure provides use of the anti-DLL3 antibody according to any one of the foregoing, or the antibody-drug conjugate or the pharmaceutically acceptable salt thereof according to any one of the foregoing, or the aforementioned pharmaceutical composition in the preparation of a medicament for treating or preventing a tumor or cancer; preferably, the tumor or cancer is selected from the group consisting of:
lung cancer (e.g., small cell lung cancer, non-small cell lung cancer, and large cell lung cancer), head and neck squamous cell carcinoma, head and neck cancer, brain cancer, neuroglioma, glioblastoma multiforme, neuroblastoma, central nervous system cancer, neuroendocrine tumor, throat cancer, pharyngeal squamous cell carcinoma, oral squamous cell carcinoma, nasopharyngeal cancer, esophageal cancer, thyroid cancer (e.g., medullary thyroid cancer), malignant pleural mesothelioma, breast cancer (e.g., triple-negative breast cancer), liver cancer, hepatobiliary cancer, pancreatic cancer, gastric cancer, gastrointestinal cancer, intestinal cancer, colorectal cancer (e.g., colon cancer and rectal cancer), kidney cancer, clear cell renal cell carcinoma, ovarian cancer, endometrial cancer, cervical cancer, bladder cancer, prostate cancer, testicular cancer, adrenal cancer, glioblastoma, skin cancer, and melanoma; more preferably, the tumor or cancer is small cell lung cancer.
More preferably, the tumor or cancer is a DLL3-expressing tumor or cancer.
In another aspect, the present disclosure further relates to a method for treating and/or preventing a tumor or cancer, the method comprising administering to a subject in need thereof a therapeutically effective dose of the anti-DLL3 antibody according to any one of the foregoing, or the antibody-drug conjugate or the pharmaceutically acceptable salt thereof according to any one of the foregoing, or the aforementioned pharmaceutical composition; preferably, the tumor or cancer is a DLL3-expressing tumor or cancer.

In another aspect, the present disclosure further relates to a method for treating or preventing a tumor or cancer, the method comprising administering to a subject in need thereof a therapeutically effective dose of the anti-DLL3 antibody according to any one of the foregoing, or the antibody-drug conjugate or the pharmaceutically acceptable salt thereof according to any one of the foregoing, or the aforementioned pharmaceutical composition, wherein the tumor or cancer is selected from the group consisting of:

lung cancer (e.g., small cell lung cancer, non-small cell lung cancer, and large cell lung cancer), head and neck squamous cell carcinoma, head and neck cancer, brain cancer, neuroglioma, glioblastoma multiforme, neuroblastoma, central nervous system cancer, neuroendocrine tumor, throat cancer, pharyngeal squamous cell carcinoma, oral squamous cell carcinoma, nasopharyngeal cancer, esophageal cancer, thyroid cancer (e.g., medullary thyroid cancer), malignant pleural mesothelioma, breast cancer (e.g., triple-negative breast cancer), liver cancer, hepatobiliary cancer, pancreatic cancer, gastric cancer, gastrointestinal cancer, intestinal cancer, colorectal cancer (e.g., colon cancer and rectal cancer), kidney cancer, clear cell renal cell carcinoma, ovarian cancer, endometrial cancer, cervical cancer, bladder cancer, prostate cancer, testicular cancer, adrenal cancer, glioblastoma, skin cancer, and melanoma; preferably, the tumor or cancer is small cell lung cancer.

In another aspect, the present disclosure further provides the aforementioned anti-DLL3 antibody or the aforementioned antibody-drug conjugate as a medicament, preferably as a medicament for treating a cancer or tumor, and more preferably as a medicament for treating a DLL3-expressing cancer or tumor.

The anti-DLL3 antibody and the antibody-drug conjugate provided by the present disclosure have good affinity for the cell surface antigen, can be effectively endocytosed by a DLL3-expressing cell, have a very strong tumor growth-inhibiting effect, and have good safety.

## BRIEF DESCRIPTION OF THE DRAWINGS

FIG. 1A to FIG. 1C show the binding of Hu6 and Hu100 antibodies to DLL3 of different species. FIG. 1A shows FACS results for the binding of Hu6 and Hu100 antibodies to H1184 cells; FIG. 1B shows FACS results for the binding of Hu6 and Hu100 antibodies to cynoDLL3/CHO-s cells; FIG. 1C shows FACS results for the binding of Hu6 and Hu100 antibodies to ratDLL3/CHO-s cells.

FIG. 2 shows the experimental results for the competitive binding of different anti-DLL3 antibodies. The results show that Hu6 and Hu100 did not compete with BI-764532, indicating that antibodies Hu6 and Hu100 bind to different epitopes than BI-764532 does.

FIG. 3 shows the results for the endocytosis of Hu6 and Hu100 antibodies by cells; the results show that both Hu6 and Hu100 can be endocytosed by cells.

FIG. 4A to FIG. 4E show the growth-inhibiting effects of ADC-1 and ADC-2 on different cells. FIG. 4A shows the results for the inhibition of the growth of DMS53 cells that lowly express DLL3 by ADC-1 and ADC-2; FIG. 4B shows the results for the inhibition of the growth of H1184 cells that highly express DLL3 by ADC-1 and ADC-2; FIG. 4C shows the results for the inhibition of the growth of HT-29 cells that do not express DLL3 by ADC-1 and ADC-2; FIG. 4D shows the results for the inhibition of the growth of U-2OS cells that do not express DLL3 by ADC-1 and ADC-2; FIG. 4E shows the results for the inhibition of the growth of CHO-K1 cells that do not express DLL3 by ADC-1 and ADC-2.

FIG. 5 shows the bystander cytotoxic effects of ADC-1 and ADC-2.

FIG. 6 shows the results for the inhibition of the growth of subcutaneous xenograft tumors of DMS53 cells in mice by ADC-1 and ADC-2.

FIG. 7 shows the results for the inhibition of the growth of subcutaneous xenograft tumors of H1184 cells in mice by ADC-1 and ADC-2.

## DETAILED DESCRIPTION

I. Terminology

To facilitate the understanding of the present disclosure, certain technical and scientific terms are described below. Unless otherwise specifically defined herein, all technical and scientific terms used herein have the same meaning as commonly understood by those of ordinary skill in the art.

Unless otherwise stated, the terms used in the specification and claims have the following meanings.

The singular forms "a", "an", and "the" used in the description and claims include plural reference unless the context clearly dictates otherwise.

Unless the context clearly dictates otherwise, throughout the description and the claims, the words "comprise", "have", "include", and the like, should be construed in an inclusive sense as opposed to an exclusive or exhaustive sense.

The term "and/or" is intended to include two meanings, "and" and "or". For example, the phrase "A, B, and/or C" is intended to encompass each of the following: A, B, and C; A, B, or C; A or C; A or B; B or C; A and C; A and B; B and C; A (alone); B (alone); and C (alone). When a trade name is used in the present disclosure, it is intended to include the formulation of the commercial product under the trade name, and the drug and active drug component of the commercial product under the trade name.

The three-letter and single-letter codes used for amino acids in the present disclosure are as described in J. Biol. Chem. 243, p3558 (1968).

The term "amino acid" refers to naturally occurring and synthetic amino acids, as well as amino acid analogs and amino acid mimics that function in a manner similar to the naturally occurring amino acids. Naturally occurring amino acids are those encoded by genetic codes and those amino acids later modified, e.g., hydroxyproline, γ-carboxyglutamic acid, and O-phosphoserine. Amino acid analogs refer to compounds that have a substantially identical chemical structure (i.e., an α carbon that binds to hydrogen, carboxyl, amino, and an R group) to naturally occurring amino acids, e.g., homoserine, norleucine, methionine sulfoxide, and methioninemethyl sulfonium. Such analogs have a modified R group (e.g., norleucine) or a modified peptide skeleton, but retain a substantially identical chemical structure to naturally occurring amino acids. Amino acid mimics refer to chemical compounds that have a different structure from amino acids, but function in a manner similar to naturally occurring amino acids.

The term "amino acid mutation" includes amino acid substitutions (also known as amino acid replacements), deletions, insertions, and modifications. Any combination of substitutions, deletions, insertions, and modifications can be made to obtain a final construct, as long as the final construct possesses the desired properties, such as reduced binding to the Fc receptor. Amino acid sequence deletions and insertions include deletions and insertions at the amino terminus and/or the carboxyl terminus of a polypeptide chain. Specific amino acid mutations may be amino acid substitutions. In one embodiment, the amino acid mutation is a non-conservative amino acid substitution, i.e., a replacement of one amino acid with another amino acid having different structural and/or chemical properties. Amino acid substitutions include replacement with non-naturally occurring amino acids or with derivatives of the 20 natural amino acids (e.g., 4-hydroxyproline, 3-methylhistidine, ornithine, homoserine, and 5-hydroxylysine). Amino acid mutations can be generated using genetic or chemical methods well known in the art. Genetic methods may include site-directed mutagenesis, PCR, gene synthesis, and the like. It is contemplated that methods for altering amino acid side chain groups other than genetic engineering, such as chemical modification, may also be used. Various names may be used herein to indicate the same amino acid mutation. Herein, the expression of "position + amino acid residue" may be used to denote an amino acid residue at a specific position. For example, 366W indicates that an amino acid residue at position 366 is W. T366W indicates that the original amino acid residue T at position 366 is replaced by W.

The term "antibody" is used in the broadest sense and encompasses a variety of antibody structures, including but not limited to monoclonal antibodies, polyclonal antibodies; monospecific antibodies, multispecific antibodies (e.g., bispecific antibodies), and full-length antibodies, and antibody fragments (or antigen-binding fragments, or antigen-binding moieties), so long as they exhibit the desired antigen-binding activity. An intact antibody typically comprises two light chains and two heavy chains. From the N-terminus to the C-terminus, each heavy chain has one variable region (VH), also known as variable heavy domain or heavy chain variable region, followed by three constant domains (CH1, CH2, and CH3). Similarly, from the N-terminus to the C-terminus, each light chain has one variable region (VL), also known as variable light domain or light chain variable domain, followed by one constant light domain (light chain constant region, CL).

The terms "full-length antibody", "intact antibody", and "whole antibody" are used herein interchangeably and refer to an antibody that has a structure substantially similar to a natural antibody structure or one in which a heavy chain has an Fc region. In a natural intact antibody, the light chain comprises light chain variable region VL and constant region CL, wherein the VL is positioned at the amino terminus of the light chain, and the light chain constant region comprises a κ chain and a λ chain; the heavy chain comprises a variable region VH and a constant region (CH1, CH2, and CH3), wherein the VH is positioned at the amino terminus of the heavy chain, and the constant region is positioned at the carboxyl terminus, wherein the CH3 is closest to the carboxyl terminus, and the heavy chain can be of any isotype, including IgG (including subtypes IgG1, IgG2, IgG3, and IgG4), IgA (including subtypes IgA1 and IgA2), IgM, and IgE. The term antibody "variable region" or "variable domain" refers to a domain in the heavy or light chain of an antibody that is involved in the binding of the antibody to an antigen. Herein, the heavy chain variable region (VH) and light chain variable region (VL) of the antibody each comprise four conserved framework regions (FRs) and three complementarity-determining regions (CDRs). The term "complementarity-determining region" or "CDR" refers to a region in the variable domain that primarily contributes to antigen binding; "framework" or "FR" refers to variable domain residues other than CDR residues. A VH comprises 3 CDRs: a HCDR1, a HCDR2, and a HCDR3; a VL comprises 3 CDRs: a LCDR1, a

LCDR2, and a LCDR3. Each VH and VL is composed of three CDRs and four FRs arranged from the amino terminus (also known as N-terminus) to the carboxyl terminus (also known as C-terminus) in the following order: FR1, CDR1, FR2, CDR2, FR3, CDR3, and FR4.

The amino acid sequence boundaries of the CDRs can be determined by a variety of well-known schemes, for example, the "Kabat" numbering scheme (see Kabat et al., (1991), Sequences of Proteins of Immunological Interest, 5th ed., Public Health Service, National Institutes of Health, Bethesda, MD), the "Chothia" numbering scheme, the "ABM" numbering scheme, the "contact" numbering scheme (see Martin, ACR. Protein Sequence and Structure Analysis of Antibody Variable Domains [J]. 2001), and the ImMunoGenTics (IMGT) numbering scheme (Lefranc, M.P. et al., Dev. Comp. Immunol., 27, 55-77 (2003); Front Immunol. 2018 Oct 16; 9: 2278), and the like. The corresponding relationships among the various numbering schemes are well known to those skilled in the art and are, e.g., as shown in Table 1 below.

Table 1. Relationships among CDR numbering schemes

| CDR | IMGT | Kabat | AbM | Chothia | Contact |
|---|---|---|---|---|---|
| HCDR1 | 27-38 | 31-35 | 26-35 | 26-32 | 30-35 |
| HCDR2 | 56-65 | 50-65 | 50-58 | 52-56 | 47-58 |
| HCDR3 | 105-117 | 95-102 | 95-102 | 95-102 | 93-101 |
| LCDR1 | 27-38 | 24-34 | 24-34 | 24-34 | 30-36 |
| LCDR2 | 56-65 | 50-56 | 50-56 | 50-56 | 46-55 |
| LCDR3 | 105-117 | 89-97 | 89-97 | 89-97 | 89-96 |

Unless otherwise specified, the Kabat numbering scheme applies to the variable regions and CDR sequences in the present disclosure.

The term "antibody fragment" refers to a molecule different from an intact antibody, which comprises a moiety of an intact antibody that binds to an antigen to which the intact antibody binds. Examples of antibody fragments include, but are not limited to, Fv, Fab, Fab', Fab'-SH, $F(ab')_2$, single-domain antibodies, single-chain Fab (scFab), diabodies, linear antibodies, single-chain antibody molecules (e.g., scFv), and multispecific antibodies formed from antibody fragments.

The term "Fc region" or "fragment crystallizable region" is used to define the C-terminal region of the heavy chain of an antibody, including native Fc regions and modified Fc regions. In some embodiments, the Fc region comprises two subunits, which may be identical or different. In some embodiments, the Fc region of the human IgG heavy chain is defined as extending from the amino acid residue at position Cys226 or from Pro230 to its carboxyl terminus. Suitable Fc regions used for the antibodies described herein include Fc regions of human IgG1, IgG2 (IgG2A and IgG2B), IgG3, and IgG4. In some embodiments, the boundaries of the Fc region may also be varied, for example, by deleting the C-terminal lysine of the Fc region (residue 447 according to the EU numbering scheme) or deleting the C-terminal glycine and lysine of the Fc region (residues 446 and 447 according to the EU numbering scheme). Unless otherwise specified, the numbering scheme for the Fc region is the EU numbering scheme, also known as the EU index.

The term "chimeric antibody" refers to an antibody in which a portion of the heavy and/or light chain in the antibody is derived from a particular source or species, while the remainder of the heavy and/or light chain is derived from another different source or species.

The term "humanized antibody" is an antibody that retains the reactivity of a non-human antibody while having low immunogenicity in humans. For example, this can be achieved by retaining the non-human CDRs and replacing the remainder of the antibody with its human counterparts (i.e., the constant regions and the framework region portion of the variable regions).

The terms "human antibody", "human-derived antibody", "fully human antibody", and "complete human antibody" are used interchangeably and refer to antibodies in which the variable and constant regions are human sequences. The term encompasses antibodies that are derived from human genes but have, for example, sequences in which possible immunogenicity is reduced, affinity is increased, cysteines or glycosylation sites that may cause undesired folding are eliminated. The term encompasses antibodies recombinantly produced in non-human cells (that may confer glycosylation not characteristic of human cells). The term also encompasses antibodies that have been cultured in transgenic mice comprising human immunoglobulin heavy and light chain loci. The meaning of the human antibody specifically excludes humanized antibodies comprising non-human antigen-binding residues.

The term "affinity" refers to the overall strength of the non-covalent interaction between a single binding site of a molecule (e.g., an antibody) and its binding ligand (e.g., an antigen). Unless otherwise indicated, as used herein, binding "affinity" refers to an internal binding affinity that reflects the interaction between members of a binding pair (e.g.,

an antibody and an antigen). The affinity of a molecule X for its ligand Y can be generally denoted by the dissociation constant (KD). Affinity can be determined by conventional methods known in the art, including those described herein.

As used herein, the term "kassoc" or "ka" refers to the association rate of a particular antibody-antigen interaction, while the term "kdis" or "kd" refers to the dissociation rate of a particular antibody-antigen interaction. The term "KD" refers to the dissociation constant, which is obtained from the ratio of kd to ka (i.e., kd/ka) and denoted by a molar concentration (M). The KD value of an antibody can be determined using methods well known in the art. For example, surface plasmon resonance is determined using a biosensing system such as a system, or affinity in a solution is determined by solution equilibrium titration (SET).

The term "effector function" refers to biological activities that can be attributed to the Fc region of an antibody (either the natural sequence Fc region or the amino acid sequence variant Fc region) and vary with the antibody isotype. Examples of effector functions of an antibody include, but are not limited to: C1q binding and complement-dependent cytotoxicity, Fc receptor binding, antibody-dependent cell-mediated cytotoxicity (ADCC), phagocytosis, down-regulation of cell surface receptors (e.g., B cell receptors), and B cell activation.

The term "monoclonal antibody" refers to a population of substantially homogeneous antibodies, that is, the amino acid sequences of the antibody molecules comprised in the population are identical, except for a small number of natural mutations that may exist. In contrast, polyclonal antibody formulations generally comprise a plurality of different antibodies having different amino acid sequences in their variable domains, which are generally specific for different epitopes. "Monoclonal" should not be construed as requiring the production of the antibody by any particular method.

In some embodiments, the antibody provided by the present disclosure is a monoclonal antibody.

The term "antigen" refers to a molecule or a portion of a molecule that is capable of being bound by such a selective binding agent as an antigen-binding protein (including, for example, an antibody). An antigen may have one or more epitopes capable of interacting with different antigen-binding proteins (e.g., antibodies).

The term "epitope" refers to an area or region on an antigen that is capable of specifically binding to an antibody or an antigen-binding fragment thereof. Epitopes can be formed from contiguous strings of amino acids (linear epitope) or comprise non-contiguous amino acids (conformational epitope), e.g., coming in spatial proximity due to the folding of the antigen (i.e., by tertiary folding). The difference between the conformational epitope and the linear epitope is that in the presence of denaturing solvents, the binding of the antibody to the conformational epitope cannot be detected. An epitope comprises at least 3, at least 4, at least 5, at least 6, at least 7, or 8-10 amino acids in a unique spatial conformation. Screening for antibodies that bind to particular epitopes (i.e., those that bind to identical epitopes) can be performed using routine methods in the art, including, for example, but not limited to, alanine scanning, peptide blotting, peptide cleavage analysis, epitope excision, epitope extraction, chemical modification of the antigen (see Prot. Sci. 9 (2000) 487-496), and cross-blocking. The terms "anti-DLL3 antibody" and "antibody that binds to DLL3" refer to an antibody that is capable of binding to DLL3 or an epitope thereof with sufficient affinity. In one embodiment, the degree of binding of the anti-DLL3 antibody to an unrelated protein is less than at least about 10% of the binding of the antibody to DLL3, which can be measured by a BIACORE® surface plasmon resonance assay.

The term "capable of specifically binding", "specifically binding", or "binding" means that an antibody is capable of binding to a certain antigen or an epitope thereof with higher affinity than to other antigens or epitopes. Generally, an antibody binds to an antigen or epitope thereof with an equilibrium dissociation constant (KD) of about $1\times10^{-7}$ M or less (e.g., about $1\times10^{-8}$ M or less). In some embodiments, the KD for the binding of an antibody to an antigen is 10% or less (e.g., 1%) of the KD for the binding of the antibody to a non-specific antigen (e.g., BSA or casein). KD may be determined using known methods, for example, by a BIACORE® surface plasmon resonance assay. However, an antibody that specifically binds to an antigen or an epitope thereof may have cross-reactivity to other related antigens, e.g. to corresponding antigens from other species (homologous), such as humans or monkeys, e.g., Macaca fascicularis (cynomolgus, cyno), Pan troglodytes (chimpanzee, chimp), or Callithrix jacchus (commonmarmoset, marmoset).

The term "nucleic acid" is used interchangeably herein with the term "polynucleotide" and refers to deoxyribonucleotide or ribonucleotide and a polymer thereof in either single-stranded or double-stranded form. The term encompasses nucleic acids containing known nucleotide analogs or modified backbone residues or linkages. The nucleic acids are synthetic, naturally occurring, and non-naturally occurring, have similar binding properties as the reference nucleic acid, and are metabolized in a similar manner to the reference nucleotides. Examples of such analogs include, but are not limited to, phosphorothioate, phosphoramidate, methylphosphonate, chiral-methylphosphonate, 2-O-methyl ribonucleotide, and peptide-nucleic acid (PNA). "Isolated" nucleic acid refers to a nucleic acid molecule that has been separated from components of its natural environment. The isolated nucleic acid includes a nucleic acid molecule comprised in a cell that generally comprises the nucleic acid molecule, but the nucleic acid molecule is present extrachromosomally or at a position different from its natural chromosomal position. An isolated nucleic acid encoding a polypeptide or a fusion protein refers to one or more nucleic acid molecules encoding the polypeptide or fusion protein, including such one or more nucleic acid molecules in a single vector or separate vectors, and such one or more nucleic acid molecules present at one or more positions in a host cell. Unless otherwise specified, a particular nucleic acid sequence also implicitly encompasses conservatively modified variants thereof (e.g., degenerate codon substitutions) and complementary sequences, as well as the sequence explicitly indicated. Specifically, as detailed below, degenerate codon substitutions may be obtained by generating sequences in which the third position of one or more selected (or all) codons is substituted with mixed bases and/or deoxyinosine residues.

The terms "polypeptide" and "protein" are used interchangeably herein and refer to a polymer of amino acid residues, wherein one or more amino acid residues are artificial chemical mimics of corresponding naturally occurring amino acids, as well as to naturally occurring amino acid polymers and non-naturally occurring amino acid polymers. Unless otherwise specified, a particular polypeptide sequence also implicitly encompasses conservatively modified variants thereof.

The term sequence "identity" refers to the degree (percentage) to which the amino acids/nucleic acids of two sequences are identical at equivalent positions; when two sequences are optimally aligned, gaps are introduced, if necessary, to achieve the maximum percent identity, and any conservative substitution is not seen as part of the sequence identity. To determine percent sequence identity, alignments can be accomplished by techniques known to those skilled in the art, for example, using publicly available computer software, such as BLAST, BLAST-2, ALIGN, ALIGN-2, or Megalign (DNASTAR) software. Those skilled in the art can determine parameters suitable for measuring alignment, including any algorithms required to achieve maximum alignment of the full length of the aligned sequences.

The term "vector" means a polynucleotide molecule capable of transporting another polynucleotide linked thereto. One type of vector is a "plasmid", which refers to a circular double-stranded DNA loop into which additional DNA segments can be ligated. Another type of vector is a viral vector, such as an adeno-associated viral vector (AAV or AAV2), wherein additional DNA segments can be ligated into the viral genome. Certain vectors are capable of autonomous replication in a host cell into which they are introduced (e.g., bacterial vectors having a bacterial origin of replication and episomal mammalian vectors). Other vectors (e.g., non-episomal mammalian vectors) can be integrated into the genome of a host cell upon introduction into the host cell, and thereby are replicated along with the host genome. The term "expression vector" or "expression construct" refers to a vector that can transform a host cell and comprises a nucleic acid sequence that directs and/or controls the expression of one or more heterologous coding regions operably linked thereto. Expression constructs may include, but are not limited to, sequences that affect or control transcription and translation and affect RNA splicing of a coding region operably linked thereto in the presence of an intron.

The terms "host cell", "host cell line", and "host cell culture" are used interchangeably and refer to cells into which exogenous nucleic acids have been introduced, including progenies of such cells. Host cells include "transformants" and "transformed cells", which include primary transformed cells and progeny derived therefrom, regardless of the number of passages. Progeny may not be exactly the same as parent cells in terms of nucleic acid content and may contain mutations. Mutant progeny that have the same function or biological activity as the cells screened or selected from the initially transformed cells are included herein. Host cells include prokaryotic and eukaryotic host cells, wherein eukaryotic host cells include, but are not limited to, mammalian cells, insect cell lines, plant cells, and fungal cells. Exemplary host cells are as follows: Chinese hamster ovary (CHO) cells, NSO, SP2 cells, HeLa cells, baby hamster kidney (BHK) cells, monkey kidney cells (COS), human hepatocellular carcinoma cells (e.g., HepG2), A549 cells, 3T3 cells and HEK-293 cells, Pichiapastoris, Pichia finlandica, Candida albicans, Aspergillus niger, Aspergillus oryzae and Trichoderma reesei.

"Antibody-drug conjugate" (ADC) is a conjugate obtained by linking an antibody (or an antigen-binding fragment thereof) to a drug directly or by a linker.

"Drug (abbreviated as D)" is any substance having biological or detectable activity (for example, therapeutic agents, detectable labels, and binding agents) and prodrugs that are metabolized to an active agent *in vivo.* Examples of therapeutic agents include cytotoxic agents, chemotherapeutic agents, cytostatic agents, and immunomodulators. Chemotherapeutic agents are chemical compounds that can be used to treat cancer. Representative therapeutic agents include cytotoxins, cytotoxic agents, and cytostatic agents.

Cytotoxic effects refer to the deletion, elimination, and/or killing of target cells. A cytotoxic agent refers to an agent that has a cytotoxic and/or cytostatic effect on a cell. The cytostatic effect refers to the inhibition of cell proliferation. A cytostatic agent refers to an agent that has a cytostatic effect on a cell, thereby inhibiting the growth and/or expansion of a particular subset of cells.

Additional representative therapeutic agents include radioisotopes, chemotherapeutic agents, immunomodulators, anti-angiogenic agents, anti-proliferative agents, pro-apoptotic agents, and lytic enzymes (e.g., RNAse). These descriptive words of drugs are not mutually exclusive and, thus, one or more of the above terms may be used to describe a therapeutic agent. For example, the radioisotope selected is also a cytotoxin. Therapeutic agents may be prepared as pharmaceutically acceptable salts, acids, or derivatives of any one of the above. Generally, conjugates having a radioisotope as the drug are referred to as radioimmunoconjugates, and those having a chemotherapeutic agent as the drug are referred to as chemoimmunoconjugates.

Examples of cytotoxic agents include, but are not limited to, anthracyclines, auristatins, CC-1065, dolastatin, duocarmycin, enediynes, geldanamycin, maytansine, puromycin, taxanes, vinca alkaloids, SN-38, tubulysin, hemiasterlin, eribulin, Trabectedin, Lurbinectedin, and stereoisomers, isosteres, analogs, or derivatives thereof. Chemotherapeutic

agents, plant toxins, other bioactive proteins, enzymes (i.e., ADEPT), radioisotopes, and photosensitizers (i.e., for photodynamic therapy) can also be used.

The term "label", when used herein, refers to a detectable compound or composition that is conjugated directly or indirectly to the antibody so as to generate a "labeled" antibody. The label may be detectable by itself (e.g., radioisotope labels or fluorescent labels) or, in the case of an enzymatic label, may catalyze a detectable chemical change in a substrate compound or composition. Radioisotopic labels include, for example, I-131, I-123, I-125, Y-90, Re-188, Re-186, At-211, Cu-67, Bi-212, and Pd-109. The label may also be a non-detectable entity, such as a toxin.

The term "linker unit" or "linker" refers to a chemical structural fragment or bond which is linked to an antibody at one end and to a drug at the other end, and may also be linked to other linkers before the antibody or drug. Attachment of a linker to an antibody may be accomplished in a variety of ways, such as through surface lysines, reductive coupling to oxidized carbohydrates, cysteine residues released by reducing interchain disulfide bonds, reactive cysteine residues engineered at specific sites, and tags containing acyl donor glutamines, or an endogenous glutamine made reactive by polypeptide engineering in the presence of transglutaminase and an amine. A variety of ADC linking systems are known in the art, including hydrazone-, disulfide-, and peptide-based linkages.

The linker may comprise one or more linker elements. Exemplary linker elements include 6-maleimidocaproyl ("MC"), maleimidopropionyl ("MP"), valine-citrulline ("val-cit" or "vc"), alanine-phenylalanine ("ala-phe"), p-aminobenzyloxycarbonyl ("PAB"), N-succinimidyl 4-(2-pyridylthio)pentanoate ("SPP"), N-succinimidyl 4-(N-maleimidomethyl)cyclohexane-1 carboxylate ("SMCC", also referred to herein as "MCC"), and N-succinimidyl(4-iodo-acetyl)aminobenzoate ("SIAB").

The linker may be selected from the group consisting of the following elements or a combination thereof: a stretcher, a spacer, and an amino acid unit. The linker may be synthesized using methods known in the art, such as those described in US20050238649A1. A linker may be a "cleavable linker" favoring the release of drugs in cells. For example, acid-labile linkers (e.g., hydrazones), protease-sensitive (e.g., peptidase-sensitive) linkers, photolabile linkers, dimethyl linkers or disulfide-containing linkers can be used (Chari et al., Cancer Research, 52: 127-131(1992); U.S. Patent No. 5,208,020).

Linker elements include, but are not limited to:

MC = 6-maleimidocaproyl, the structure of which is as follows:

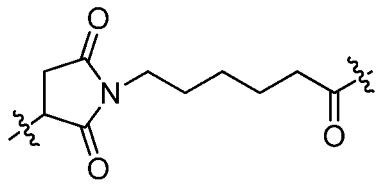

,

Val-Cit or "vc" = valine-citrulline (an exemplary dipeptide in a protease cleavable linker),
citrulline = 2-amino-5-ureidopentanoic acid,
PAB = p-aminobenzyloxycarbonyl (an example of "self-immolative" linker elements),
Me-Val-Cit = N-methyl-valine-citrulline (where the linker peptide bond has been modified to prevent it from being cleaved by cathepsin B),
MC(PEG)6-OH = maleimidocaproyl-polyethylene glycol (attachable to antibody cysteine),
SPP = N-succinimidyl 4-(2-pyridylthio)valerate,
SPDP = N-succinimidyl 3-(2-pyridyldithio)propionate,
SMCC = succinimidyl-4-(N-maleimidomethyl)cyclohexane-1-carboxylate, and
IT = iminothiolane.

"L-D" is the linker-drug moiety resulting from the attachment of the drug (D) to the linker (L).

"Drug loading", also known as drug-to-antibody ratio (DAR), refers to the average number of drugs coupled to each antibody in an ADC. It may range, for example, from about 1 to about 10 drugs conjugated to each antibody, and in certain embodiments, from about 1 to about 8 drugs conjugated to each antibody, preferably selected from the group consisting of 2-8, 2-7, 2-6, 2-5, 2-4, 1-3, 3-4, 3-5, 5-6, 5-7, 5-8, and 6-8 drugs conjugated to each antibody. The ADC general formulas of the present disclosure include a set of antibody-drug conjugates within a certain range as described above. In the embodiments of the present disclosure, drug loading may be represented by n and is a decimal or an integer. Drug loading can be determined by conventional methods such as UV/visible spectroscopy, mass spectrometry, ELISA assays, HIC, and RP-HPLC.

In one embodiment of the present disclosure, the drug is conjugated to the reactive group (e.g., sulfhydryl) of

the antibody by a linker.

The drug loading of an ADC can be controlled by the following non-limiting methods, including:

(1) controlling a molar ratio of a linking reagent to a monoclonal antibody,
(2) controlling reaction time and temperature, and
(3) selecting different reaction reagents.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkyl). The alkyl is preferably an alkyl group having 1 to 12 carbon atoms (i.e., $C_{1-12}$ alkyl), more preferably an alkyl group having 1 to 6 carbon atoms (i.e., $C_{1-6}$ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched-chain isomers thereof, and the like. Alkyl may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a divalent alkyl group, wherein the alkyl is as defined above; it has 1 to 20 (e.g., 1, 2, 3, 4, 5, 6, 7, 8, 9, 10, 11, 12, 13, 14, 15, 16, 17, 18, 19, or 20) carbon atoms (i.e., $C_{1-20}$ alkylene). The alkylene is preferably an alkylene group having 1 to 12 carbon atoms (i.e., $C_{1-12}$ alkylene), more preferably an alkylene group having 1 to 6 carbon atoms (i.e., $C_{1-6}$ alkylene). Non-limiting examples include: $-CH_2-$, $-CH(CH_3)-$, $-C(CH_3)_2-$, $-CH_2CH_2-$, $-CH(CH_2CH_3)-$, $-CH_2CH(CH_3)-$, $-CH_2C(CH_3)_2-$, $-CH_2CH_2CH_2-$, $-CH_2CH_2CH_2CH_2-$, and the like. Alkylene may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl is as defined above, and it has 2 to 12 (e.g., 2, 3, 4, 5, 6, 7, 8, 9, 10, 11, or 12) carbon atoms (i.e., $C_{2-12}$ alkenyl). The alkenyl is preferably an alkenyl group having 2 to 6 carbon atoms (i.e., $C_{2-6}$ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl is as defined above, and it has 2 to 12 (e.g., 2, 3, 4, 5, 6, 7, 8, 9, 10, 11, or 12) carbon atoms (i.e., $C_{2-12}$ alkynyl). The alkynyl is preferably an alkynyl group having 2 to 6 carbon atoms (i.e., $C_{2-6}$ alkynyl). Non-limiting examples include: ethynyl, propynyl, butynyl, pentynyl, hexynyl, and the like. Alkynyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e., 3- to 12-membered cycloalkyl), more preferably a cycloalkyl group having 3 to 8 ring atoms (i.e., 3- to 8-membered cycloalkyl), and most preferably a cycloalkyl group having 3 to 6 ring atoms (i.e., 3- to 6-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings, which may contain one or more double bonds in the rings or may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that the system contains at least one all-carbon ring and the point of attachment is on the all-carbon ring; the system has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). The spirocycloalkyl is preferably a spirocycloalkyl group having 6 to 14 ring atoms (i.e., 6-to 14-membered spirocycloalkyl), more preferably a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl), preferably monospirocycloalkyl or bispirocycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples include:

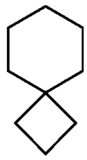 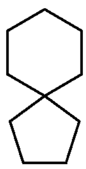 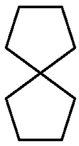 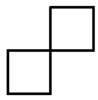 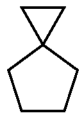 ,

wherein the point of attachment may be at any position;

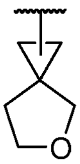 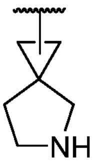 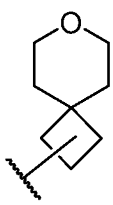 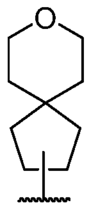 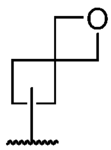 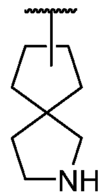 ,

and the like.

The term "fused cycloalkyl" refers to a polycyclic system in which two adjacent carbon atoms are shared between rings, which is formed by fusing a monocyclic cycloalkyl group with one or more monocyclic cycloalkyl groups or fusing a monocyclic cycloalkyl group with one or more of heterocyclyl, aryl, or heteroaryl, wherein the point of attachment is on the monocyclic cycloalkyl group; the system may contain one or more double bonds in the rings and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). The fused cycloalkyl is preferably a fused cycloalkyl group having 6 to 14 ring atoms (i.e., 6- to 14-membered fused cycloalkyl), more preferably a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl), preferably bicyclic fused cycloalkyl or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl. Non-limiting examples include:

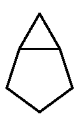 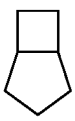 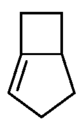 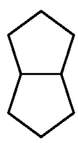 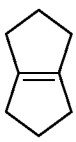 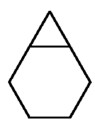 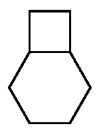 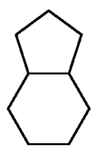 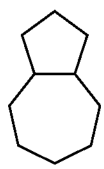 ,

wherein the point of attachment may be at any position;

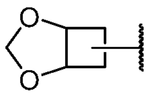 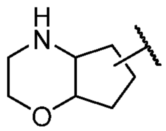 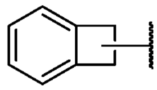 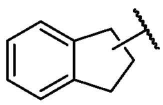 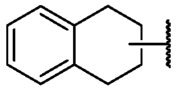

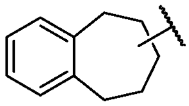 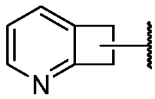 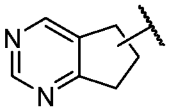 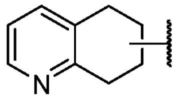 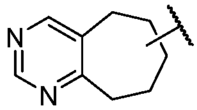 ,

and the like.

The term "bridged cycloalkyl" refers to an all-carbon polycyclic system in which two carbon atoms not directly linked are shared between rings, which may contain one or more double bonds in the rings and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). The bridged cycloalkyl is preferably a bridged cycloalkyl group having 6 to 14 carbon atoms (i.e., 6- to 14-membered bridged cycloalkyl), more preferably a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl or tetracyclic bridged cycloalkyl), preferably bicyclic bridged cycloalkyl or tricyclic bridged cycloalkyl. Non-limiting examples include:

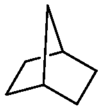 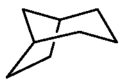 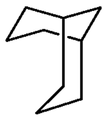 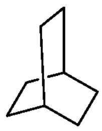 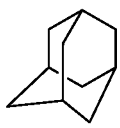 ,

wherein the point of attachment may be at any position.

Cycloalkyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl), which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). The heterocyclyl is preferably a heterocyclyl group having 3 to 12 ring atoms (i.e., 3- to 12-membered heterocyclyl), further preferably a heterocyclyl group having 3 to 8 ring atoms (i.e., 3- to 8-membered heterocyclyl), more preferably a heterocyclyl group having 3 to 6 ring atoms (i.e., 3- to 6-membered heterocyclyl), and most preferably a heterocyclyl group having 5 or 6 ring atoms (i.e., 5- or 6-membered heterocyclyl).

Non-limiting examples of the monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, which may contain one or more double bonds in the rings and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that the system contains at least one monocyclic heterocyclyl group and the point of attachment is on the monocyclic heterocyclyl group; the system has 5 to 20 (e.g., 5, 6, 7, 8, 9, 10, 11, 12, 13, 14, 15, 16, 17, 18, 19, or 20) ring atoms (i.e., 5-to 20-membered spiroheterocyclyl). The spiroheterocyclyl is preferably a spiroheterocyclyl group having 6 to 14 ring atoms (i.e., 6- to 14-membered spiroheterocyclyl), more preferably a spiroheterocyclyl group having 7 to 10 ring atoms (i.e., 7- to 10-membered spiroheterocyclyl). The spiroheterocyclyl includes monospiroheterocyclyl and polyspiroheterocyclyl (e.g., bispiroheterocyclyl), preferably monospiroheterocyclyl or bispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples include:

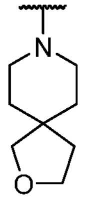 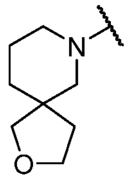 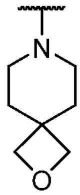 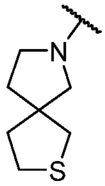 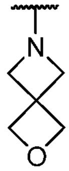 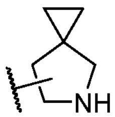 ,

and the like.

The term "fused heterocyclyl" refers to a polycyclic heterocyclic system in which two adjacent atoms are shared between rings, which may contain one or more double bonds in the rings and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); the system is formed by fusing a monocyclic heterocyclyl group with one or more monocyclic heterocyclyl groups or fusing a monocyclic heterocyclyl group with one or more of cycloalkyl, aryl, or heteroaryl, wherein the point of attachment is on the monocyclic heterocyclyl group, and the system has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). The fused heterocyclyl is preferably a fused heterocyclyl group having 6 to 14 ring atoms (i.e., 6-to 14-membered fused heterocyclyl), more preferably a fused heterocyclyl group having 7 to 10 ring atoms (i.e., 7- to 10-membered fused heterocyclyl). The fused heterocyclyl includes bicyclic heterocyclyl and polycyclic fused heterocyclyl (e.g., tricyclic fused heterocyclyl and tetracyclic fused heterocyclyl), preferably bicyclic fused heterocyclyl or tricyclic fused heterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples include:

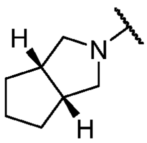 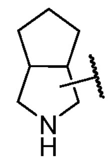 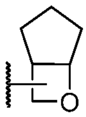 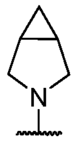 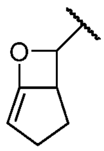 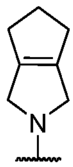 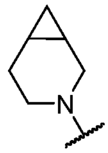

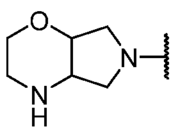 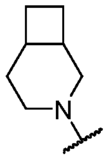 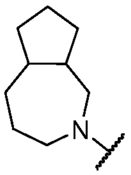 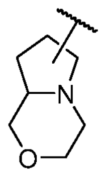 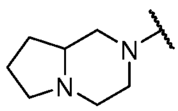 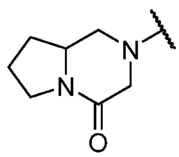

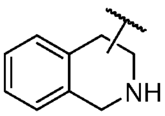 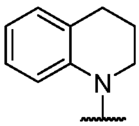 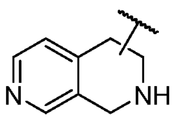 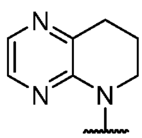 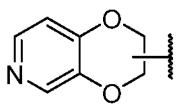 ,

and the like.

The term "bridged heterocyclyl" refers to a polycyclic heterocyclic system in which two atoms not directly linked are shared between rings, which may contain one or more double bonds in the rings and contains in the rings at least one (e.g., 1, 2, 3, or 4) heteroatom selected from the group consisting of nitrogen, oxygen, and sulfur (the nitrogen may

be optionally oxidized to form a nitrogen oxide; the sulfur may be optionally substituted with oxo to form a sulfoxide or sulfone, excluding -O-O-, -O-S-, or -S-S-); the system has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). The bridged heterocyclyl is preferably a bridged heterocyclyl group having 6 to 14 ring atoms (i.e., 6- to 14-membered bridged heterocyclyl), more preferably a bridged heterocyclyl group having 7 to 10 ring atoms (i.e., 7- to 10-membered bridged heterocyclyl). According to the number of constituent rings, the bridged heterocyclyl may be bicyclic bridged heterocyclyl or polycyclic bridged heterocyclyl (e.g., tricyclic bridged heterocyclyl or tetracyclic bridged heterocyclyl), preferably bicyclic bridged heterocyclyl or tricyclic bridged heterocyclyl. Non-limiting examples include:

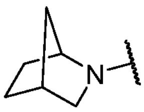 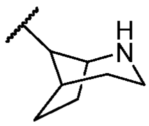 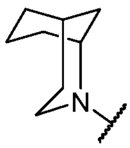 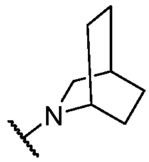 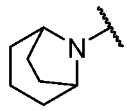 ,

and the like.

Heterocyclyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated $\pi$-electron system, which has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl). The monocyclic aryl is, for example, phenyl. Non-limiting examples of the polycyclic aryl include: naphthyl, anthracenyl, phenanthrenyl, and the like. The polycyclic aryl also includes those formed by fusing phenyl with one or more of heterocyclyl or cycloalkyl or fusing naphthyl with one or more of heterocyclyl or cycloalkyl, wherein the point of attachment is on the phenyl or naphthyl, and in the circumstances the number of ring atoms continues to represent the number of ring atoms in the polycyclic aromatic ring system; non-limiting examples include:

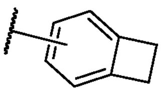 , 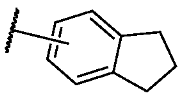 , 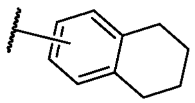 , 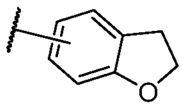 , 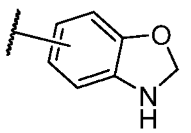 ,

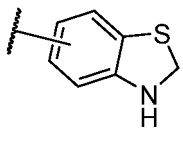 ,

and the like.

Aryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated $\pi$-electron system, which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 5 to 14 (e.g., 5, 6, 7, 8, 9, 10, 11, 12, 13, or 14) ring atoms (i.e., 5- to 14-membered heteroaryl). The heteroaryl is preferably a heteroaryl group having 5 to 10 ring atoms (i.e., 5- to 10-membered heteroaryl), more preferably a heteroaryl group having 5 or 6 ring atoms (i.e., 5- or 6-membered heteroaryl).

Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridinonyl, N-alkylpyridinone (e.g.,

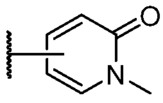),

pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, quinazolinyl, benzothiazolyl, carbazolyl, and the like. The polycyclic heteroaryl also includes those formed by fusing a monocyclic heteroaryl group with one or more aryl groups, wherein the point of attachment is on an aromatic ring, and in the circumstances the number of ring atoms continues to represent the number of ring atoms in the polycyclic heteroaromatic ring system. The polycyclic heteroaryl also includes those formed by fusing monocyclic heteroaryl with one or more of cycloalkyl or heterocyclyl, wherein the point of attachment is on the monocyclic heteroaromatic ring, and in the circumstances the number of ring atoms continues to represent the number of ring atoms in the polycyclic heteroaromatic ring system. Non-limiting examples include:

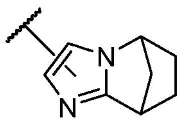 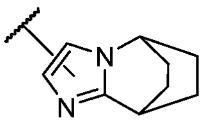 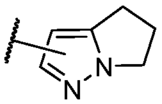 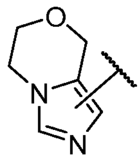 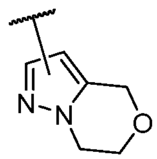 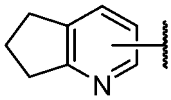

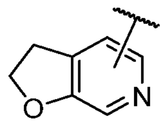

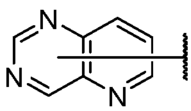 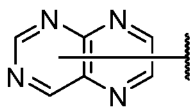 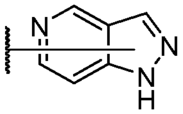 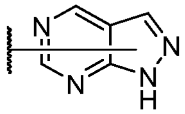 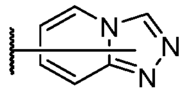

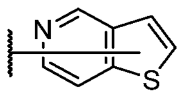 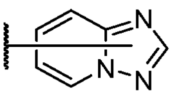 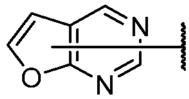,

and the like.

Heteroaryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "amino protecting group" refers to an easily removable group that is introduced onto an amino group in order for the amino group to remain unchanged when reactions take place on other parts of the molecule. Non-limiting examples include: (trimethylsilyl)ethoxymethyl, tetrahydropyranyl, tert-butyloxycarbonyl (Boc), benzyloxycarbonyl (Cbz), fluorenylmethoxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulfonyl (Tos), trifluoroacetyl (Tfa), trityl (Trt), 2,4-dimethoxybenzyl (DMB), acetyl, benzyl, allyl, p-methoxybenzyl, and the like.

The term "hydroxy protecting group" refers to an easily removable group that is introduced onto a hydroxy group to block or protect the hydroxy group such that reactions take place on other functional groups of the compound. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to an alkyl group substituted with one or more halogens, wherein the alkyl is as defined above.

The term "haloalkoxy" refers to an alkoxy group substituted with one or more halogens, wherein the alkoxy is as defined above.

The term "deuterated alkyl" refers to an alkyl group substituted with one or more deuterium atoms, wherein the alkyl is as defined above.

The term "hydroxyalkyl" refers to an alkyl group substituted with one or more hydroxy groups, wherein the alkyl is as defined above.

The term "methylidene" refers to $=CH_2$.

The term "halogen" refers to fluorine, chlorine, bromine, or iodine.

The term "hydroxy" refers to -OH.

The term "sulfhydryl" refers to -SH.

The term "amino" refers to $-NH_2$.

The term "cyano" refers to -CN.

The term "nitro" refers to $-NO_2$.

The term "oxo" refers to "=O".

The term "carbonyl" refers to C=O.

The term "carboxyl" refers to -C(O)OH.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

In chemical formulas, the abbreviation "Me" refers to methyl.

In chemical formulas, the abbreviation "Ph" refers to phenyl.

The term "THF" refers to tetrahydrofuran.

The term "EtOAc" refers to ethyl acetate.

The term "MeOH" refers to methanol.

The term "DMF" refers to N,N-dimethylformamide.

The term "DIPEA" refers to diisopropylethylamine.

The term "TFA" refers to trifluoroacetic acid.

The term "MeCN" refers to acetonitrile.

The term "DMA" refers to N,N-dimethylacetamide.

The term "$Et_2O$" refers to diethyl ether.

The term "DCE" refers to 1,2-dichloroethane.

The term "DIPEA" refers to N,N-diisopropylethylamine.

The term "NBS" refers to N-bromosuccinimide.

The term "NIS" refers to N-iodosuccinimide.

The term "Cbz-Cl" refers to benzyl chloroformate.

The term "$Pd_2(dba)_3$" refers to tris(dibenzylideneacetone)dipalladium(0). The term "Dppf' refers to 1,1'-bis(diphenylphosphino)ferrocene.

The term "HATU" refers to 2-(7-benzotriazole oxide)-N,N,N',N'-tetramethylurea hexafluorophosphate.

The term "KHMDS" refers to potassium hexamethyldisilylamide.

The term "LiHMDS" refers to lithium bis(trimethylsilyl)amide.

The term "MeLi" refers to methyllithium.

The term "n-BuLi" refers to n-butyllithium.

The term "$NaBH(OAc)_3$" refers to sodium triacetoxyborohydride.

The term "DCM" refers to dichloromethane.

The term "DMAP" refers to 4-dimethylaminopyridine.

The term "DMBOH" refers to 2,4-dimethoxybenzyl alcohol.

The term "EDCI" refers to 1-(3-dimethylaminopropyl)-3-ethylcarbodiimide.

The term "MTBE" refers to methyl tert-butyl ether.

The term "DMF" refers to N,N-dimethylformamide.

The term "DMTMM" refers to 4-(4,6-dimethoxytriazin-2-yl)-4-methylmorpholine hydrochloride.

The term "EtOAc" refers to ethyl acetate.

The compounds of the present disclosure may exist in specific stereoisomeric forms. The term "stereoisomer" refers to isomers that are structurally identical but differ in the arrangement of the atoms in space. It includes cis and trans (or *Z* and *E*) isomers, (-)-and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and

(*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. Furthermore, separation of enantiomers and diastereomers is generally accomplished by chromatography.

In the chemical structures of the compounds according to the present disclosure, a bond

“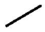”

indicates an unspecified configuration; that is, if chiral isomers exist in the chemical structure, the bond

“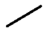”

may be

“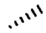” or “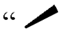”,

or includes both the configurations of

“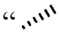” and “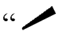”.

The compounds of the present disclosure may exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers; that is, it is present in the form of a single isomer or in the form of a mixture of the tautomers in any ratio. Non-limiting examples include: keto-enol, imine-enamine, lactam-lactim, and the like. The lactam-lactim in equilibrium is as follows:

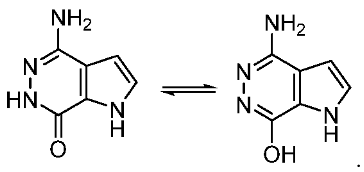

For example, reference to pyrazolyl is understood to include any one of the following two structures or a mixture of the two tautomers:

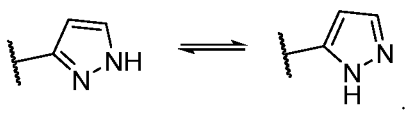

All tautomeric forms fall within the scope of the present disclosure, and the nomenclature of the compounds does not exclude any tautomer.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as $^{2}H$ (deuterium, D), $^{3}H$ (deuterium, T), $^{11}C$, $^{13}C$, $^{14}C$, $^{15}N$, $^{17}O$, $^{18}O$, $^{32}p$, $^{33}p$, $^{33}S$, $^{34}S$, $^{35}S$, $^{36}S$, $^{18}F$, $^{36}Cl$, $^{82}Br$, $^{123}I$, $^{124}I$, $^{125}I$, $^{129}I$, and $^{131}I$; deuterium is preferred.

Compared with non-deuterated drugs, deuterated drugs have the advantages of reduced toxic and side effects, increased drug stability, enhanced curative effect, prolonged biological half-lives, and the like. All isotopic variations of the compound of the present disclosure, whether radioactive or not, are intended to be included within the scope of the

present disclosure. Each available hydrogen atom linked to a carbon atom may be independently replaced with a deuterium atom, wherein replacement of deuterium may be partial or complete, and replacement of partial deuterium refers to replacement of at least one hydrogen atom with at least one deuterium atom.

When a position is specifically assigned "deuterium" or "D", the position should be construed as the abundance of deuterium being at least 1000 times greater than the natural abundance of deuterium (which is 0.015%) (i.e., at least 15% deuterium incorporation). In some embodiments, the abundance of deuterium of each of the assigned deuterium atoms is at least 1000 times greater than the natural abundance of deuterium (i.e., at least 15% deuterium incorporation). In some embodiments, the abundance of deuterium of each of the assigned deuterium atoms is at least 2000 times greater than the natural abundance of deuterium (i.e., at least 30% deuterium incorporation). In some embodiments, the abundance of deuterium of each of the assigned deuterium atoms is at least 3000 times greater than the natural abundance of deuterium (i.e., at least 45% deuterium incorporation). In some embodiments, the abundance of deuterium of each of the assigned deuterium atoms is at least 3340 times greater than the natural abundance of deuterium (i.e., at least 50.1% deuterium incorporation). In some embodiments, the abundance of deuterium of each of the assigned deuterium atoms is at least 3500 times greater than the natural abundance of deuterium (i.e., at least 52.5% deuterium incorporation). In some embodiments, the abundance of deuterium of each of the assigned deuterium atoms is at least 4000 times greater than the natural abundance of deuterium (i.e., at least 60% deuterium incorporation). In some embodiments, the abundance of deuterium of each of the assigned deuterium atoms is at least 4500 times greater than the natural abundance of deuterium (i.e., at least 67.5% deuterium incorporation). In some embodiments, the abundance of deuterium of each of the assigned deuterium atoms is at least 5000 times greater than the natural abundance of deuterium (i.e., at least 75% deuterium incorporation). In some embodiments, the abundance of deuterium of each of the assigned deuterium atoms is at least 5500 times greater than the natural abundance of deuterium (i.e., at least 82.5% deuterium incorporation). In some embodiments, the abundance of deuterium of each of the assigned deuterium atoms is at least 6000 times greater than the natural abundance of deuterium (i.e., at least 90% deuterium incorporation). In some embodiments, the abundance of deuterium of each of the assigned deuterium atoms is at least 6333.3 times greater than the natural abundance of deuterium (i.e., at least 95% deuterium incorporation). In some embodiments, the abundance of deuterium of each of the assigned deuterium atoms is at least 6466.7 times greater than the natural abundance of deuterium (i.e., at least 97% deuterium incorporation). In some embodiments, the abundance of deuterium of each of the assigned deuterium atoms is at least 6600 times greater than the natural abundance of deuterium (i.e., at least 99% deuterium incorporation). In some embodiments, the abundance of deuterium of each of the assigned deuterium atoms is at least 6633.3 times greater than the natural abundance of deuterium (i.e., at least 99.5% deuterium incorporation).

"Substitution" or "substituted" means that one or more, preferably 1 to 6, and more preferably 1 to 3, hydrogen atoms in the group are independently substituted with a corresponding number of substituents. Those skilled in the art can determine (experimentally or theoretically) possible or impossible substitutions without undue effort. For example, it may be unstable when an amino or hydroxy group with free hydrogen binds to a carbon atom with an unsaturated bond (e.g., an alkene).

The present disclosure also includes various deuterated forms of antibody-drug conjugates of formula (Pc-L-Da) or (Pc-L-D). Each available hydrogen atom connected to a carbon atom may be independently replaced by a deuterium atom. Those skilled in the art are able to synthesize deuterated forms of antibody-drug conjugates of formula (Pc-L-Da) or (Pc-L-D) by reference to the relevant literature. Commercially available deuterated starting materials can be used in preparing deuterated forms of antibody-drug conjugates of formula (Pc-L-Da) or (Pc-L-D), or they can be synthesized using conventional techniques with deuterated reagents including, but not limited to, deuterated borane, tri-deuterated borane in tetrahydrofuran, deuterated lithium aluminum hydride, deuterated iodoethane, deuterated iodomethane, and the like.

"Optional" or "optionally" means that the event or circumstance subsequently described may, but does not necessarily, occur; this includes an instance where the event or circumstance occurs and an instance where it does not. For example, "alkyl that is optionally substituted with halogen or cyano" includes an instance where the alkyl is substituted with halogen or cyano and an instance where the alkyl is not substituted with halogen or cyano.

The term "pharmaceutical composition" refers to a mixture containing one or more of the compounds or the physiologically/pharmaceutically acceptable salts or pro-drugs thereof described herein, and other chemical components, as well as other components such as physiologically/pharmaceutically acceptable carriers and excipients. The pharmaceutical composition is intended to promote administration to an organism and facilitate the absorption of the active ingredient so that it can exert its biological activity. The pharmaceutical composition may be in the form of a sterile injectable aqueous solution. Acceptable vehicles or solvents include water, Ringer's solution, and isotonic sodium chloride solution. The sterile injectable formulation may be a sterile injectable oil-in-water microemulsion in which the active ingredient is dissolved in the oil phase. For example, the active ingredient is dissolved in a mixture of soybean oil and lecithin. The oil solution is then added to a mixture of water and glycerol and treated to form a microemulsion. The injection or microemulsion can be locally injected into the bloodstream of a subject in large quantities. Alternatively, the

solution and microemulsion are administered in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition may be in the form of a sterile injectable aqueous or oily suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent, e.g., a solution prepared in 1,3-butanediol. In addition, a sterile fixed oil may be conventionally used as a solvent or a suspending medium. For this purpose, any blend fixed oil including synthetic monoglycerides or diglycerides can be used. In addition, fatty acids such as oleic acid may also be used in the preparation of injections. The term "pharmaceutically acceptable salt" refers to salts of the antibodies or antibody-drug conjugates of the present disclosure that are safe and effective when administered to a subject and that possess the requisite biological activity. As an example, the antibody or antibody-drug conjugate of the present disclosure at least comprises one amino group and thus may form a salt with an acid. Non-limiting examples of the pharmaceutically acceptable salts include: hydrochloride, hydrobromide, hydriodate, sulfate, bisulfate, citrate, acetate, succinate, ascorbate, oxalate, nitrate, sorbate, hydrophosphate, dihydrophosphate, salicylate, hydrocitrate, tartrate, maleate, fumarate, formate, benzoate, mesylate, ethanesulfonate, benzenesulphonate, and p-toluenesulfonate.

The term "pharmaceutically acceptable carrier" refers to an ingredient in a pharmaceutical formulation that is different from the active ingredient and is not toxic to the subject. Pharmaceutically acceptable carriers include, but are not limited to, buffers, stabilizers, or preservatives.

The term "excipient" is an addition, besides the active ingredient, to a pharmaceutical formulation. It may also be referred to as an auxiliary material. For example, binders, fillers, disintegrants and lubricants in tablets; the matrix part in semisolid ointment and cream preparations; preservatives, antioxidants, corrigents, fragrances, cosolvents, emulsifiers, solubilizers, osmotic pressure regulators, colorants and the like in liquid formulations can all be referred to as excipients.

The term "diluent", also referred to as a filler, is used primarily to increase the weight and volume of the tablet. The addition of the diluent not only ensures a certain volume, but also reduces the dose deviation of the main ingredients, and improves the drug's compression moldability and the like. When the drug in the tablet form contains oily components, an absorbent is necessarily added to absorb the oily components so as to maintain a "dry" state and thus facilitate the preparation of the tablet. Examples include starch, lactose, inorganic salts of calcium, microcrystalline cellulose and the like.

The term "subject" or "individual" includes humans and non-human animals. Non-human animals include all vertebrates (e.g., mammals and non-mammals) such as non-human primates (e.g., cynomolgus monkeys), sheep, dogs, cows, fowls, amphibians, and reptiles. Unless indicated, the terms "patient" and "subject" are used interchangeably herein. As used herein, the term "cynomolgus monkey (cyno)" or "cynomolgus" refers to Macaca fascicularis. In certain embodiments, the individual or subject is a human.

"Administrating" or "giving", when applied to animals, humans, experimental subjects, cells, tissue, organs, or biological fluids, refers to contact of an exogenous drug, a therapeutic agent, a diagnostic agent, or a composition with the animals, humans, subjects, cells, tissue, organs, or biological fluids.

The term "sample" refers to a collection of fluids, cells, or tissue isolated from a subject, as well as fluids, cells, or tissue present in a subject. Exemplary samples are biological fluids (such as blood; serum; serosal fluids; plasma; lymph; urine; saliva; cystic fluids; tears; excretions; sputum; mucosal secretions of secretory tissue and organs; vaginal secretions; ascites; fluids in the pleura, pericardium, peritoneum, abdominal cavity, and other body cavities; fluids collected from bronchial lavage; synovial fluids; liquid solutions in contact with a subject or biological source, e.g., cell and organ culture media (including cell or organ conditioned media); lavage fluids; and the like), tissue biopsy samples, fine needle punctures, surgically excised tissues, organ cultures, or cell cultures.

"Treatment" or "treat" (and grammatical variations thereof) refers to clinical intervention in an attempt to alter the pathological course of the treated individual, which may be performed either for prophylaxis or during the course of clinical pathology. Desirable effects of the treatment include, but are not limited to, preventing the occurrence or recurrence of a disease, alleviating symptoms, alleviating/reducing any direct or indirect pathological consequences of the disease, preventing metastasis, decreasing the rate of disease progression, ameliorating or alleviating the disease state, and regressing or improving prognosis. In some embodiments, the antibody of the present disclosure is used to delay the development of or slow the progression of a disease.

"Effective amount" is generally an amount sufficient to reduce the severity and/or frequency of symptoms, eliminate symptoms and/or underlying causes, prevent the appearance of symptoms and/or their underlying causes, and/or ameliorate or improve damage caused by or associated with a disease state. In some embodiments, the effective amount is a therapeutically effective amount or a prophylactically effective amount. "Therapeutically effective amount" is an amount sufficient to treat a disease state or symptom, particularly a state or symptom associated with the disease

state, or to otherwise prevent, hinder, delay, or reverse the progression of the disease state or any other undesirable symptoms associated with the disease in any way. "Prophylactically effective amount" is an amount that, when administered to a subject, will have a predetermined prophylactic effect, e.g., preventing or delaying the onset (or recurrence) of the disease state, or reducing the likelihood of the onset (or recurrence) of the disease state or associated symptoms. A complete therapeutic or prophylactic effect does not necessarily occur after administration of one dose and may occur after administration of a series of doses. Thus, a therapeutically or prophylactically effective amount may be administered in one or more doses. "Therapeutically effective amount" and "prophylactically effective amount" may vary depending on a variety of factors such as the disease state, age, sex, and weight of the individual, and the ability of a therapeutic agent or combination of therapeutic agents to elicit a desired response in the individual. Exemplary indicators of an effective therapeutic agent or combination of therapeutic agents include, for example, improved health condition of a patient.

II. Description of specific embodiments

**A. Exemplary anti-DLL3 antibodies**

In one aspect, the present disclosure provides an antibody that binds to DLL3. In one aspect, an isolated antibody that binds to DLL3 is provided. In one aspect, the present disclosure provides an antibody that specifically binds to DLL3.

In certain embodiments, the aforementioned anti-DLL3 antibody has at least one of the following properties:

a) the anti-DLL3 antibody binding to human DLL3 or an epitope thereof with a KD value of ≤3 nM, ≤2 nM, ≤1 nM, ≤0.9 nM, ≤0.8 nM, ≤0.7 nM, ≤0.6 nM, ≤0.5 nM, or ≤0.4 nM, as determined by Biacore;
b) the anti-DLL3 antibody binding to DLL3-expressing H1184 cells with an EC50 of ≤3 nM, an EC50 of ≤2 nM, an EC50 of ≤1 nM, an EC50 of ≤0.5 nM, an EC50 of ≤0.2 nM, an EC50 of ≤0.1 nM, an EC50 of ≤0.09 nM, an EC50 of ≤0.08 nM, an EC50 of ≤0.07 nM, an EC50 of ≤0.06 nM, as determined by FACS; and
c) the anti-DLL3 antibody being capable of being endocytosed by a DLL3-expressing cell; and
d) the anti-DLL3 antibody binding to DLL3 or an epitope thereof with an EC50 of ≤0.1 nM (≤0.09 nM, ≤0.08 nM, ≤0.07 nM, ≤0.06 nM, ≤0.05 nM, or ≤0.04 nM), as determined by ELISA; and
e) the anti-DLL3 antibody recognizing a different DLL3 epitope than a positive antibody (e.g., BI-764532) does.

In one aspect, the present disclosure provides an anti-DLL3 antibody comprising a group selected from the group consisting of the following:

(i) a HCDR1 comprising the amino acid sequence of SEQ ID NO: 22; (ii) a HCDR2 comprising the amino acid sequence of SEQ ID NO: 23; (iii) a HCDR3 comprising the amino acid sequence of SEQ ID NO: 57; (iv) a LCDR1 comprising the amino acid sequence of SEQ ID NO: 25; (v) a LCDR2 comprising the amino acid sequence of SEQ ID NO: 26; and (vi) a LCDR3 comprising the amino acid sequence of SEQ ID NO: 27, wherein, SEQ ID NO: 57 is represented by PLYX$_1$YGRSYNX$_2$VAY, wherein $X_1$ is Y or H; $X_2$ is A or G; or
(i) a HCDR1 comprising the amino acid sequence of SEQ ID NO: 16; (ii) a HCDR2 comprising the amino acid sequence of SEQ ID NO: 17; (iii) a HCDR3 comprising the amino acid sequence of SEQ ID NO: 18; (iv) a LCDR1 comprising the amino acid sequence of SEQ ID NO: 19; (v) a LCDR2 comprising the amino acid sequence of SEQ ID NO: 20; and (vi) a LCDR3 comprising the amino acid sequence of SEQ ID NO: 21. In one aspect, the present disclosure provides an anti-DLL3 antibody comprising a group selected from the group consisting of the following:
(i) a HCDR1 comprising the amino acid sequence of SEQ ID NO: 22; (ii) a HCDR2 comprising the amino acid sequence of SEQ ID NO: 23; (iii) a HCDR3 comprising the amino acid sequence of SEQ ID NO: 24; (iv) a LCDR1 comprising the amino acid sequence of SEQ ID NO: 25; (v) a LCDR2 comprising the amino acid sequence of SEQ ID NO: 26; and (vi) a LCDR3 comprising the amino acid sequence of SEQ ID NO: 27; or
(i) a HCDR1 comprising the amino acid sequence of SEQ ID NO: 22; (ii) a HCDR2 comprising the amino acid sequence of SEQ ID NO: 23; (iii) a HCDR3 comprising the amino acid sequence of SEQ ID NO: 30; (iv) a LCDR1 comprising the amino acid sequence of SEQ ID NO: 25; (v) a LCDR2 comprising the amino acid sequence of SEQ ID NO: 26; and (vi) a LCDR3 comprising the amino acid sequence of SEQ ID NO: 27; or
(i) a HCDR1 comprising the amino acid sequence of SEQ ID NO: 22; (ii) a HCDR2 comprising the amino acid sequence of SEQ ID NO: 23; (iii) a HCDR3 comprising the amino acid sequence of SEQ ID NO: 31; (iv) a LCDR1 comprising the amino acid sequence of SEQ ID NO: 25; (v) a LCDR2 comprising the amino acid sequence of SEQ ID NO: 26; and (vi) a LCDR3 comprising the amino acid sequence of SEQ ID NO: 27. In one aspect, the present disclosure provides an anti-DLL3 antibody, comprising the HCDRs in a heavy chain variable region of SEQ ID NO: 14 and the LCDRs in a light chain variable region of SEQ ID NO: 15; or

comprising the HCDRs of a heavy chain variable region of SEQ ID NO: 12 and the LCDRs of a light chain variable region of SEQ ID NO: 13; or
comprising the HCDRs of a heavy chain variable region of SEQ ID NO: 52 and the LCDRs of a light chain variable region of SEQ ID NO: 15; or
comprising the HCDRs of a heavy chain variable region of SEQ ID NO: 53 and the LCDRs of a light chain variable region of SEQ ID NO: 15.

In some embodiments, the CDR sequences are obtained according to numbering methods well known in the art, including, but not limited to, Kabat, Chothia, IMGT, AbM, and Contact.

In one aspect, the present disclosure provides an anti-DLL3 antibody comprising:

a HCDR1 the sequence of which is set forth in SEQ ID NO: 22 or, compared to SEQ ID NO: 22, comprises 3, 2, or 1 amino acid mutation;
a HCDR2 the sequence of which is set forth in SEQ ID NO: 23 or, compared to SEQ ID NO: 23, comprises 3, 2, or 1 amino acid mutation;
a HCDR3 the sequence of which is set forth in SEQ ID NO: 24 or, compared to SEQ ID NO: 24, comprises 3, 2, or 1 amino acid mutation;
a LCDR1 the sequence of which is set forth in SEQ ID NO: 25 or, compared to SEQ ID NO: 25, comprises 3, 2, or 1 amino acid mutation;
a LCDR2 the sequence of which is set forth in SEQ ID NO: 26 or, compared to SEQ ID NO: 26, comprises 3, 2, or 1 amino acid mutation; and
a LCDR3 the sequence of which is set forth in SEQ ID NO: 27 or, compared to SEQ ID NO: 27, comprises 3, 2, or 1 amino acid mutation.

In one aspect, the present disclosure provides an anti-DLL3 antibody comprising:

a HCDR1 the sequence of which is set forth in SEQ ID NO: 22 or, compared to SEQ ID NO: 22, comprises 3, 2, or 1 amino acid mutation;
a HCDR2 the sequence of which is set forth in SEQ ID NO: 23 or, compared to SEQ ID NO: 23, comprises 3, 2, or 1 amino acid mutation;
a HCDR3 the sequence of which is set forth in SEQ ID NO: 30 or, compared to SEQ ID NO: 30, comprises 3, 2, or 1 amino acid mutation;
a LCDR1 the sequence of which is set forth in SEQ ID NO: 25 or, compared to SEQ ID NO: 25, comprises 3, 2, or 1 amino acid mutation;
a LCDR2 the sequence of which is set forth in SEQ ID NO: 26 or, compared to SEQ ID NO: 26, comprises 3, 2, or 1 amino acid mutation; and
a LCDR3 the sequence of which is set forth in SEQ ID NO: 27 or, compared to SEQ ID NO: 27, comprises 3, 2, or 1 amino acid mutation.

In one aspect, the present disclosure provides an anti-DLL3 antibody comprising:

a HCDR1 the sequence of which is set forth in SEQ ID NO: 22 or, compared to SEQ ID NO: 22, comprises 3, 2, or 1 amino acid mutation;
a HCDR2 the sequence of which is set forth in SEQ ID NO: 23 or, compared to SEQ ID NO: 23, comprises 3, 2, or 1 amino acid mutation;
a HCDR3 the sequence of which is set forth in SEQ ID NO: 31 or, compared to SEQ ID NO: 31, comprises 3, 2, or 1 amino acid mutation;
a LCDR1 the sequence of which is set forth in SEQ ID NO: 25 or, compared to SEQ ID NO: 25, comprises 3, 2, or 1 amino acid mutation;
a LCDR2 the sequence of which is set forth in SEQ ID NO: 26 or, compared to SEQ ID NO: 26, comprises 3, 2, or 1 amino acid mutation; and
a LCDR3 the sequence of which is set forth in SEQ ID NO: 27 or, compared to SEQ ID NO: 27, comprises 3, 2, or 1 amino acid mutation.

In one aspect, the present disclosure provides an anti-DLL3 antibody comprising:

a HCDR1 the sequence of which is set forth in SEQ ID NO: 16 or, compared to SEQ ID NO: 16, comprises 3, 2, or 1 amino acid mutation;

a HCDR2 the sequence of which is set forth in SEQ ID NO: 17 or, compared to SEQ ID NO: 17, comprises 3, 2, or 1 amino acid mutation;
a HCDR3 the sequence of which is set forth in SEQ ID NO: 18 or, compared to SEQ ID NO: 18, comprises 3, 2, or 1 amino acid mutation;
a LCDR1 the sequence of which is set forth in SEQ ID NO: 19 or, compared to SEQ ID NO: 19, comprises 3, 2, or 1 amino acid mutation;
a LCDR2 the sequence of which is set forth in SEQ ID NO: 20 or, compared to SEQ ID NO: 20, comprises 3, 2, or 1 amino acid mutation; and
a LCDR3 the sequence of which is set forth in SEQ ID NO: 21 or, compared to SEQ ID NO: 21, comprises 3, 2, or 1 amino acid mutation.

In one aspect, the present disclosure provides an anti-DLL3 antibody comprising a heavy chain variable region and a light chain variable region, wherein:

the heavy chain variable region comprises a HCDR1, a HCDR2, and a HCDR3 set forth in SEQ ID NO: 22, SEQ ID NO: 23, and SEQ ID NO: 24, respectively; and
the light chain variable region comprises a LCDR1, a LCDR2, and a LCDR3 set forth in SEQ ID NO: 25, SEQ ID NO: 26, and SEQ ID NO: 27, respectively.

In one aspect, the present disclosure provides an anti-DLL3 antibody comprising a heavy chain variable region and a light chain variable region, wherein:

the heavy chain variable region comprises a HCDR1, a HCDR2, and a HCDR3 set forth in SEQ ID NO: 22, SEQ ID NO: 23, and SEQ ID NO: 30, respectively; and
the light chain variable region comprises a LCDR1, a LCDR2, and a LCDR3 set forth in SEQ ID NO: 25, SEQ ID NO: 26, and SEQ ID NO: 27, respectively.

In one aspect, the present disclosure provides an anti-DLL3 antibody comprising a heavy chain variable region and a light chain variable region, wherein:

the heavy chain variable region comprises a HCDR1, a HCDR2, and a HCDR3 set forth in SEQ ID NO: 22, SEQ ID NO: 23, and SEQ ID NO: 31, respectively; and
the light chain variable region comprises a LCDR1, a LCDR2, and a LCDR3 set forth in SEQ ID NO: 25, SEQ ID NO: 26, and SEQ ID NO: 27, respectively.

In one aspect, the present disclosure provides an anti-DLL3 antibody comprising a heavy chain variable region and a light chain variable region, wherein:

the heavy chain variable region comprises a HCDR1, a HCDR2, and a HCDR3 set forth in SEQ ID NO: 16, SEQ ID NO: 17, and SEQ ID NO: 18, respectively; and
the light chain variable region comprises a LCDR1, a LCDR2, and a LCDR3 set forth in SEQ ID NO: 19, SEQ ID NO: 20, and SEQ ID NO: 21, respectively.

In another aspect, the heavy chain variable region of the anti-DLL3 antibody comprises a sequence having at least 90%, 91%, 92%, 93%, 94%, 95%, 96%, 97%, 98%, 99%, or 100% identity to any one of the amino acid sequences of SEQ ID NO: 14, 50, 51, 52, 53, or 54. In certain embodiments, the sequence has a substitution (e.g., a conservative substitution), an insertion, or a deletion, but the anti-DLL3 antibody comprising the sequence retains the capacity to bind to DLL3. In certain embodiments, the substitution, insertion, or deletion occurs in a region outside the CDRs (i.e., in an FR).

In another aspect, the light chain variable region of the anti-DLL3 antibody comprises a sequence having at least 90%, 91%, 92%, 93%, 94%, 95%, 96%, 97%, 98%, 99%, or 100% identity to any one of the amino acid sequences of SEQ ID NO: 15, 55, or 56. In certain embodiments, the sequence has a substitution (e.g., a conservative substitution), an insertion, or a deletion, but the anti-DLL3 antibody comprising the sequence retains the capacity to bind to DLL3. In certain embodiments, the substitution, insertion, or deletion occurs in a region outside the CDRs (i.e., in an FR).

In another aspect, the heavy chain variable region of the anti-DLL3 antibody comprises a sequence having at least 90%, 91%, 92%, 93%, 94%, 95%, 96%, 97%, 98%, 99%, or 100% identity to any one of the amino acid sequences of SEQ ID NO: 12, 32, 33, 34, 35, 36, 37, 38, 39, 40, 41, 42, 43, or 44. In certain embodiments, the sequence has a substitution (e.g., a conservative substitution), an insertion, or a deletion, but the anti-DLL3 antibody comprising the sequence retains the capacity to bind to DLL3. In certain embodiments, the substitution, insertion, or deletion occurs in

a region outside the CDRs (i.e., in an FR).

In another aspect, the light chain variable region of the anti-DLL3 antibody comprises a sequence having at least 90%, 91%, 92%, 93%, 94%, 95%, 96%, 97%, 98%, 99%, or 100% identity to any one of the amino acid sequences of SEQ ID NO: 13, 45, 46, 47, 48, or 49. In certain embodiments, the sequence has a substitution (e.g., a conservative substitution), an insertion, or a deletion, but the anti-DLL3 antibody comprising the sequence retains the capacity to bind to DLL3. In certain embodiments, the substitution, insertion, or deletion occurs in a region outside the CDRs (i.e., in an FR).

In some embodiments, the aforementioned anti-DLL3 antibody comprises a heavy chain variable region set forth in any one of SEQ ID NO: 50, 14, 51, 52, 53, or 54, and/or a light chain variable region set forth in any one of SEQ ID NO: 55, 15, or 56. In some embodiments, the aforementioned anti-DLL3 antibody comprises a heavy chain variable region set forth in any one of SEQ ID NO: 43, 12, 32, 33, 34, 35, 36, 37, 38, 39, 40, 41, 42, or 44, and/or a light chain variable region set forth in any one of SEQ ID NO: 48, 13, 45, 46, 47, or 49.

In some embodiments, the aforementioned anti-DLL3 antibody comprises a heavy chain variable region set forth in SEQ ID NO: 14, and/or a light chain variable region set forth in any one of SEQ ID NO: 15.

In some embodiments, the aforementioned anti-DLL3 antibody comprises a heavy chain variable region selected from the group consisting of any one of SEQ ID NOs: 50, 51, 52, 53, and 54, and/or a light chain variable region set forth in any one of SEQ ID NO: 55 or 56.

In some embodiments, the aforementioned anti-DLL3 antibody comprises a heavy chain variable region set forth in SEQ ID NO: 12, and/or a light chain variable region set forth in SEQ ID NO: 13.

In some embodiments, the aforementioned anti-DLL3 antibody comprises a heavy chain variable region selected from the group consisting of any one of SEQ ID NOs: 43, 32, 33, 34, 35, 36, 37, 38, 39, 40, 41, 42, and 44, and/or a light chain variable region selected from the group consisting of any one of SEQ ID NOs: 48, 45, 46, 47, and 49. In some embodiments, the aforementioned anti-DLL3 antibody comprises a heavy chain variable region set forth in SEQ ID NO: 50, and a light chain variable region set forth in SEQ ID NO: 55.

In some embodiments, the aforementioned anti-DLL3 antibody comprises a heavy chain variable region set forth in SEQ ID NO: 43, and a light chain variable region set forth in SEQ ID NO: 48.

In some embodiments, the aforementioned anti-DLL3 antibody comprises a heavy chain constant region and a light chain constant region.

In some embodiments, the sequence of the heavy chain constant region of the aforementioned anti-DLL3 antibody is set forth in SEQ ID NO: 28.

In some embodiments, the sequence of the light chain constant region of the aforementioned anti-DLL3 antibody is set forth in SEQ ID NO: 29.

In some embodiments, the aforementioned anti-DLL3 antibody comprises a heavy chain and a light chain, wherein:

the heavy chain comprises an amino acid sequence having at least 85% identity to SEQ ID NO: 60, and/or the light chain comprises an amino acid sequence having at least 85% identity to SEQ ID NO: 61; or

the heavy chain comprises an amino acid sequence having at least 85% identity to SEQ ID NO: 58, and/or the light chain comprises an amino acid sequence having at least 85% identity to SEQ ID NO: 59.

In some embodiments, the aforementioned anti-DLL3 antibody comprises a heavy chain set forth in SEQ ID NO: 60, and a light chain set forth in SEQ ID NO: 61.

In some embodiments, the aforementioned anti-DLL3 antibody comprises a heavy chain set forth in SEQ ID NO: 58, and a light chain set forth in SEQ ID NO: 59.

## B. Antibody structure

In certain embodiments, the antibody provided herein is a full-length antibody.

In certain embodiments, the antibody provided herein is an antibody fragment.

In one embodiment, the antibody fragment is a Fab, Fab', Fab'-SH, or $F(ab')_2$ fragment, particularly a Fab fragment. "Fab" is a monovalent fragment consisting of the VL, VH, CL, and CH1 domains. "Fab fragment" may be produced by papain cleavage of an antibody. "Fab'" comprises VL, CL, and VH and CH1, and also comprises the region between the CH1 and CH2 domains, so that an interchain disulfide bond can be formed between the two heavy chains of two Fab' fragments to form a F(ab')2 molecule. "Fab'-SH" is a Fab' fragment in which the cysteine residues of the constant domains have a free sulfhydryl group. "$F(ab')_2$" is a bivalent fragment comprising two Fab fragments linked by a disulfide bond at the hinge region.

In another embodiment, the antibody fragment is a diabody, a triabody, or a tetrabody. Diabodies are antibody fragments having two antigen-binding sites. The fragment comprises a VH and a VL linked in the same polypeptide chain (VH-VL). By using a linker that is too short to allow pairing between two domains on the same chain, the domains

are forced to pair with the complementary domains of another chain, thereby resulting in two antigen-binding sites. The two antigens may be identical or different. In another embodiment, the antibody fragment is a single-chain Fab fragment. "Single-chain Fab fragment" or "scFab" is a polypeptide consisting of VH, CH1, VL, CL, and a linker, wherein the domains and the linker have one of the following orders in the N-terminus to C-terminus direction: a) VH-CH1-linker-VL-CL, b) VL-CL-linker-VH-CH1, c) VH-CL-linker-VL-CH1, or d) VL-CH1-linker-VH-CL. In one embodiment, the linker is a polypeptide of at least 30 amino acids. In another embodiment, the linker is a polypeptide of between 32 and 50 amino acids. The single-chain Fab fragment is stabilized via the natural disulfide bond between CL and CH1. In addition, these single-chain Fab molecules may be further stabilized by generating interchain disulfide bonds through insertion of cysteine residues (e.g., position 44 in the heavy chain variable region and position 100 in the light chain variable region, according to Kabat numbering).

In another embodiment, the antibody fragment is a single-chain variable fragment (scFv). "scFv" is a fusion protein comprising at least one antibody fragment comprising a light chain variable region and at least one antibody fragment comprising a heavy chain variable region, wherein the light and heavy chain variable regions are contiguously linked by a short flexible polypeptide linker and are capable of being expressed as a single-chain polypeptide, and wherein the scFv retains the specificity of the intact antibody from which it is derived. Unless specifically indicated, an scFv may have VL and VH variable regions in any order herein; for example, with respect to the N-terminus and C-terminus of the polypeptide, the scFv may comprise VL-linker-VH or may comprise VH-linker-VL.

In another embodiment, the antibody fragment is an Fd fragment consisting of the VH and CH1 domains.

In another embodiment, the antibody fragment is an Fv fragment consisting of the VH and VL domains of a single arm of the antibody.

In another embodiment, the antibody fragment is a dsFv obtained by linking polypeptides in which one amino acid residue of each of VH and VL is substituted with a cysteine residue by a disulfide bond between the cysteine residues. The amino acid residues substituted with cysteine residues can be selected according to known methods (Protein Engineering, 7:697 (1994)) based on prediction of the three-dimensional structure of the antibody.

In another embodiment, the antibody fragment is a single-domain antibody. Single-domain antibodies are antibody fragments comprising all or a portion of the heavy chain variable domain or all or a portion of the light chain variable domain of an antibody.

In another embodiment, the antibody fragment is a domain antibody (dAb); see, for example, U.S. Patent No. 6,248,516. Domain antibodies (dAbs) are functional binding domains of antibodies, corresponding to the variable regions of the heavy (VH) or light (VL) chains of human antibodies. dABs have a molecular weight of about 13 kDa, or are less than one-tenth the size of an intact antibody. dABs are well expressed in a variety of hosts including bacterial, yeast, and mammalian cell systems. In addition, dAbs are highly stable and retain activity even after being subjected to harsh conditions, such as lyophilization or heat denaturation. See, for example, U.S. Patents 6,291,158; 6,582,915; 6,593,081; and 6,172,197; U.S. Serial No. 2004/0110941; European Patent 0368684; U.S. Patent 6,696,245; WO04/058821; WO04/003019 and WO03/002609. In certain embodiments, the antibody provided herein is a chimeric antibody. In one example, a chimeric antibody comprises a non-human variable region (e.g., a variable region derived from a mouse, rat, hamster, domestic rabbit, or non-human primate, such as a monkey) and a human constant region. In another example, a chimeric antibody is a "class switched" antibody in which the class or subclass has been changed from the class or subclass of the parent antibody.

In certain embodiments, the antibody is a humanized antibody. Typically, non-human antibodies are humanized to reduce immunogenicity to humans while retaining the specificity and affinity of the parent non-human antibody. Generally, a humanized antibody comprises one or more variable regions in which the CDRs or portions thereof are derived from a non-human antibody and the FRs or portions thereof are derived from a human antibody. Optionally, a humanized antibody will also comprise a portion of a human constant region. In some embodiments, some FR residues in a humanized antibody may be replaced with corresponding residues from a non-human antibody (e.g., an antibody that provides CDR sequences).

Humanized antibodies and methods for their production are reviewed, e.g., in Almagro and Fransson, Front. Biosci. 13:1619-1633 (2008), and further described, e.g., in Riechmann et al., Nature 332:323-329 (1988); Queen et al., Proc. Nat'l Acad. Sci. USA 86:10029-10033 (1989); U.S. Patent Nos. 5,821,337, 7,527,791, 6,982,321, and 7,087,409; Kashmiri et al., Methods 36:25-34 (2005) (describing specificity-determining region (SDR) grafting); Padlan, Mol. Immunol. 28:489-498 (1991) (describing "resurfuacing"); Dall' Acqua et al., Methods 36:43-60 (2005) (describing "FR shuffling"); and Osbourn et al., Methods 36:61-68 (2005) and Klimka et al., Br. J. Cancer 83:252-260 (2000) (describing the "guided selection" method for FR shuffling). Human framework regions that can be used for humanization include, but are not limited to: framework regions selected using the "best-fit" method (see, e.g., Sims et al., J. Immunol. 151:2296 (1993)); framework regions derived from the consensus sequence of human antibodies of a particular subgroup of light or heavy chain variable regions (see, e.g., Carter et al., Proc. Natl. Acad. Sci. USA, 89:4285 (1992); and Presta et al., J. Immunol., 151:2623 (1993)); human mature (somatically mutated) framework regions or human germline framework regions (see, e.g., Almagro and Fransson, Front. Biosci. 13:1619-1633 (2008)); and framework regions obtained by

screening FR libraries (see, e.g., Baca et al., J. Biol. Chem. 272:10678-10684 (1997) and Rosok et al., J. Biol. Chem. 271:22611-22618 (1996)).

### C. Modification of antibodies

In certain embodiments, amino acid sequence variants of the antibody provided herein are encompassed. For example, it may be desirable to improve the binding affinity and/or other biological properties of the antibody. Amino acid sequence variants of an antibody can be prepared by introducing appropriate modifications into the nucleotide sequence encoding the antibody, or by peptide synthesis. Such modifications include, for example, deletions from, and/or insertions into and/or substitutions of residues within the amino acid sequence of the antibody. Any combination of deletions, insertions, and substitutions can be made to obtain the final construct, provided that the final construct possesses the desired characteristics, e.g., antigen binding.

a) Substitution, insertion, and deletion variants

In certain embodiments, antibody variants having one or more amino acid substitutions are provided. Positions of interest for substitutional mutagenesis include CDRs and FRs. Conservative substitutions are shown in Table 2 under the heading of "Preferred substitution". More substantial changes are provided in Table 2 under the heading of "Exemplary substitution", and as further described below with reference to amino acid side chain classes. Amino acid substitutions can be introduced into an antibody of interest and the products are screened for a desired activity, e.g., retained/improved antigen binding, reduced immunogenicity, or improved ADCC or CDC.

Table 2

| Original residue | Exemplary substitution | Preferred substitution |
|---|---|---|
| Ala(A) | Val; Leu; Ile | Val |
| Arg(R) | Lys; Gln; Asn | Lys |
| Asn(N) | Gln; His; Asp,Lys; Arg | Gln |
| Asp(D) | Glu; Asn | Glu |
| Cys(C) | Ser; Ala | Ser |
| Gln(Q) | Asn; Glu | Asn |
| Glu(E) | Asp; Gln | Asp |
| Gly(G) | Ala | Ala |
| His(H) | Asn; Gln; Lys; Arg | Arg |
| Ile(I) | Leu; Val; Met; Ala; Phe; norleucine | Leu |
| Leu(L) | Norleucine; Ile; Val; Met; Ala; Phe | Ile |
| Lys(K) | Arg; Gln; Asn | Arg |
| Met(M) | Leu; Phe; Ile | Leu |
| Phe(F) | Trp; Leu; Val; Ile; Ala; Tyr | Tyr |
| Pro(P) | Ala | Ala |
| Ser(S) | Thr | Thr |
| Thr(T) | Ser | Ser |
| Trp(W) | Tyr; Phe | Tyr |
| Tyr(Y) | Trp; Phe; Thr; Ser | Phe |
| Val(V) | Ile; Leu; Met; Phe; Ala; norleucine | Leu |

According to common side-chain properties, amino acids can be grouped as follows:

(1) hydrophobic: norleucine, Met, Ala, Val, Leu, Ile;

(2) neutral, hydrophilic: Cys, Ser, Thr, Asn, Gln;
(3) acidic: Asp, Glu;
(4) basic: His, Lys, Arg;
(5) residues affecting chain orientation: Gly, Pro;
(6) aromatic: Trp, Tyr, Phe.

Non-conservative substitutions will involve substituting a member of one of these classes for a member of another class.

In certain embodiments, substitutions, insertions or deletions may occur within one or more CDRs so long as such changes do not substantially reduce the ability of the antibody to bind to the antigen. For example, conservative changes (e.g., conservative substitutions as provided herein) that do not substantially reduce binding affinity may be made in CDRs. Such changes may be, for example, outside of the antigen-contacting residues in CDRs. In certain embodiments of the variant VH and VL sequences provided above, each CDR is unchanged or contains no more than 1, 2 or 3 amino acid substitutions.

A useful method for identifying residues or regions of an antibody that can be used as mutagenesis targets is called "alanine scanning mutagenesis", as described in Cunningham and Wells (1989) Science, 244:1081-1085. In this method, a residue or group of residues (e.g., charged residues such as Arg, Asp, His, Lys and Glu) are identified and replaced with a neutral or negatively charged amino acid (e.g., Ala or polyalanine) to determine whether the interaction of the antibody with the antigen is affected. Further substitutions may be introduced at amino acid locations that show functional sensitivity to the initial substitutions. In addition, a crystal structure of an antigen-antibody complex may be studied to identify contact points between the antibody and antigen. These contact residues and neighboring residues may be targeted or eliminated as substitution candidates. Variants may be screened to determine whether they contain the desired properties.

Amino acid sequence insertions include amino- and/or carboxyl-terminal fusions ranging in length from 1 residue to polypeptides of 100 or more residues, and intrasequence insertions of single or multiple amino acid residues. Examples of terminal insertions include an antibody with an N-terminal methionyl residue. Other insertional variants of the antibody molecule include the fusion of the N- or C-terminus of the antibody to an enzyme or a polypeptide that increases the serum half-life of the antibody.

b) Fc region modifications

In certain embodiments, one or more amino acid modifications may be introduced into the Fc region of the antibody provided herein.

In some embodiments, the one or more amino acid modifications can reduce binding of Fc to an Fc receptor, e.g., its binding to an Fcy receptor, and reduce or eliminate effector function. In some embodiments, the engineered Fc region shows no less than 50%, 80%, 90%, or 95% reduction in binding affinity for an Fc receptor as compared to the native Fc region. In some embodiments, the Fc receptor is a human Fcγ receptor, e.g., FcγRI, FcγRIIa, FcγRIIB, or FcγRIIIa. In some embodiments, the engineered Fc region also has reduced binding affinity for a complement (e.g., C1q) as compared to the native Fc region. In some embodiments, the engineered Fc region has enhanced binding affinity for a neonatal Fc receptor (FcRn) as compared to the native Fc region; for example, M252Y/S254T/T256E mutations are introduced into the Fc region. In some embodiments, the engineered Fc region has reduced effector functions, which may include, but are not limited to, one or more of the following: reduced complement-dependent cytotoxicity (CDC), reduced antibody-dependent cell-mediated cytotoxicity (ADCC), reduced antibody-dependent cellular phagocytosis (ADCP), reduced cytokine secretion, reduced immune complex-mediated antigen uptake by antigen presenting cells, reduced binding to NK cells, reduced binding to macrophages, reduced binding to monocytes, reduced binding to polymorphonuclear cells, reduced direct signaling-induced apoptosis, reduced dendritic cell maturation, and reduced T cell priming. For the $IgG_1$ Fc region, amino acid residue substitutions at positions such as positions 238, 265, 269, 270, 297, 327, and 329 can reduce effector functions. In some embodiments, the Fc region is a human $IgG_1$ Fc region, and the amino acid residues at positions 234 and 235 are A, as numbered according to the EU index. For the $IgG_4$ Fc region, amino acid residue substitutions at positions such as position 228 can reduce effector functions.

In certain embodiments, the antibody comprises one or more amino acid substitutions that improve ADCC, e.g., substitutions at positions 298, 333, and/or 334 (using the EU numbering scheme) of the Fc region.

In certain embodiments, the Fc domain of the antibody herein comprises "knobs-into-holes" mutations. "Knobs-into-holes" is a design strategy for engineering antibody heavy chain homodimers for heterodimerization (e.g., for the effective production of bispecific antibodies, multispecific antibodies, or one-armed antibodies). Generally, such technology involves introducing a protuberance ("knob") at the interface of a first polypeptide (such as a first CH3 domain in a first antibody heavy chain) and a corresponding cavity ("hole") at the interface of a second polypeptide (such as a second CH3 domain in a second antibody heavy chain), such that the protuberance can be positioned in the cavity so

as to promote heterodimer formation and hinder homodimer formation. Protuberances are constructed by replacing small amino acid side chains from the interface of the first polypeptide (such as a first CH3 domain in a first antibody heavy chain) with larger side chains (e.g. arginine, phenylalanine, tyrosine, or tryptophan). Compensatory cavities of identical or similar size to the protuberances are created in the interface of the second polypeptide (such as a second CH3 domain in a second antibody heavy chain) by replacing large amino acid side chains with smaller side chains (e.g. alanine, serine, valine, or threonine). Protuberances and cavities can be generated by altering the nucleic acid encoding the polypeptides (e.g., by site-specific mutagenesis) or by peptide synthesis. In some embodiments, a knob modification comprises the amino acid substitution T366W in one of the two subunits of the Fc domain, and a hole modification comprises the amino acid substitutions T366S, L368A, and Y407V in the other one of the two subunits of the Fc domain. In some embodiments, the subunit of the Fc domain comprising the knob modification additionally comprises the amino acid substitution S354C, and the subunit of the Fc domain comprising the hole modification additionally comprises the amino acid substitution Y349C. Introduction of the two cysteine residues results in the formation of a disulfide bridge between the two subunits of the Fc region, thus further stabilizing the dimer (Carter, J. Immunol. Methods 248:7-15 (2001)). Exemplary combinations of knobs-into-holes mutations include, but are not limited to, those shown in Table 3.

Table 3

| | | | | | | | | |
|---|---|---|---|---|---|---|---|---|
| Fc monomer 1 | Y407T | Y407A | F405A | T394S | T366S L368A Y407V | T394W Y407T | T394W Y407A | T366W T394S |
| Fc monomer 2 | T366Y | T366W | T394W | F405W | T366W | T366Y F405A | T366W F405W | F405W Y407A |

Details regarding "knobs-into-holes" technology are described in, e.g., U.S. Patent No. 5,731,168; U.S. Patent No. 7,695,936; WO 2009/089004; US 2009/0182127; Marvin and Zhu, Acta Pharmacologica Sincia (2005) 26(6):649-658; Kontermann, Acta Pharmacologica Sincia (2005) 26:1-9; Ridgway et al., Prot Eng 9:617-621 (1996); and Carter, J Immunol Meth 248:7-15 (2001).

The C-terminus of the Fc region may be an intact C-terminus ending with amino acid residues PGK or a truncated C-terminus, e.g., a truncated C-terminus in which one or two C-terminal amino acid residues have been removed. In a preferred aspect, the C-terminus of the heavy chain is a truncated C-terminus ending with PG. Thus, in some embodiments, a composition of intact antibodies may comprise antibody populations with all K447 residues and/or G446 + K447 residues removed. In some embodiments, a composition of intact antibodies may comprise antibody populations without K447 residue and/or G446 + K447 residue removed. In some embodiments, a composition of intact antibodies comprises a mixture of antibodies with and without K447 residues and/or G446 + K447 residues.

**D. Recombination method**

Anti-DLL3 antibodies can be produced using recombinant methods. For these methods, one or more isolated nucleic acids encoding the antibodies are provided.

In one embodiment, the present disclosure provides an isolated nucleic acid encoding the aforementioned antibody. Such nucleic acids may each independently encode any one of the aforementioned polypeptide chains. In another aspect, the present disclosure provides one or more vectors (e.g., expression vectors) comprising such nucleic acids. In another aspect, the present disclosure provides a host cell comprising such nucleic acids. In one embodiment, provided is a method for preparing an anti-DLL3 antibody, wherein the method comprises culturing a host cell comprising a nucleic acid encoding the antibody, as provided above, under conditions suitable for expression, and optionally collecting the antibody from the host cell (or host cell medium).

For recombinant production of the antibody, the nucleic acid encoding the antibody is isolated and inserted into one or more vectors for further cloning and/or expression in a host cell. Such nucleic acids can be readily isolated and sequenced using conventional procedures, or produced by recombination methods or obtained by chemical synthesis. Suitable host cells for cloning or expressing a vector encoding the antibody include prokaryotic or eukaryotic cells described herein. For example, it can be produced in bacteria, particularly when glycosylation and Fc effector functions are not needed. After expression, it can be isolated from the bacterial cell paste in a soluble fraction and can be further purified.

### E. Assays

The polypeptide or conjugate provided herein can be identified, screened, or characterized for their physical/chemical characteristics and/or biological activities by a variety of assays known in the art. In one aspect, the activity of the polypeptide or conjugate of the present disclosure is tested, e.g., by known methods such as ELISA, western blot, and the like.

### F. Treatment method and route of administration

Any of the aforementioned anti-DLL3 antibodies, antibody-drug conjugates comprising same, or pharmaceutically acceptable salts thereof provided herein can be used for treating a disease.

In one aspect, the present disclosure provides use of an anti-DLL3 antibody, an antibody-drug conjugate comprising same, or a pharmaceutically acceptable salt thereof in the preparation of a medicament. In some embodiments, the present disclosure provides use of an anti-DLL3 antibody, an antibody-drug conjugate comprising same, or a pharmaceutically acceptable salt thereof in the preparation of a medicament for treating a tumor or cancer. In some embodiments, the tumor or cancer includes, but is not limited to, lung cancer (e.g., small cell lung cancer, non-small cell lung cancer, and large cell lung cancer), head and neck squamous cell carcinoma, head and neck cancer, brain cancer, neuroglioma, glioblastoma multiforme, neuroblastoma, central nervous system cancer, neuroendocrine tumor, throat cancer, pharyngeal squamous cell carcinoma, oral squamous cell carcinoma, nasopharyngeal cancer, esophageal cancer, thyroid cancer (e.g., medullary thyroid cancer), malignant pleural mesothelioma, breast cancer (e.g., triple-negative breast cancer), liver cancer, hepatobiliary cancer, pancreatic cancer, gastric cancer, gastrointestinal cancer, intestinal cancer, colorectal cancer (e.g., colon cancer and rectal cancer), kidney cancer, clear cell renal cell carcinoma, ovarian cancer, endometrial cancer, cervical cancer, bladder cancer, prostate cancer, testicular cancer, adrenal cancer, glioblastoma, skin cancer, and melanoma; preferably, the tumor or cancer is small cell lung cancer.

In some embodiments, the present disclosure provides use of an anti-DLL3 antibody, an antibody-drug conjugate comprising same, or a pharmaceutically acceptable salt thereof in the preparation of a medicament for treating a DLL3-related disease.

In one such embodiment, the use further comprises administering to a subject a therapeutically effective amount of at least one additional therapeutic agent (e.g., one, two, three, four, five, or six additional therapeutic agents).

In yet another aspect, provided is a pharmaceutical composition comprising the anti-DLL3 antibody or the drug conjugate thereof, e.g., for any of the above pharmaceutical uses or treatment methods. In one embodiment, the pharmaceutical composition comprises any of the polypeptides or drug conjugates provided herein and a pharmaceutically acceptable carrier. In another embodiment, the pharmaceutical composition also comprises at least one additional therapeutic agent.

The anti-DLL3 antibody or the drug conjugate thereof of the present disclosure can be used alone or in combination with other agents for treatment. For example, the antibody of the present disclosure can be co-administered with at least one additional therapeutic agent.

The anti-DLL3 antibody or the drug conjugate thereof of the present disclosure may be administered by any suitable means, including parenteral, intrapulmonary, and intranasal administration, and if required for local treatment, intralesional administration. Parenteral infusion includes intramuscular, intravenous, intra-arterial, intraperitoneal, or subcutaneous administration. Administration may be performed by any suitable route, e.g., by injection, such as intravenous or subcutaneous injection, depending in part on whether the administration is short-term or long-term. Various administration schedules are contemplated herein, including but not limited to, single administration or multiple administrations at multiple time points, bolus administration, and pulse infusion.

The anti-DLL3 antibody or the drug conjugate thereof of the present disclosure will be formulated, administered, and applied in a manner consistent with Good Medical Practice. Factors considered in this context include the specific disorder being treated, the specific mammal being treated, the clinical state of the individual patient, the cause of the disorder, the site of delivery of the agent, the method of administration, the timing of administration, and other factors known to medical practitioners. The anti-DLL3 antibody or the drug conjugate thereof may be formulated together with one or more additional agents. The effective amount of such additional agents depends on the amount present in the pharmaceutical composition, the type of the disorder or treatment, and other factors. These are generally used in the same dosages and routes of administration as described herein, or in about 1% to 99% of the dosages described herein, or in other dosages, and by any route empirically/clinically determined to be appropriate.

For the prevention or treatment of disease, the appropriate dosage of the anti-DLL3 antibody or the drug conjugate thereof of the present disclosure (when used alone or in combination with one or more other additional therapeutic agents) will depend on the type of the disease to be treated, the type of the therapeutic molecule, the severity and course of the disease, whether the therapeutic molecule is administered for preventive or therapeutic purposes, previous therapy, the patient's clinical history and response to the therapeutic molecule, and the discretion of the attending

physician. The therapeutic molecule is suitably administered to a patient in one or a series of treatments.

### G. Product

In another aspect of the present disclosure, provided is a product (e.g., a medication box) comprising materials useful for the treatment, prevention, and/or diagnosis of the above disorders. The product comprises a container and a label or package insert on or associated with the container. Suitable containers include, for example, bottles, vials, syringes, IV solution bags, and the like. The container may be formed from a variety of materials such as glass or plastic.
The container contains the anti-DLL3 antibody or the drug conjugate thereof of the present disclosure, alone or in combination with another composition. The container may have a sterile access port (e.g., the container may be an intravenous solution bag or vial with a stopper). At least one active agent in the composition is the anti-DLL3 antibody or the drug conjugate thereof of the present disclosure. The label or package insert indicates that the composition is used to treat the selected condition.
Further, the product may comprise: (a) a first container containing the anti-DLL3 antibody or the drug conjugate thereof; and (b) a second container containing a composition, wherein the composition comprises an additional cytotoxic agent or otherwise therapeutic agent.
Alternatively or additionally, the product may further comprise a second (or third) container containing a pharmaceutically acceptable buffer. From a commercial and user standpoint, it may further contain other materials as required, including other buffers, diluents, filters, needles, and syringes.

## Examples

The present disclosure is further described below with reference to examples, but these examples are not intended to limit the scope of the present invention.
Experimental methods without specific conditions indicated in the examples or test examples of the present disclosure were generally conducted under conventional conditions or conditions recommended by the manufacturer of the starting materials or commercial products. See Sambrook et al., Molecular Cloning: A Laboratory Manual, Cold Spring Harbor Laboratory Press; Current Protocols in Molecular Biology, Ausubel et al., Greene Publishing Association, Wiley Interscience, NY Reagents without specific origins indicated are commercially available conventional reagents.

### I. Preparation of Antibodies

#### Example 1: Preparation of DLL3 Antigens, Proteins for Detection, and Stably Transfected Cell Strains

Chinese hamster ovary CHO-s cells (Invitrogen, R80007) were transfected with DLL3 genes of different species and human DLL1 and DLL4 genes to construct CHO-s cell strains expressing DLL3 proteins of different species for subsequent screening and identification of antibodies. The amino acid sequences of the related proteins were as follows:

Human DLL3 full-length protein (Uniprot, Q9NYJ7):

MVSPRMSGLLSQTVILALIFLPQTRPAGVFELQIHSFGPGPGPGAPRSPCSARLP
CRLFFRVCLKPGLSEEAAESPCALGAALSARGPVYTEQPGAPAPDLPLPDGLL
QVPFRDAWPGTFSFIIETWREELGDQIGGPAWSLLARVAGRRRLAAGGPWARD
IQRAGAWELRFSYRARCEPPAVGTACTRLCRPRSAPSRCGPGLRPCAPLEDECE
APLVCRAGCSPEHGFCEQPGECRCLEGWTGPLCTVPVSTSSCLSPRGPSSATTG
CLVPGPGPCDGNPCANGGSCSETPRSFECTCPRGFYGLRCEVSGVTCADGPCF
NGGLCVGGADPDSAYICHCPPGFQGSNCEKRVDRCSLQPCRNGGLCLDLGHA
LRCRCRAGFAGPRCEHDLDDCAGRACANGGTCVEGGGAHRCSCALGFGGRD
CRERADPCAARPCAHGGRCYAHFSGLVCACAPGYMGARCEFPVHPDGASAL
PAAPPGLRPGDPQRYLLPPALGLLVAAGVAGAALLLVHVRRRGHSQDAGSRLL
AGTPEPSVHALPDALNNLRTQEGSGDGPSSSVDWNRPEDVDPQGIYVISAPSI

YAREVATPLFPPLHTGRAGQRQHLLFPYPSSILSVK

SEQ ID NO: 1

Cynomolgus monkey DLL3 full-length protein (Uniprot, A0A2K5WSR4):

MVSPRMSRLLSQTVILALIFIPQARPAGVFELQIHSFGPGPGPGAPRSPCSARGP
CRLFFRVCLKPGLSEEAAESPCALGAALSARGPVYTEQPEAPAPDLPLPNGLL
QVPFRDAWPGTFSLIIETWREELGDQIGGPAWSLLARVTRRRRLAAGGPWARD
IQRAGAWELRFSYRARCELPAVGTACTRLCRPRSAPSRCGPGLRPCAPLEDEC
EAPPVCRAGCSLEHGFCEQPGECRCLEGWTGPLCMVPVSTSSCLGLRGPSSTT
TGCLVPGPGPCDGNPCANGGSCSETPGSFECTCPRGFYGLRCEVSGVTCADGP
CFNGGLCVGGADPDSAYICHCPPGFQGSNCEKRVDRCSLQPCRNGGLCLDLG
HALRCRCRAGFAGPRCEHDLDDCAGRACANGGTCVEGGGAHRCSCALGFG
GRNCRERADPCAARPCAHGGRCYAHFSGLVCACAPGYMGARCEFPVHPDGV
SALPAAPPGLRPGDPQRYLLPPALGLLVAAGVAGAALLLVHVRRRGHAQDAG
SRLLAGTPEPSVHALPDALNNLRTQEGPGDVPSSSVDWNRPEDVDSRGIYVIS
APSIYAREVAMPLFPPLHTGRAGQRQNLLFPFPSSILSVK

SEQ ID NO: 2

Rat DLL3 full-length protein (Uniprot, O88671):

MVSLQVSSLPQTLILAFLLPQALPAGVFELQIHSFGPGPGPGTPRSPCNARGPC
RLFFRVCLKPGVSQEAAESLCALGAALSTSGPVYTEQPGVPAAALSLPDGLVR
VPFLDAWPGTFSLIIETWREQLGERAAGPAWNLLARVAGRRRLAAGAPWARD
VQRTGAWELHFSYRARCEPPAVGAACARLCRSRSAPSRCGPGLRPCTPFPDEC
EAPRESLTVCRAGCSPEHGYCEEPDECHCLEGWTGPLCTVPVSTSSCLNSRVS
GPAGTGCLLPGPGPCDGNPCANGGSCSETPGSFECACPRGFYGPRCEVSGVTC
ADGPCFNGGLCVGGEDPDSAYVCHCPPAFQGSNCERRVDRCSLQPCQNGGLC
LDLGHALRCRCRAGFAGPRCEHDLDDCAGRACANGGTCVEGGGARRCSCAL
GFGGRDCRERADPCASRPCAHGGRCYAHFSGLVCACAPGYMGVRCEFAVRP
DGADAVPAAPRGLRQADSQRFLLPPALGLLAAAALAGAALLLIHVRRRGPGR
DTGTRLLSGTREPSVHTLPDALNNLRLQDGAGDGPTSSADWNHPEDGDSRSI
YVIPAPSIYAREA

SEQ ID NO: 3

Mouse DLL3 full-length protein (Uniprot, O88516):

MVSLQVSPLSQTLILAFLLPQALPAGVFELQIHSFGPGPGLGTPRSPCNARGPC
RLFFRVCLKPGVSQEATESLCALGAALSTSVPVYTEHPGESAAALPLPDGLVR
VPFRDAWPGTFSLVIETWREQLGEHAGGPAWNLLARVVGRRRLAAGGPWAR
DVQRTGTWELHFSYRARCEPPAVGAACARLCRSRSAPSRCGPGLRPCTPFPDE
CEAPSVCRPGCSPEHGYCEEPDECRCLEGWTGPLCTVPVSTSSCLNSRVPGPA
STGCLLPGPGPCDGNPCANGGSCSETSGSFECACPRGFYGLRCEVSGVTCADG
PCFNGGLCVGGEDPDSAYVCHCPPGFQGSNCEKRVDRCSLQPCQNGGLCLDL
GHALRCRCRAGFAGPRCEHDLDDCAGRACANGGTCVEGGGSRRCSCALGFG
GRDCRERADPCASRPCAHGGRCYAHFSGLVCACAPGYMGVRCEFAVRPDGA

DAVPAAPRGLRQADPQRFLLPPALGLLVAAGLAGAALLVIHVRRRGPGQDTGT
RLLSGTREPSVHTLPDALNNLRLQDGAGDGPSSSADWNHPEDGDSRSIYVIPA
PSIYAREDWLIQVLF

SEQ ID NO: 4

Human DLL1 full-length protein (Uniprot, O00548):

MGSRCALALAVLSALLCQVWSSGVFELKLQEFVNKKGLLGNRNCCRGGAGP
PPCACRTFFRVCLKHYQASVSPEPPCTYGSAVTPVLGVDSFSLPDGGGADSAF
SNPIRFPFGFTWPGTFSLIIEALHTDSPDDLATENPERLISRLATQRHLTVGEEWS
QDLHSSGRTDLKYSYRFVCDEHYYGEGCSVFCRPRDDAFGHFTCGERGEKVC
NPGWKGPYCTEPICLPGCDEQHGFCDKPGECKCRVGWQGRYCDECIRYPGCL
HGTCQQPWQCNCQEGWGGLFCNQDLNYCTHHKPCKNGATCTNTGQGSYTC
SCRPGYTGATCELGIDECDPSPCKNGGSCTDLENSYSCTCPPGFYGKICELSAM
TCADGPCFNGGRCSDSPDGGYSCRCPVGYSGFNCEKKIDYCSSSPCSNGAKC
VDLGDAYLCRCQAGFSGRHCDDNVDDCASSPCANGGTCRDGVNDFSCTCPP
GYTGRNCSAPVSRCEHAPCHNGATCHERGHRYVCECARGYGGPNCQFLLPEL
PPGPAVVDLTEKLEGQGGPFPWVAVCAGVILVLMLLLGCAAVVVCVRLRLQK
HRPPADPCRGETETMNNLANCQREKDISVSIIGATQIKNTNKKADFHGDHSAD
KNGFKARYPAVDYNLVQDLKGDDTAVRDAHSKRDTKCQPQGSSGEEKGTPTT
LRGGEASERKRPDSGCSTSKDTKYQSVYVISEEKDECVIATEV

SEQ ID NO: 5

Human DLL4 full-length protein (Uniprot, Q9NR61):

MAAASRSASGWALLLLVALWQQRAAGSGVFQLQLQEFINERGVLASGRPCEP
GCRTFFRVCLKHFQAVVSPGPCTFGTVSTPVLGTNSFAVRDDSSGGGRNPLQL
PFNFTWPGTFSLIIEAWHAPGDDLRPEALPPDALISKIAIQGSLAVGQNWLLDE
QTSTLTRLRYSYRVICSDNYYGDNCSRLCKKRNDHFGHYVCQPDGNLSCLPG
WTGEYCQQPICLSGCHEQNGYCSKPAECLCRPGWQGRLCNECIPHNGCRHGT
CSTPWQCTCDEGWGGLFCDQDLNYCTHHSPCKNGATCSNSGQRSYTCTCRP
GYTGVDCELELSECDSNPCRNGGSCKDQEDGYHCLCPPGYYGLHCEHSTLSC
ADSPCFNGGSCRERNQGANYACECPPNFTGSNCEKKVDRCTSNPCANGGQCL
NRGPSRMCRCRPGFTGTYCELHVSDCARNPCAHGGTCHDLENGLMCTCPAG
FSGRRCEVRTSIDACASSPCFNRATCYTDLSTDTFVCNCPYGFVGSRCEFPVGL
PPSFPWVAVSLGVGLAVLLVLLGMVAVAVRQLRLRRPDDGSREAMNNLSDFQ
KDNLIPAAQLKNTNQKKELEVDCGLDKSNCGKQQNHTLDYNLAPGPLGRGT
MPGKFPHSDKSLGEKAPLRLHSEKPECRISAICSPRDSMYQSVCLISEERNECV
IATEV

SEQ ID NO: 6.

### 1.1. Construction of cell strains highly expressing DLL3, DLL1, and DLL4

ApCDH lentiviral expression vector plasmid comprising SEQ ID NO: 1-6 (synthesized by GENEWIZ), along with a pVSVG or pCMV lentiviral packaging vector, was transfected into 293T cells (Cell Bank of the Chinese Academy of Sciences, GNHu17) using Lipofectamine 3000 (Invitrogen, L3000015). The virus-containing medium supernatants were collected, filtered, and subjected to ultracentrifugation. After the supernatants were discarded, resuspension was performed in 0.2 mL of sterile PBS. The concentrated viruses were then used to infect Chinese hamster ovary cells CHO-s (Invitrogen, R80007), DMS53 (ATCC, CRL-2062), and H82 (ATCC, HTB-175). After two to three weeks of screening with puromycin, FACS single-cell sorting was performed. The selected monoclonal cell strains were expanded and cryopreserved.

### 1.2. Preparation of antigens

Human DLL3 (Uniprot, Q9NYJ7), cynomolgus monkey DLL3 (Uniprot, A0A2K5WSR4), and mouse DLL3 (Uniprot, 088516) sequences were used as templates to design DLL3 ECD fusion proteins containing different tags, and the fusion proteins were cloned onto pTT5 vectors. After expression by 293E cells, antigens were obtained. The amino acid sequences of the related proteins were as follows:

1) His-hDLL3(ECD):

METDTLLLWVLLLWVPGSTGHHHHHHHHHHGGGGGSGGGGSAGVFELQIHSFGPGPGPGAPRSPCSARLPCRLFFRVCLKPGLSEEAAESPCALGAALSARGPVYTEQPGAPAPDLPLPDGLLQVPFRDAWPGTFSFIIETWREELGDQIGGPAWSLLARVAGRRRLAAGGPWARDIQRAGAWELRFSYRARCEPPAVGTACTRLCRPRSAPSRCGPGLRPCAPLEDECEAPLVCRAGCSPEHGFCEQPGECRCLEGWTGPLCTVPVSTSSCLSPRGPSSATTGCLVPGPGPCDGNPCANGGSCSETPRSFECTCPRGFYGLRCEVSGVTCADGPCFNGGLCVGGADPDSAYICHCPPGFQGSNCEKRVDRCSLQPCRNGGLCLDLGHALRCRCRAGFAGPRCEHDLDDCAGRACANGGTCVEGGGAHRCSCALGFGGRDCRERADPCAARPCAHGGRCYAHFSGLVCACAPGYMGARCEFPVHPDGASALPAAPPGLRPGDPQRYL

SEQ ID NO: 7

Note: The signal peptide sequence is highlighted with a dotted line; the his tag and the linker are single underlined; and the extracellular region of DLL3 is double underlined.

2) Fc-hDLL3(ECD):

METDTLLLWVLLLWVPGSTGEPKSSDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGGGGGSGGGGSAGVFELQIHSFGPGPGPGAPRSPCSARLPCRLFFRVCLKPGLSEEAAESPCALGAALSARGPVYTEQPGAPAPDLPLPDGLLQVPFRDAWPGTFSFIIETWREELGDQIGGPAWSLLARVAGRRRLAAGGPWARDIQRAGAWELRFSYRARCEPPAVGTACTRLCRPRSAPSRCGPGLRPCAPLEDECEAPLVCRAGCSPEHGFCEQPGECRCLEGWTGPLCTVPVSTSSCLSPRGPSSATTGCLVPGPGPCDGNPCANGGSCSETPRSFECTCPRGFYGLRCEVSGVTCADGPCFNGGLCVGGADPDSAYICHCPPGFQGSNCEKRVDRCSLQPCRNGGLCLDLGHALRCRCRAGFAGPRCEHDLDDCA

GRACANGGTCVEGGGAHRCSCALGFGGRDCRERADPCAARPCAHGGRCYAHFSGLVCACAPGYMGARCEFPVHPDGASALPAAPPGLRPGDPQRYL

SEQ ID NO: 8

Note: The signal peptide sequence is highlighted with a dotted line; the Fc tag and the linker are single underlined; and the extracellular region of DLL3 is double underlined.

3) hDLL3(ECD)-strep twin:

METDTLLLWVLLLWVPGSTGAGVFELQIHSFGPGPGPGAPRSPCSARLPCRLF
FRVCLKPGLSEEAAESPCALGAALSARGPVYTEQPGAPAPDLPLPDGLLQVPF
RDAWPGTFSFIIETWREELGDQIGGPAWSLLARVAGRRRLAAGGPWARDIQRA
GAWELRFSYRARCEPPAVGTACTRLCRPRSAPSRCGPGLRPCAPLEDECEAPL
VCRAGCSPEHGFCEQPGECRCLEGWTGPLCTVPVSTSSCLSPRGPSSATTGCL
VPGPGPCDGNPCANGGSCSETPRSFECTCPRGFYGLRCEVSGVTCADGPCFNG
GLCVGGADPDSAYICHCPPGFQGSNCEKRVDRCSLQPCRNGGLCLDLGHALR
CRCRAGFAGPRCEHDLDDCAGRACANGGTCVEGGGAHRCSCALGFGGRDC
RERADPCAARPCAHGGRCYAHFSGLVCACAPGYMGARCEFPVHPDGASALP
AAPPGLRPGDPQRYLWSHPQFEKGGGSGGGSGGSAWSHPQFEK

SEQ ID NO: 9

Note: The signal peptide sequence is highlighted with a dotted line; the extracellular region of DLL3 is double-underlined; and the strep twin tag is single-underlined.

4) cynoDLL3(ECD)-strep twin:

METDTLLLWVLLLWVPGSTGAGVFELQIHSFGPGPGPGAPRSPCSARGPCRLF
FRVCLKPGLSEEAAESPCALGAALSARGPVYTEQPEAPAPDLPLPNGLLQVPF
RDAWPGTFSLIIETWREELGDQIGGPAWSLLARVTRRRRLAAGGPWARDIQRA
GAWELRFSYRARCELPAVGTACTRLCRPRSAPSRCGPGLRPCAPLEDECEAPP
VCRAGCSLEHGFCEQPGECRCLEGWTGPLCMVPVSTSSCLGLRGPSSTTGCL
VPGPGPCDGNPCANGGSCSETPGSFECTCPRGFYGLRCEVSGVTCADGPCFN
GGLCVGGADPDSAYICHCPPGFQGSNCEKRVDRCSLQPCRNGGLCLDLGHAL
RCRCRAGFAGPRCEHDLDDCAGRACANGGTCVEGGGAHRCSCALGFGGRN
CRERADPCAARPCAHGGRCYAHFSGLVCACAPGYMGARCEFPVHPDGVSAL
PAAPPGLRPGDPQRYLWSHPQFEKGGGSGGGSGGSAWSHPQFEK

SEQ ID NO: 10

Note: The signal peptide sequence is highlighted with a dotted line; the extracellular region of DLL3 is double-underlined; and the strep twin tag is single-underlined.

5) mouDLL3(ECD)-strep twin:

METDTLLLWVLLLWVPGSTGAGVFELQIHSFGPGPGLGTPRSPCNARGPCRLF
FRVCLKPGVSQEATESLCALGAALSTSVPVYTEHPGESAAALPLPDGLVRVPF
RDAWPGTFSLVIETWREQLGEHAGGPAWNLLARVVGRRRLAAGGPWARDVQ
RTGTWELHFSYRARCEPPAVGAACARLCRSRSAPSRCGPGLRPCTPFPDECEA
PSVCRPGCSPEHGYCEEPDECRCLEGWTGPLCTVPVSTSSCLNSRVPGPASTG
CLLPGPGPCDGNPCANGGSCSETSGSFECACPRGFYGLRCEVSGVTCADGPCF

NGGLCVGGEDPDSAYVCHCPPGFQGSNCEKRVDRCSLQPCQNGGLCLDLGH
ALRCRCRAGFAGPRCEHDLDDCAGRACANGGTCVEGGGSRRCSCALGFGGR
DCRERADPCASRPCAHGGRCYAHFSGLVCACAPGYMGVRCEFAVRPDGADA
VPAAPRGLRQADPQRFLWSHPQFEKGGGSGGGSGGSAWSHPQFEK

SEQ ID NO: 11

Note: The signal peptide sequence is highlighted with a dotted line; the extracellular region of DLL3 is double-underlined; and the strep twin tag is single-underlined.

**Example 2: Preparation of Mouse Anti-Human DLL3 Monoclonal Antibodies**

**1. Immunization**

Anti-human DLL3 monoclonal antibodies were produced by immunization of mice. Laboratory SJL mice, female, 6-8 weeks of age (Shanghai SLAC Laboratory Animal Co., Ltd., animal production license number: SCXK(Shanghai)2017-0005). Housing environment: SPF. The purchased mice were housed in a laboratory environment for 1 week, with a 12/12 hour light/dark cycle, at a temperature of 20-25 °C with humidity at 40-60%. The acclimatized mice were immunized according to the following scheme. Immunization scheme:
The immunizing antigen for the first group of mice was His-hDLL3(ECD) (SEQ ID NO: 7). Cross-immunizations were performed using the adjuvants TiterMax® Gold Adjuvant (Sigma Cat No. T2684) and Thermo Imject® Alum (Thermo Cat No. 77161). The ratio of the antigen to the adjuvant TiterMax® Gold Adjuvant was 1: 1, and the ratio of the antigen to the adjuvant Thermo Imject® Alum was 3: 1. The dose for the first immunization was 50 μg/mouse/immunization, and the dose for boost immunization was 25 μg/mouse/immunization. The antigen was emulsified before inoculation. Immunizations were performed at days 0, 7, 14, and 21. Blood was collected at days 7 and 21, and mouse serum was assayed for antibody titer by ELISA. After the 4th immunization, mice in which the antibody titer in the serum was high and tended to plateau were selected for splenocyte fusion. Boost immunizations were performed 3 days before splenocyte fusion; each mouse was intraperitoneally (i.p.) injected with 25 μg of antigen solution prepared in normal saline.

The immunizing antigens for the second group of mice were DLL3 CHO-s and Fc-hDLL3(ECD) (SEQ ID NO: 8), and immunizations were alternately performed with cell and protein antigens. Before the first immunization with DLL3 CHO-s cells, each mouse was intraperitoneally injected with 0.1 mL of TiterMax® Gold Adjuvant (Sigma Cat No. T2684) in advance, and after half an hour, each mouse was intraperitoneally injected with 0.1 mL of cell liquid diluted with normal saline to the concentration of $10^8$/mL. The cells were well pipetted, and then inoculation was performed at days 0, 14, 28, and 42. For the Fc-hDLL3(ECD) antigen, cross-immunizations were performed using the adjuvants TiterMax® Gold Adjuvant (Sigma Cat No. T2684) and Thermo Imject® Alum (Thermo Cat No. 77161). The ratio of the antigen to the adjuvant TiterMax® Gold Adjuvant was 1:1, and the ratio of the antigen to the adjuvant Thermo Imject® Alum was 3:1. The dose for the first immunization was 50 μg/mouse/immunization, and the dose for boost immunization was 25 μg/mouse/immunization. For hDLL3- Fc, immunizations were performed at days 7, 21, 35, and 49. Blood was collected at days 14, 35, and 49, and mouse serum was assayed for antibody titer by ELISA. After the 8th immunization, mice in which the antibody titer in the serum was high and tended to plateau were selected for splenocyte fusion. Boost immunizations were performed 3 days before splenocyte fusion; each mouse was intraperitoneally (i.p.) injected with 50 μg of hDLL3-Fc protein antigen solution prepared in normal saline.

**2. Splenocyte fusion**

Spleen lymphocytes and the myeloma cells, Sp2/0 cells (ATCC® CRL-8287™), were fused by following an optimized electrofusion procedure to obtain hybridoma cells.

The resulting hybridoma cells were resuspended in complete medium (IMDM medium containing 20% FBS, 1× HAT, and 1 × OPI) at a density of 3-4 × $10^5$/mL and seeded in a 96-well plate at 150 μL/well. After 3-4 days of incubation at 37 °C with 5% $CO_2$, the supernatant was removed and HT complete medium (IMDM medium containing 20% FBS, 1 × HT, and 1 × OPI) was added at 200 μL/well. After 3 days of culture at 37 °C with 5% $CO_2$, screening and assays were performed.

**3. Hybridoma cell screening and antibody sequence determination**

Based on the growth density of hybridoma cells, the hybridoma culture supernatant was assayed using an

ELISA method with binding to DLL3 protein and a FACS method with binding to DLL3 CHO-s cells. Clones that bind to human DLL3 protein, monkey DLL3 protein, and DLL3 CHO-s cells but not to wild-type CHO-s cells were selected and cryopreserved, expanded, conserved, and subcloned one to two times in time until single cell clones were obtained. Through the screening experiment above, hybridoma clones mAb100 and mAb6 were obtained.

The hybridoma clones were expanded, and RNA was extracted. Reverse transcription amplification (RT-PCR) was performed using a degenerate primer of mouse-Ig, and the variable region sequences of the antibodies were finally obtained.

The heavy chain variable region of mAb6:

EVQLQQSGPVLVKPGASVKMSCKASGYTFTDYYMNWVKQSHGKSLEWIGII
NPYSGVTIYNHKFKGKATLTVDKSSNTAYMELNSLTSEDSAVYYCARQDSLLD
YAMDYWGQGTSVTVSS

SEQ ID NO: 12

The light chain variable region of mAb6:

DILMTQSPSSMSVSLGDTVSITCHASQGISGNMGWLQQKPGKSFKGLIYHGA
NLEDGVPSRFSGSGSGADYSLTISSLESEDFADYYCVQYAQFPYTFGGGTKLEI
K

SEQ ID NO: 13

The heavy chain variable region of mAb100:

EVQLVESGGGLVKPGGSLKLSCAASGFTFSDSGMHWIRQAPEKGLEWVAYISS
GSSTIHYADTVKGRFTISRDNAKNTLFLQMTSLRSEDTAMYYCASPLYYYGRS
YNAVAYWGQGTSVTVSS

SEQ ID NO: 14

The light chain variable region of mAb100:

DIQMNQSPSSLSASLGDTITITCHASQNINVWLSWYQQKPGNIPKLLIYKASDL
HTGVPSRFSGSGSGTGFTLTISSLQPEDIATYYCQQGQSYPLTFGGGTKLEIK

SEQ ID NO: 15.

Table 4. The CDR sequences of the murine H6 and H100 antibodies

| Antibody | Name | Sequence | No. |
|---|---|---|---|
| mAb6 | HCDR1 | DYYMN | SEQ ID NO: 16 |
| | HCDR2 | IINPYSGVTIYNHKFKG | SEQ ID NO: 17 |
| | HCDR3 | QD SLLDYAMDY | SEQ ID NO: 18 |
| | LCDR1 | HASQGISGNMG | SEQ ID NO: 19 |
| | LCDR2 | HGANLED | SEQ ID NO: 20 |
| | LCDR3 | VQYAQFPYT | SEQ ID NO: 21 |

(continued)

| Antibody | Name | Sequence | No. |
|---|---|---|---|
| mAb100 | HCDR1 | DSGMH | SEQ ID NO: 22 |
| | HCDR2 | YISSGSSTIHYADTVKG | SEQ ID NO: 23 |
| | HCDR3 | PLYYYGRSYNAVAY | SEQ ID NO: 24 |
| | LCDR1 | HASQNINVWLS | SEQ ID NO: 25 |
| | LCDR2 | KASDLHT | SEQ ID NO: 26 |
| | LCDR3 | QQGQSYPLT | SEQ ID NO: 27 |
| Note: The amino acid residues of the CDRs of the VH/VL were determined and annotated using the Kabat numbering scheme. | | | |

The heavy chain variable regions and the light chain variable regions of the murine antibodies were cloned into pTT 5 vector plasmids containing a human IgG1 heavy chain constant region set forth in SEQ ID NO: 28 and a κ light chain constant region set forth in SEQ ID NO: 29, and HEK293 cells were transfected with the resulting plasmids to obtain anti-DLL3 chimeric antibodies M6CHI and M100CHI.

Human IgG1 heavy chain constant region:

ASTKGPSVFPLAPSSKSTSGGTAALGCLVKDYFPEPVTVSWNSGALTSGVHTF
PAVLQSSGLYSLSSVVTVPSSSLGTQTYICNVNHKPSNTKVDKKVEPKSCDKT
HTCPPCPAPELLGGPSVFLFPPKPKDTLMISRTPEVTCVVVDVSHEDPEVKFN
WYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVE
WESNGQPENNYKTTPPVLDSDGSFFLYSKLTVDKSRWQQGNVFSCSVMHEAL
HNHYTQKSLSLSPGK

SEQ ID NO: 28

Human κ light chain constant region:

RTVAAPSVFIFPPSDEQLKSGTASVVCLLNNFYPREAKVQWKVDNALQSGNS

QESVTEQDSKDSTYSLSSTLTLSKADYEKHKVYACEVTHQGLSSPVTKSFNRG
EC

SEQ ID NO: 29.

**Example 3: Humanization of Murine Anti-DLL3 Monoclonal Antibodies**

Through comparison with a Kabat human antibody heavy and light chain variable region germline gene database, heavy and light chain variable region germline genes with high homology were selected as templates, and the CDRs of the murine antibodies were grafted onto the corresponding human templates to form variable region sequences structured as FR1-CDR1-FR2-CDR2-FR3-CDR3-FR4. Amino acids in the variable regions were subjected to back mutation followed by recombination with constant regions (for example, the human IgG1 heavy chain constant region set forth in SEQ ID NO: 28 and the human κ light chain constant region set forth in SEQ ID NO: 29) to obtain full-length antibodies.

For the mAb6 antibody, the human germline light chain template was IGKV1-16*01, and the human germline heavy chain templates were IGHV1-3*01, IGHV7-4-1*02, and IGHV3-73*01. For the H100 antibody, the human germline light chain template was IGKV1-27*01, and the human germline heavy chain template was IGHV3-11*01.

In addition, the amino acid residue at position 4 of the HCDR3 of the heavy chain variable region of mAb100 (PLYYYGRSYNAVAY (SEQ ID NO: 24)) was mutated from Y to H or the amino acid residue at position 11 was mutated from A to G to obtain a new HCDR3: PLY**H**YGRSYNAVAY (SEQ ID NO: 30) or PLYYYGRSYN**G**VAY (SEQ ID NO: 31).

Table 5. Humanization templates and corresponding point mutations for the mAb6 and mAb100 antibodies

| | **VL** | | **VH** | |
|---|---|---|---|---|
| hAb6 | VI,1 | Graft (IGKV1-16*01) + F36L, S46G | VH1 | Graft(IGHV1-3*01) + Q1E, R71V, T73K |
| | VL2 | Graft(IGKV1-16*01) + F36L, S46G, T69A, F71Y | VH2 | Graft(IGHV1-3*01) + Q1E, I69L, R71V, T73K, S76N |
| | VL3 | Graft(IGKV1-16*01) + F36L, A43S, P44F, S46G, T69A, F71Y | VH3 | Graft(IGHV1-3*01) + Q1E, M48I, V67A, I69L, R71V, T73K, S76N |
| | VL4 | Graft(IGKV1-16*01) + F36L, S46G, T69A, F71Y, T85D | VH4 | Graft(IGHV1-3*01) + Q1E, I69L, R71V, T73K, S76N, Q43K |
| | VL5 | Graft (IGKV1-16*01) | VH5 | Graft(IGHV7-4-1*02) + Q1E, L71V, T73K, F69L |
| | | | VH6 | Graft(IGHV7-4-1*02) + Q1E, F69L, L71V, T73K, V75S, S76N |
| | | | VH7 | Graft(IGHV3-73 *01) + F27Y, S30T, I69L, R71V, D73K,T93A |
| | | | VH8 | Graft(IGHV1-3*01) |
| | | | VH9 | Graft(IGHV7-4-1*02) |
| | | | VH10 | Graft(IGHV3-73*01) |
| | | | VH11 | Graft(IGHV7-4-1*02) + Q1E, F69L, L71V, T73K,V75S, S76N, R38K |
| | | | VH12 | Graft(IGHV7-4-1*02) + Q1E, F69L, L71V, T73K, V75S, S76N, V68A |
| | | | VH13 | Graft(IGHV7-4-1*02) + Q1E, F69L, L71V, T73K, V75S, S76N, R38K,V68A |
| hAb100 | VI,1 | Graft(IGKV1-27*01) | VH1 | Graft(IGHV3-11*01) + Q1E,R94S |
| | VL2 | Graft(IGKV1-27*01) + V43I | VH2 | Graft(IGHV3-11*01) + Q1E, S49A, R94S |
| | | | VH3 | Graft(IGHV3-11*01) + Q1E, S49A, R94S,Y98H |
| | | | VH4 | Graft(IGHV3-11*01) + Q1E, S49A, R94S, A100eG |
| | | | VH5 | Graft(IGHV3-11*01) |
| Note: Taking A100G in the table as an example, it represents the mutation of A at position 100E under kabat numbering to G. | | | | |

The sequences of the humanized antibody variable regions obtained were as follows:

hAb6 VH1 (Q1E, R71V, T73K)

**E**VQLVQSGAEVKKPGASVKVSCKASGYTFTDYYMNWVRQAPGQRLEWMGIINPYSGVTIYNHKFKGRVTIT**V**D**K**SASTAYMELSSLRSEDTAVYYCARQDSLLDYAMDYWGQGTTVTVSS

SEQ ID NO: 32

hAb6 VH2 (Q1E, I69L, R71V, T73K, S76N)

**E**VQLVQSGAEVKKPGASVKVSCKASGYTFTDYYMNWVRQAPGQRLEWMGIINPYSGVTIYNHKFKGRVT**L**T**V**D**K**SA**N**TAYMELSSLRSEDTAVYYCARQDSLLDYAMDYWGQGTTVTVSS

SEQ ID NO: 33

hAb6 VH3 (Q1E, M48I, V67A, I69L, R71V, T73K, S76N)

**E**VQLVQSGAEVKKPGASVKVSCKASGYTFTDYYMNWVRQAPGQRLEW**I**GIINPYSGVTIYNHKFKGR**A**T**L**T**V**D**K**SA**N**TAYMELSSLRSEDTAVYYCARQDSLLDYAMDYWGQGTTVTVSS

SEQ ID NO: 34

hAb6 VH4 (Q1E, Q43K, I69L, R71V, T73K, S76N)

**E**VQLVQSGAEVKKPGASVKVSCKASGYTFTDYYMNWVRQAPG**K**RLEWMGIINPYSGVTIYNHKFKGRVT**L**T**V**D**K**SA**N**TAYMELSSLRSEDTAVYYCARQDSLLDYAMDYWGQGTTVTVSS

SEQ ID NO: 35

hAb6 VH5 (Q1E, L71V, T73K, F69L)

**E**VQLVQSGSELKKPGASVKVSCKASGYTFTDYYMNWVRQAPGQGLEWMGIINPYSGVTIYNHKFKGRFV**L**S**V**D**K**SVSTAYLQISSLKAEDTAVYYCARQDSLLDYAMDYWGQGTTVTVSS

SEQ ID NO: 36

hAb6 VH6 (Q1E, F69L, L71V, T73K, V75S, S76N)

**E**VQLVQSGSELKKPGASVKVSCKASGYTFTDYYMNWVRQAPGQGLEWMGIINPYSGVTIYNHKFKGRFV**L**S**V**D**K**S**SN**TAYLQISSLKAEDTAVYYCARQDSLLDYAMDYWGQGTTVTVSS

SEQ ID NO: 37

hAb6 VH7 (F27Y, S30T, I69L, R71V, D73K,T93A)

EVQLVESGGGLVQPGGSLKLSCAASG**Y**TF**T**DYYMNWVRQASGKGLEWVGIINPYSGVTIYNHKFKGRFT**L**S**V**D**K**SKNTAYLQMNSLKTEDTAVYYC**A**RQDSLLDYAMDYWGQGTTVTVSS

SEQ ID NO: 38

hAb6VH8 (Graft IGHV1-3*01)

QVQLVQSGAEVKKPGASVKVSCKASGYTFTDYYMNWVRQAPGQRLEWMGIINPYSGVTIYNHKFKGRVTITRDTSASTAYMELSSLRSEDTAVYYCARQDSLLDYAMDYWGQGTTVTVSS

SEQ ID NO: 39

hAb6VH9 (Graft IGHV7-4-1*02)

QVQLVQSGSELKKPGASVKVSCKASGYTFTDYYMNWVRQAPGQGLEWMGIINPYSGVTIYNHKFKGRFVFSLDTSVSTAYLQISSLKAEDTAVYYCARQDSLLDYAMDYWGQGTTVTVSS

SEQ ID NO: 40

hAb6VH10 (Graft IGHV3-73*01)

EVQLVESGGGLVQPGGSLKLSCAASGFTFSDYYMNWVRQASGKGLEWVGIINPYSGVTIYNHKFKGRFTISRDDSKNTAYLQMNSLKTEDTAVYYCTRQDSLLDYAMDYWGQGTTVTVSS

SEQ ID NO: 41

hAb6VH11 (Q1E, F69L, L71V, T73K,V75S, S76N, R38K)

**E**VQLVQSGSELKKPGASVKVSCKASGYTFTDYYMNWV**K**QAPGQGLEWMGIINPYSGVTIYNHKFKGRFV**L**S**V**D**K**S**SN**TAYLQISSLKAEDTAVYYCARQDSLLDYAMDYWGQGTTVTVSS

SEQ ID NO: 42

hAb6VH12 (Q1E, V68A, F69L, L71V, T73K, V75S, S76N)

**E**VQLVQSGSELKKPGASVKVSCKASGYTFTDYYMNWVRQAPGQGLEWMGIINPYSGVTIYNHKFKGRF**AL**S**V**D**K**S**SN**TAYLQISSLKAEDTAVYYCARQDSLLDYAMDYWGQGTTVTVSS

SEQ ID NO: 43

hAb6VH13 (Q1E, R38K, V68A, F69L, L71V, T73K, V75S, S76N)

EVQLVQSGSELKKPGASVKVSCKASGYTFTDYYMNWVKQAPGQGLEWMGIINPYSGVTIYNHKFKGRFALSVDKSSNTAYLQISSLKAEDTAVYYCARQDSLLDYAMDYWGQGTTVTVSS

SEQ ID NO: 44

hAb6 VL1 (F36L, S46G)

DIQMTQSPSSLSASVGDRVTITCHASQGISGNMGWLQQKPGKAPKGLIYHGANLEDGVPSRFSGSGSGTDFTLTISSLQPEDFATYYCVQYAQFPYTFGGGTKVEIK

SEQ ID NO: 45

hAb6 VL2 (F36L, S46G, T69A, F71Y)

DIQMTQSPSSLSASVGDRVTITCHASQGISGNMGWLQQKPGKAPKGLIYHGANLEDGVPSRFSGSGSGADYTLTISSLQPEDFATYYCVQYAQFPYTFGGGTKVEIK

SEQ ID NO: 46

hAb6 VL3 (F36L, A43S, P44F, S46G, T69A, F71Y)

DIQMTQSPSSLSASVGDRVTITCHASQGISGNMGWLQQKPGKSFKGLIYHGANLEDGVPSRFSGSGSGADYTLTISSLQPEDFATYYCVQYAQFPYTFGGGTKVEIK

SEQ ID NO: 47

hAb6 VL4 (F36L, S46G, T69A, F71Y, T85D)

DIQMTQSPSSLSASVGDRVTITCHASQGISGNMGWLQQKPGKAPKGLIYHGANLEDGVPSRFSGSGSGADYTLTISSLQPEDFADYYCVQYAQFPYTFGGGTKVEIK

SEQ ID NO: 48

hAb6VL5 (Graft IGKV1-16*01)

DIQMTQSPSSLSASVGDRVTITCHASQGISGNMGWFQQKPGKAPKSLIYHGANLEDGVPSRFSGSGSGTDFTLTISSLQPEDFATYYCVQYAQFPYTFGGGTKVEIK

SEQ ID NO: 49

hAb100 VH1 (Q1E, R94S)

EVQLVESGGGLVKPGGSLRLSCAASGFTFSDSGMHWIRQAPGKGLEWVSYISSGSSTIHYADTVKGRFTISRDNAKNSLYLQMNSLRAEDTAVYYCASPLYYYGRSYNAVAYWGQGTTVTVSS

SEQ ID NO: 50

hAb100 VH2 (Q1E, S49A, R94S)

EVQLVESGGGLVKPGGSLRLSCAASGFTFSDSGMHWIRQAPGKGLEWVAYIS SGSSTIHYADTVKGRFTISRDNAKNSLYLQMNSLRAEDTAVYYCASPLYYYGR SYNAVAYWGQGTTVTVSS

SEQ ID NO: 51

hAb100 VH3 (Q1E, S49A, R94S, Y98H)

EVQLVESGGGLVKPGGSLRLSCAASGFTFSDSGMHWIRQAPGKGLEWVAYIS SGSSTIHYADTVKGRFTISRDNAKNSLYLQMNSLRAEDTAVYYCASPLYHYGR SYNAVAYWGQGTTVTVSS

SEQ ID NO: 52

hAb100 VH4 (Q1E, S49A, R94S, A100eG)

EVQLVESGGGLVKPGGSLRLSCAASGFTFSDSGMHWIRQAPGKGLEWVAYIS SGSSTIHYADTVKGRFTISRDNAKNSLYLQMNSLRAEDTAVYYCASPLYYYGR SYNGVAYWGQGTTVTVSS

SEQ ID NO: 53

hAb100VH5 (Graft IGHV3-11*01)

QVQLVESGGGLVKPGGSLRLSCAASGFTFSDSGMHWIRQAPGKGLEWVSYIS SGSSTIHYADTVKGRFTISRDNAKNSLYLQMNSLRAEDTAVYYCARPLYYYGR SYNAVAYWGQGTTVTVSS

SEQ ID NO: 54

hAb100 VL1 (Graft IGKV1-27*01)

DIQMTQSPSSLSASVGDRVTITCHASQNINVWLSWYQQKPGKVPKLLIYKASD LHTGVPSRFSGSGSGTDFTLTISSLQPEDVATYYCQQGQSYPLTFGGGTKVEIK

SEQ ID NO: 55

hAb100 VL2 (V43I)

DIQMTQSPSSLSASVGDRVTITCHASQNINVWLSWYQQKPGKIPKLLIYKASD LHTGVPSRFSGSGSGTDFTLTISSLQPEDVATYYCQQGQSYPLTFGGGTKVEIK

SEQ ID NO: 56.

Note: CDRs are single underlined, and mutation sites are double underlined.

Table 6. General formulas for the CDRs of the hAb 100 antibody

| Name | Sequence | Sequence No. |
|---|---|---|
| HCDR1 | DSGMH | SEQ ID NO: 22 |
| HCDR2 | YISSGSSTIHYADTVKG | SEQ ID NO: 23 |
| HCDR3 | PLYX$_1$YGRSYNX$_2$VAY | SEQ ID NO: 57 |
| LCDR1 | HASQNINVWLS | SEQ ID NO: 25 |
| LCDR2 | KASDLHT | SEQ ID NO: 26 |
| LCDR3 | QQGQSYPLT | SEQ ID NO: 27 |

Xi is Y or H; $X_2$ is A or G.

Exemplary heavy and light chain variable region combinations for humanized antibodies were as follows:

Table 7. Humanized antibodies of mAb6

| Variable region | VL1 | VL2 | VL3 | VL4 |
|---|---|---|---|---|
| VH1 | hAb6L1H1 | hAb6L2H1 | hAb6L3H1 | hAb6L4H1 |
| VH2 | hAb6L1H2 | hAb6L2H2 | hAb6L3H2 | hAb6L4H2 |
| VH3 | hAb6L1H3 | hAb6L2H3 | hAb6L3H3 | hAb6L4H3 |
| VH4 | hAb6L1H4 | hAb6L2H4 | hAb6L3H4 | hAb6L4H4 |
| VH5 | hAb6L1H5 | hAb6L2H5 | hAb6L3H5 | hAb6L4H5 |
| VH6 | hAb6L1H6 | hAb6L2H6 | hAb6L3H6 | hAb6L4H6 |
| VH7 | hAb6L1H7 | hAb6L2H7 | hAb6L3H7 | hAb6L4H7 |
| VH11 | hAb6L1H11 | hAb6L2H11 | hAb6L3H11 | hAb6L4H11 |
| VH12 | hAb6L1H12 | hAb6L2H12 | hAb6L3H12 | hAb6L4H12 |
| VH13 | hAb6L1H13 | hAb6L2H13 | hAb6L3H13 | hAb6L4VH13 |
| Note: hAb6L1H1 indicates that the antibody comprises the heavy chain variable region hAb6VH1 and the light chain variable region hAb6VL1 and that the sequence of the heavy chain constant region is SEQ ID NO: 28 and the sequence of the light chain constant region is SEQ ID NO: 29. This applies to the others. | | | | |

Table 8. Humanized antibodies of mAb100

| Variable region | VI,1 | VL2 |
|---|---|---|
| VH1 | hAb 100L1H1 | hAb100L2H1 |
| VH2 | hAb100L1H2 | hAb100L2H2 |
| VH3 | hAb100L1H3 | hAb100L2H3 |
| VH4 | hAb100L1H4 | hAb100L2H4 |
| Note: hAb100L1H1 indicates that the antibody comprises the heavy chain variable region hAb100VH1 and the light chain variable region hAb100VL1 and that the sequence of the heavy chain constant region is SEQ ID NO: 28 and the sequence of the light chain constant region is SEQ ID NO: 29. This applies to the others. | | |

The above antibodies were cloned, expressed, and purified, and through a protein binding assay (Test Example 1), a cell binding assay (Test Example 2), and Biacore (Test Example 4), humanized antibodies with good activity were finally selected. The heavy and light chain amino acid sequences of exemplary humanized antibodies were as follows:

The heavy chain of Hu6 (also known as hAb6L4H12):

<u>EVQLVQSGSELKKPGASVKVSCKASGYTFTDYYMNWVRQAPGQGLEWMGII NPYSGVTIYNHKFKGRFALSVDKSSNTAYLQISSLKAEDTAVYYCARQDSLLD YAMDYWGQGTTVTVSS</u>*ASTKGPSVFPLAPSSKSTSGGTAALGCLVKDYFPEPVTV SWNSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVE WESNGQPENNYKTTPPVLDSDGSFFLYSKLTVDKSRWQQGNVFSCSVMHEALHN HYTQKSLSLSPGK*

Note: In the sequence, the variable region is underlined, and the constant region is italicized.
SEQ ID NO: 58 The light chain of Hu6 (also known as hAb6L4H12):

<u>DIQMTQSPSSLSASVGDRVTITCHASQGISGNMGWLQQKPGKAPKGLIYHGA NLEDGVPSRFSGSGSGADYTLTISSLQPEDFADYYCVQYAQFPYTFGGGTKVEI K</u>*RTVAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC*

Note: In the sequence, the variable region is underlined, and the constant region is italicized.
SEQ ID NO: 59 The heavy chain of Hu100 (also known as hAb100L1H1):

<u>EVQLVESGGGLVKPGGSLRLSCAASGFTFSDSGMHWIRQAPGKGLEWVSYISS GSSTIHYADTVKGRFTISRDNAKNSLYLQMNSLRAEDTAVYYCASPLYYYGRS YNAVAYWGQGTTVTVSS</u>*ASTKGPSVFPLAPSSKSTSGGTAALGCLVKDYFPEPVT VSWNSGALTSGVHTFPAVLQSSGLYSLSSVVTVPSSSLGTQTYICNVNHKPSNTKVDK KVEPKSCDKTHTCPPCPAPELLGGPSVFLFPPKPKDTLMISRTPEVTCVVVDVSHE DPEVKFNWYVDGVEVHNAKTKPREEQYNSTYRVVSVLTVLHQDWLNGKEYKCKV SNKALPAPIEKTISKAKGQPREPQVYTLPPSRDELTKNQVSLTCLVKGFYPSDIAVE WESNGQPENNYKTTPPVLDSDGSFFLYSKLTVDKSRWQQGNVFSCSVMHEALHN HYTQKSLSLSPGK*

Note: In the sequence, the variable region is underlined, and the constant region is italicized.
SEQ ID NO: 60 The light chain of Hu100 (also known as hAb100L1H1):

<u>DIQMTQSPSSLSASVGDRVTITCHASQNINVWLSWYQQKPGKVPKLLIYKASD LHTGVPSRFSGSGSGTDFTLTISSLQPEDVATYYCQQGQSYPLTFGGGTKVEIK</u> *RTVAAPSVFIFPPSDEQLKSGTASVVCLLNNFYPREAKVQWKVDNALQSGNSQESV TEQDSKDSTYSLSSTLTLSKADYEKHKVYACEVTHQGLSSPVTKSFNRGEC*

Note: In the sequence, the variable region is underlined, and the constant region is italicized.
SEQ ID NO: 61. The positive control molecule used in the present disclosure was BI-764532 (constructed by reference to WO2019234220A1), and the negative control was C25. Their sequences were as follows:

The heavy chain of BI-764532:

QVQLVQSGAEVKKPGASVKVSCKASGYTFTSYYVHWVRQAPGQGLEWMVIINPGGGTTSYAQKFLGRVTMTRDTSTNTVYMELKSLRSEDTAVYYCARGEAVTGNYFYYGMDVWGQGTTVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*

Note: In the sequence, the variable region is underlined, and the constant region is italicized.
SEQ ID NO: 62 The light chain of BI-764532:

DIQMTQSPSAMSASVGDRVTITCRASQGISNYLVWFQQKPGKAPKRLIYAVSSLYSGVPSRFSGSGSGTEFTLTISSLQPEDFATYYCLQHDSYPYTFGQGTKLEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

Note: In the sequence, the variable region is underlined, and the constant region is italicized.
SEQ ID NO: 63 The heavy chain of C25:

QVQLVQSGAEVKKPGASVKVSCKASGYTFTNSWIGWFRQAPGQGLEWIGDIYPGGGYTNYNEIFKGKATMTADTSTNTAYMELSSLRSEDTAVYYCSRGIPGYAMDYWGQGTLVTVSS*ASTKGPSVFPLAPSSKSTSGGTAALGCLVKDYFPEPVTVSWNSGALTSGVHTFPAVLQSSGLYSLSSVVTVPSSSLGTQTYICNVNHKPSNTKVDKKVEPKSCDKTHTCPPCPAPELLGGPSVFLFPPKPKDTLMISRTPEVTCVVVDVSHEDPEVKFNWYVDGVEVHNAKTKPREEQYNSTYRVVSVLTVLHQDWLNGKEYKCKVSNKALPAPIEKTISKAKGQPREPQVYTLPPSRDELTKNQVSLTCLVKGFYPSDIAVEWESNGQPENNYKTTPPVLDSDGSFFLYSKLTVDKSRWQQGNVFSCSVMHEALHNHYTQKSLSLSPGK*

SEQ ID NO: 64

The light chain of C25:

DIQMTQSPSSLSASVGDRVTMSCKSSQSLLNSGDQKNYLTWYQQKPGKAPKLLIYWASTGESGVPSRFSGSGSGTDFTFTISSLQPEDIATYYCQNDYSYPWTFGQGTKVEIK*RTVAAPSVFIFPPSDEQLKSGTASVVCLLNNFYPREAKVQWKVDNALQSGNSQESVTEQDSKDSTYSLSSTLTLSKADYEKHKVYACEVTHQGLSSPVTKSFNRGEC*

SEQ ID NO: 65.

Note: In the sequence, the variable region is underlined, and the constant region is italicized.

## II. Preparation of Compounds

Experimental methods without specific conditions indicated in the examples of the present disclosure were generally conducted under conventional conditions or conditions recommended by the manufacturer of the starting materials or commercial products. Reagents without specific origins indicated are commercially available conventional reagents.

The structures of the compounds were determined by nuclear magnetic resonance (NMR) spectroscopy or mass spectrometry (MS). The NMR analyses were performed on a Bruker AVANCE-400 nuclear magnetic resonance instrument, with dimethyl sulfoxide-D6 (*DMSO-d6*), chloroform-D ($CDCl_3$), and methanol-D4 ($CD_3OD$) as solvents and tetramethylsilane (TMS) as an internal standard. The chemical shifts were given in unit of $10^{-6}$ (ppm).

The MS analyses were performed on a FINNIGAN LCQAd (ESI) mass spectrometer (Thermo, model: Finnigan LCQ advantage MAX).

The UPLC analyses were performed on a Waters Acquity UPLC SQD liquid chromatography-mass spectrometry system.

The HPLC analyses were performed on an Agilent 1200DAD high pressure liquid chromatograph (Sunfire C18 150 $\times$ 4.6 mm chromatography column) and a Waters 2695-2996 high pressure liquid chromatograph (Gimini C18 150 $\times$ 4.6 mm chromatography column).

The UV-HPLC analyses were performed on a Thermo nanodrop2000 ultraviolet spectrophotometer.

The thin-layer chromatography silica gel plates used were Yantai Huanghai HSGF254 or Qingdao GF254 silica gel plates. The silica gel plates used in the thin-layer chromatography (TLC) analyses had a layer thickness of 0.15 mm to 0.2 mm, and those used in the thin-layer chromatography separation and purification had a layer thickness of 0.4 mm to 0.5 mm.

In the column chromatography steps, 200 to 300 mesh Yantai Huanghai silica gel was generally used as a carrier.

The known starting materials of the present disclosure may be synthesized by using or following methods known in the art, or may be purchased from ABCR GmbH & Co.KG, Acros Organnics, Aldrich Chemical Company, Accela ChemBio Inc, Chembee Chemicals, etc.

In the examples, the reactions were performed in an argon atmosphere or nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator or an HC2-SS hydrogenator.

The hydrogenation reactions generally involved 3 vacuumization and hydrogen filling cycles.

The microwave reactions were performed using a CEM Discover-S 908860 microwave reactor.

In the examples, the solutions in the reactions were aqueous solutions unless otherwise specified.

In the examples, the reaction temperature was room temperature which ranges from 20 °C to 30 °C unless otherwise specified.

Preparation of the PBS buffer with a pH of 6.5 in the Examples: 8.5 g of $KH_2PO_4$, 8.56 g of $K_2HPO_4.3H_2O$, 5.85 g of NaCl, and 1.5 g of EDTA were added to a flask, and a solvent was added to make up to 2 L; they were all ultrasonically dissolved and well shaken to give the desired buffer.

The eluent systems used in the column chromatography purification of the compounds and the developing solvent systems used in the thin-layer chromatography analyses include: A: a system of dichloromethane and isopropanol, B: a system of dichloromethane and methanol, and C: a system of petroleum ether and ethyl acetate. The volume ratio of the solvents was adjusted depending on the polarity of the compound, or by adding a small amount of triethylamine and an acidic or basic reagent. Some of the compounds of the present disclosure were characterized by Q-TOF LC/MS. The Q-TOF LC/MS analyses were performed using an Agilent 6530 accurate-mass quadrupole time-of-flight mass spectrometer and an Agilent 1290-Infinity ultrahigh performance liquid chromatograph (Agilent Poroshell 300SB-C8 5 $\mu$m, 2.1 $\times$ 75 mm chromatography column).

The drug moieties of the antibody-drug conjugates of the present disclosure may be selected from cytotoxic drugs known in the art. Exemplary drugs are the ecteinascidin derivatives described in WO2021218896A1 (application No. PCT/CN2021089838), which is incorporated herein by reference in its entirety.

Synthesis schemes for exemplary antibody-drug conjugates in the present disclosure were as follows.

### Example 4-1. Synthesis of Compound 7 (Linker)

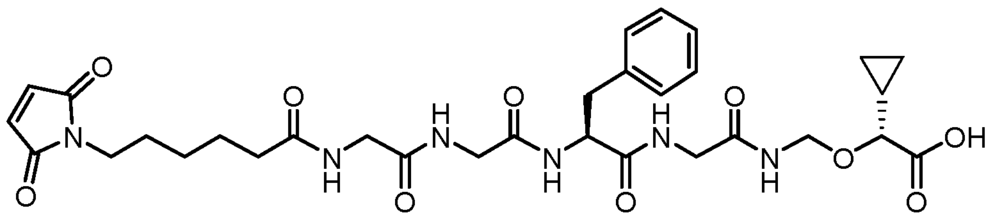

7

**Step 1**

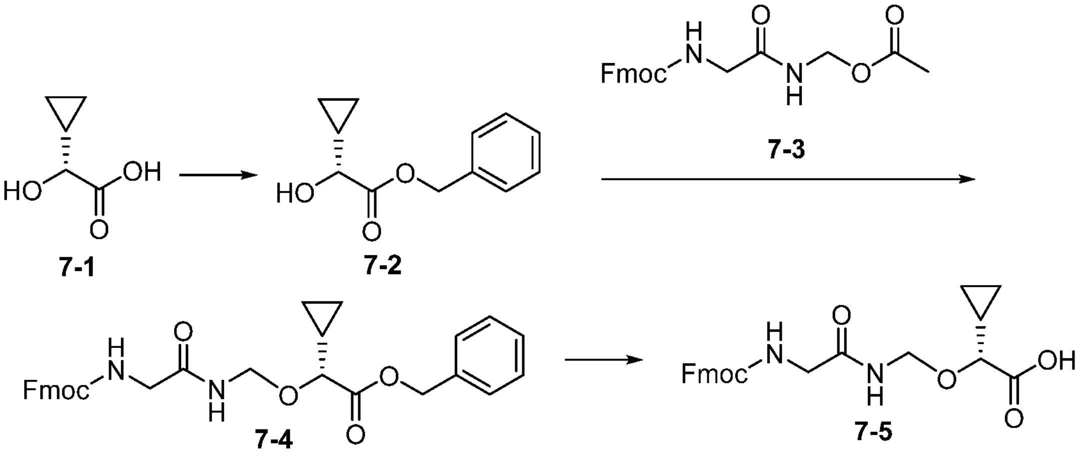

7-1 7-2 7-3 7-4 7-5

Compound **7-1** (1.3 g, prepared using the method disclosed in WO2013106717A1) was dissolved in 50 mL of acetonitrile, and potassium carbonate (6.2 g), benzyl bromide (1.35 mL), and tetrabutylammonium iodide (415 mg) were sequentially added. The mixture was stirred at room temperature, filtered, concentrated, and purified by silica gel column chromatography with petroleum ether/ethyl acetate as the developing solvent to give compound **7-2.**

Compounds **7-2** (121 mg) and **7-3** (180 mg) were added to a reaction flask, and 4 mL of tetrahydrofuran was added. In a nitrogen atmosphere, the mixture was cooled to about 0 °C in an ice-water bath. Potassium tert-butoxide (109 mg, 0.98 mmol) was added, and the mixture was warmed to room temperature and stirred for 40 min. 10 mL of ice water was added, and extraction was performed with ethyl acetate (20 mL × 2) and chloroform (10 mL × 5). The organic phases were combined and concentrated. The resulting residue was dissolved in 4 mL of dioxane. 2 mL of water was added, and sodium bicarbonate (49.2 mg, 0.586 mmol) and 9-fluorenylmethyl chloroformate (126 mg, 0.49 mmol) were added. The mixture was stirred at room temperature for 2 h. 20 mL of water was added, and extraction was performed with ethyl acetate (10 mL × 3). The organic phase was washed with saturated sodium chloride solution (20 mL), dried over anhydrous sodium sulfate, and filtered. The filtrate was concentrated under reduced pressure. The residue was purified by silica gel column chromatography with petroleum ether/ethyl acetate as the developing solvent to give compound **7-4**, MS m/z (ESI): 515.0 $[M+1]^+$.

Compound **7-4** (20 mg, 0.038 mmol) was dissolved in 4.5 mL of a mixture of the solvents tetrahydrofuran and ethyl acetate (V:V = 2:1), and palladium on carbon (12 mg, 10% loading, dry basis) was added. The system was purged with hydrogen gas three times, and the mixture was stirred at room temperature for 1 h. The reaction mixture was filtered through celite, and the filter cake was rinsed with ethyl acetate. The filtrate was concentrated to give a crude product of the title compound **7-5** (13 mg). The product was directly used in the next step without purification.

MS m/z (ESI): 424.9 [M+1].

**Step 2**

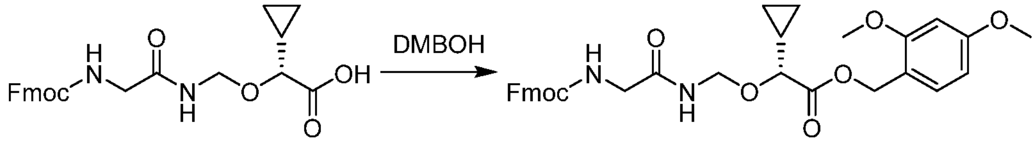


7-5 7-6

Compound **7-5** (8.00 g, 18.9 mmol, 1 eq) was placed in a 250 mL three-necked flask, and dry dichloromethane (100 mL) was added in a nitrogen atmosphere. The mixture was stirred to dissolve the compound and cooled to 0 °C, and 4-dimethylaminopyridine (250 mg, 2.05 mmol), 2,4-dimethoxybenzyl alcohol (4.45 g, 26.5 mmol), and 1-(3-dimethylaminopropyl)-3-ethylcarbodiimide (5.04 g, 28.6 mmol) were sequentially added. The mixture was stirred at 0 °C for 4 h. The reaction mixture was quenched with water (50 mL), warmed to room temperature, and extracted twice with methyl tert-butyl ether (100 mL). The organic phases were combined, washed with saturated sodium chloride solution (50 mL), dried over anhydrous sodium sulfate, and concentrated to dryness under reduced pressure to give a crude product of compound **7-6** (13.1 g).

MS Calc: 574.2, found: 575.0 $[M+H]^+$.

**Step 3**

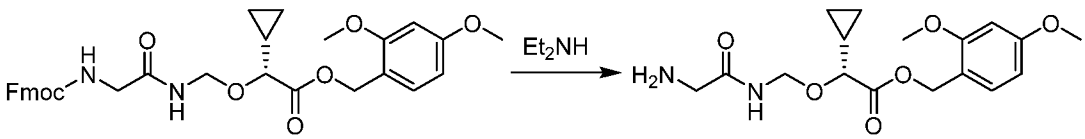


7-6 7-7

The crude product of compound **7-6** (13.1 g) was placed in a 500 mL reaction flask, and DCM (160 mL) was added. The mixture was stirred to dissolve the compound, and diethylamine (80 mL) was added. The mixture was left to react at room temperature (15-18 °C) for 3 h. The reaction mixture was concentrated to dryness under reduced pressure to give a crude product, and the crude product was separated by silica gel column chromatography. The desired fraction was collected and evaporated to dryness (water temperature < 35 °C) to give compound **7-7** as an oil (5.47 g, yield: 82.2% over two steps).

MS Calc: 352.2, found: 353.1 $[M+H]^+$.

**Step 4**

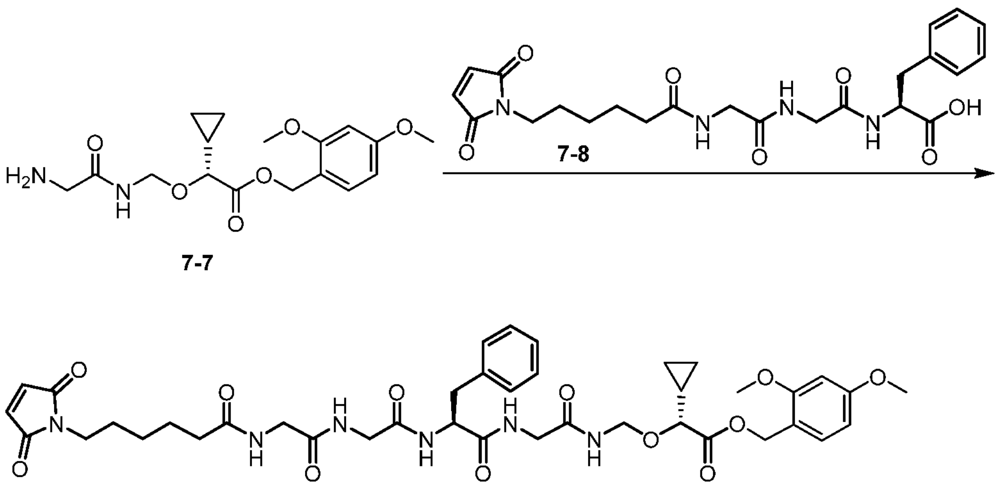


7-7

7-9

Compound **7-7** (4.36 g, 12.4 mmol) and compound **7-8** (7.03 g, 14.9 mmol, 6-(2,5-dioxo-2,5-dihydro-1H-pyrrol-1-yl)hexanoyl)glycylglycyl-*L*-phenylalanine, prepared using the method in Example 73 in the patent application "EP2907824B1") were placed in a 250 mL reaction flask, and dry N,N-dimethylformamide (50 mL) was added in a nitrogen atmosphere. The mixture was stirred to dissolve the compounds and cooled to 0 °C, and 4-(4,6-dimethoxytriazin-2-yl)-4-methylmorpholine hydrochloride (4.80 g, 16.3 mmol) was added. The mixture was warmed to 18 °C and stirred for 1 h. The reaction mixture was cooled in an ice-water bath, quenched with water (150 mL), and extracted twice with ethyl acetate (300 mL). The organic phases were combined, washed once with saturated aqueous sodium chloride solution (50 mL), dried over anhydrous sodium sulfate, filtered, and concentrated to dryness under reduced pressure. The crude product was purified by silica gel column chromatography to give an impure pale yellow solid **7-9** (6.02 g). The solid was purified by trituration with methyl tert-butyl ether (60 mL) to give compound **7-9** (5.08 g) as an off-white solid (yield: 50.8%). MS Calc: 806.4, found: 807.2 $[M+H]^+$.

**Step 5**

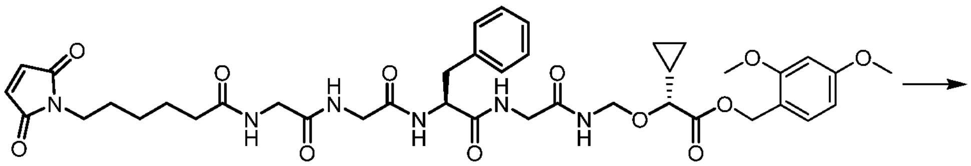

**7-9**

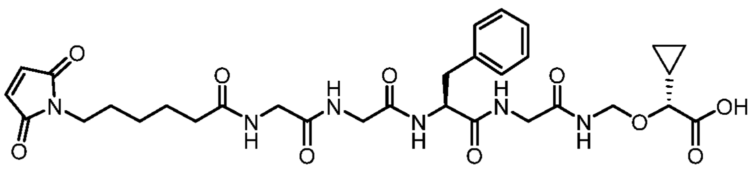

**7**

Compound **7-9** (150 mg, 0.186 mmol) was placed in a 50 mL single-necked flask, and dry dichloromethane (6 mL) and anisole (60 mg, 0.558 mmol) were added in a nitrogen atmosphere. The mixture was stirred and cooled to 0 °C, and dichloroacetic acid (0.24 mL, 2.9 mmol) was added. The substrate was dissolved, and the mixture was left to react at 0 °C for 3-4 h. The reaction mixture was triturated with methyl tert-butyl ether (18 mL) at 0 °C for 30 min. The triturate was filtered, and the filter cake was drained under reduced pressure. The solid was triturated with methyl tert-butyl ether (18 mL) at room temperature for 30 min. The triturate was filtered, and the filter cake was drained under reduced pressure to give compound **7** (120 mg, 0.183 mmol, yield: 98%). $^1$H NMR (400 MHz, DMSO-$d^6$) $\delta$ 12.56 (brs, 1H), 8.50 (t, J = 6.8 Hz, 1H), 8.28 (t, J = 6.0 Hz, 1H), 8.20-8.05 (m, 2H), 8.00 (t, J = 5.6 Hz, 1H), 7.30-7.15 (m, 5H), 6.99 (s, 2H), 4.65-4.40 (m, 3H), 3.80-3.55 (m, 7H), 3.45 (d, J = 7.6 Hz, 2H), 3.10-3.00 (m, 1H), 2.85-2.75 (m, 1H), 2.11 (t, J= 7.6 Hz, 2H), 1.55-1.40 (m, 4H), 1.25-1.15 (m, 2H), 1.05-0.95 (m, 1H), 0.55-0.40 (m, 2H), 0.40-0.30 (m, 2H).

MS Calc: 656.3, found: 679.2 $[M+Na]^+$.

**Example 4-2. Synthesis of L-9**

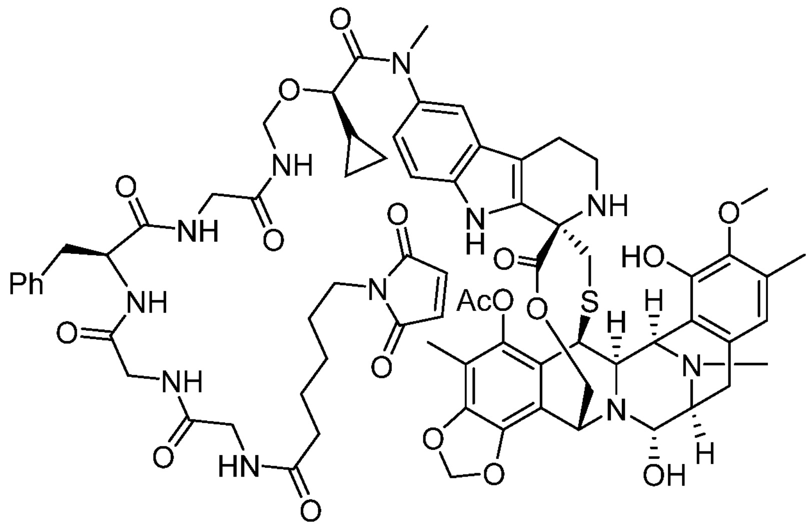

**L-9**

**Step 1**

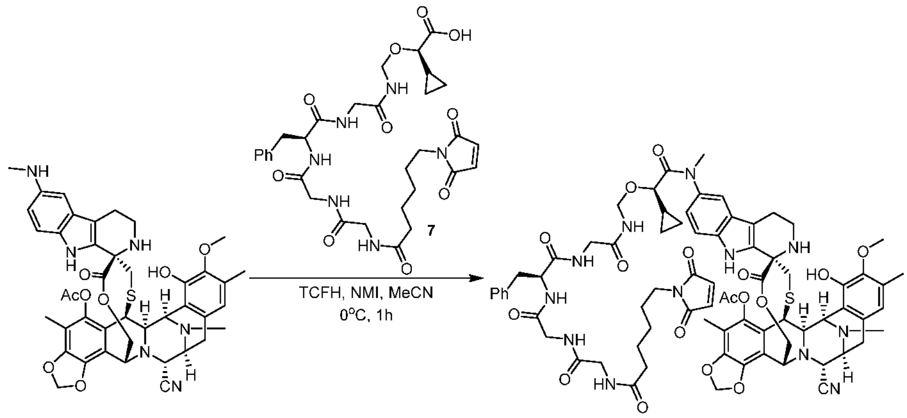


**8-6** **L-8-6**

Compound **8-6** (50 mg, 0.063 mmol, synthesized using the method described in Example 8 in WO2021218896A1) and compound **7** (85 mg, 0.13 mmol) were weighed into a reaction flask, and the system was put under a nitrogen atmosphere. A solution of N-methylimidazole (21 mg, 0.25 mmol) in dry MeCN (4 mL) was added. The system was cooled in an ice-water bath, and a solution of TCFH (45 mg, 0.16 mmol, chloro-N,N,N',N'-tetramethylformamidinium hexafluorophosphate) in dry MeCN (1 mL) was added dropwise. The mixture was left to react in the ice-water bath for 80 min. A sample was taken for LCMS analysis, and the result showed that the starting materials had been completely consumed. The reaction mixture was filtered with a sand core funnel under reduced pressure. The filter cake was washed with dry MeCN. The filtrates were combined and concentrated under reduced pressure to give a crude product, and the crude product was purified by column chromatography (methanol/dichloromethane = 1:25) to give **L-8-6** as a white solid (about 83 mg, yield: 92.22%).

MS Calc: 1430.6, found: 1431.4 [M+H].

**Step 2**

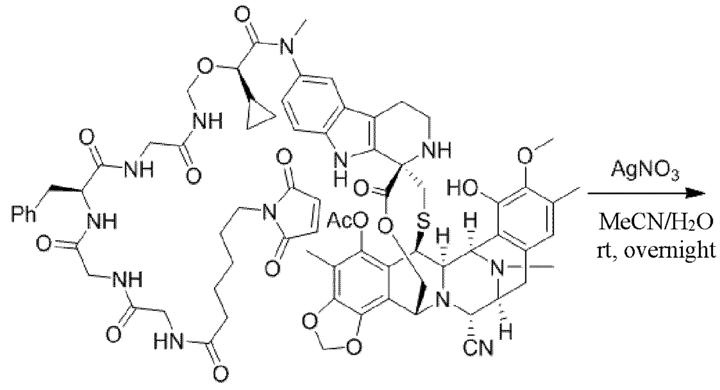


L-8-6

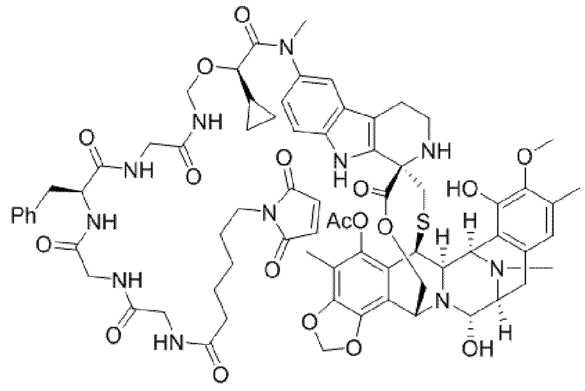

L-9

Compound **L-8-6** (81 mg, 0.057 mmol) was weighed out and dissolved in dry MeCN (7 mL). A solution of $AgNO_3$ (781 mg, 4.60 mmol) in water (4.7 mL) was added. The mixture was left to react in the dark at room temperature in a nitrogen atmosphere for 21 h. A sample was taken for LCMS analysis, and the result showed that the starting materials had been completely consumed. To the reaction mixture were added saturated aqueous $NaHCO_3$ solution (11.7 mL) and saturated NaCl solution (11.7 mL). The mixture was stirred vigorously for 30 min and filtered through celite. The filtrate was extracted with ($MeOH/CHCl_3$ = 1/10, 20 mL $\times$ 3). The organic phases were combined, dried over $Na_2SO_4$, filtered, and concentrated to dryness under reduced pressure to give a crude product, and the crude product was purified by prep-HPLC to give **L-9** as a white solid (about 52 mg, yield: 64.6%).

$^1H$ NMR (400 MHz, $CD_3OD$) $\delta$ 7.32-7.18 (m, 7H), 6.97 (d, J = 8.4 Hz, 1H), 6.75 (s, 2H), 6.66 (s, 1H), 6.25 (s, 1H), 6.07 (s, 1H), 5.23 (d, J = 11.2 Hz, 1H), 4.77 (s, 1H), 4.62-4.56 (m, 4H), 4.50 (dd, J = 6.0, 8.4 Hz, 1H), 4.41 (s, 1H), 4.18 (d, J = 10.8 Hz, 1H), 3.87-3.55 (m, 12H), 3.45 (t, J = 6.8 Hz, 2H), 3.27 (s, 3H), 3.22-3.17 (m, 2H), 3.05-2.88 (m, 3H), 2.79 (d, J = 15.2 Hz, 1H), 2.65-2.59 (m, 2H), 2.39 (s, 3H), 2.37 (s, 3H), 2.29 (s, 3H), 2.24 (t, J = 7.6 Hz, 2H), 2.12 (d, J = 15.9 Hz, 1H), 2.03 (s, 4H), 1.63-1.50 (m, 4H), 1.31-1.23 (m, 2H), 1.01-0.95 (m, 1H), 0.44-0.22 (m, 3H), -0.09--0.14 (m, 1H).

### III. Preparation of ADCs

#### Example 5-1. Preparation of ADC-1

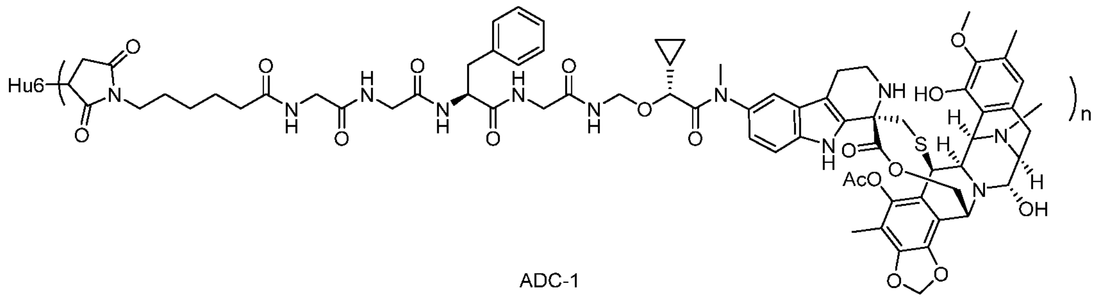

ADC-1

To an aqueous solution of antibody **Hu6** in PBS (a pH 6.5, 0.05 M aqueous PBS solution; 10.0 mg/mL, 3.6 mL, 243.1 nmol) at 37 °C was added a prepared aqueous solution of tris(2-carboxyethyl)phosphine (TCEP hydrochloride) (10 mM, 67.5 μL, 675 nmol). The mixture was shaken on a water bath shaker at 37 °C for 3 h, and then the reaction was stopped. The reaction mixture was cooled to 25 °C in a water bath. Compound **L-9** (3.46 mg, 2431 nmol) was dissolved in 170 μL of DMSO, and the solution was added to the above reaction mixture. The resulting mixture was shaken on a water bath shaker at 25 °C for 3 h, and then the reaction was stopped. The reaction mixture was desalted and purified using a Sephadex G25 gel column (elution phase: a pH 6.5, 0.05 M aqueous PBS solution) to give the title product **ADC-1** in PBS (2.83 mg/mL, 16.0 mL), and the product was stored at 4 °C.

The drug loading of each batch of ADC-1 was calculated using a HIC method, and the DAR value was: n = 3.73-4.27.

**Example 5-2. Preparation of ADC-2**

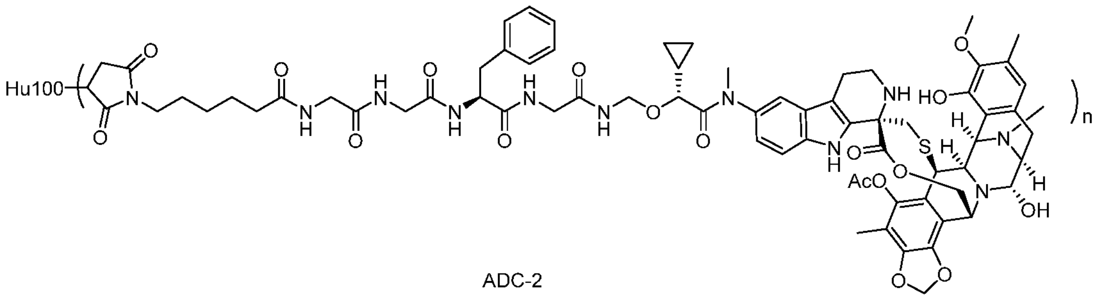

ADC-2

To an aqueous solution of antibody **Hu100** in PBS (a pH 6.5, 0.05 M aqueous PBS solution; 10.0 mg/mL, 4.0 mL, 270.1 nmol) at 37 °C was added a prepared aqueous solution of tris(2-carboxyethyl)phosphine (TCEP hydrochloride) (10 mM, 75.1 μL, 751 nmol). The mixture was shaken on a water bath shaker at 37 °C for 3 h, and then the reaction was stopped. The reaction mixture was cooled to 25 °C in a water bath. Compound **L-9** (3.84 mg, 2701 nmol) was dissolved in 190 μL of DMSO, and the solution was added to the above reaction mixture. The resulting mixture was shaken on a water bath shaker at 25 °C for 3 h, and then the reaction was stopped. The reaction mixture was desalted and purified using a Sephadex G25 gel column (elution phase: a pH 6.5, 0.05 *M* aqueous PBS solution) to give the title product **ADC-2** in PBS (3.52 mg/mL, 16.0 mL), and the product was stored at 4 °C.

The drug loading of each batch of ADC-2 was calculated using a HIC method, and the DAR value was: n = 3.72-4.88.

**Drug loading analysis of ADC stock solutions**

ADCs refer to antibody-drug conjugates. Their mechanism in treating diseases is that they deliver the drugs to cells through the targeting of the antibodies, thereby killing cells or inhibiting cell growth. Drug loading plays a decisive role in drug efficacy.

In the present disclosure, a HIC method was used to analyze drug loading, and the process was basically as follows:

**Reagents and instruments:**

Ammonium sulfate: produced by Sinopharm, 500 mg/bottle; isopropyl alcohol: LC grade, 4 L/bottle, produced by Thermo Fisher; anhydrous sodium dihydrogen phosphate: produced by Sinopharm, 500 mg/bottle.

High performance liquid chromatograph: Agilent 1260.

**Preparation of solutions:**

Mobile phase A (a 50 mM anhydrous sodium dihydrogen phosphate solution, pH 7.0): 1000 mL of purified water was measured out using a measuring cylinder, and 6.49 g of anhydrous sodium dihydrogen phosphate solid was added; after the solution was well stirred and the pH was adjusted to 7.0, it was filtered before use and was stored at 2-8 °C for 14 days.

Mobile phase B (2 M ammonium sulfate dissolved in a 50 mM anhydrous sodium dihydrogen phosphate solution, pH 7.0): 264.3 g of ammonium sulfate solid was added to the prepared 50 mM anhydrous sodium dihydrogen phosphate solution; after the solution was well stirred, it was filtered before use and was stored at 2-8 °C for 14 days. Mobile phase C (IPA): 1 L of isopropyl alcohol.

Naked antibody and test samples (concentration 1 mg/mL, about 200 μL) to be injected.

**Chromatography conditions:**

Chromatography column: TSKgel Butyl-NPR 4.6 mm × 10 cm 2.5 μm;
Column temperature: 30 °C;
DAD detector; detection wavelength 280 nm (370 nm);
Sample chamber temperature: 4 °C; flow rate: 0.5 mL/min; injection volume: 40 μL; Chromatography gradient: A% (initially 35%-15 min 75%-20 min 75%); C% (initially 5%-15 min 25%-20 min 25%).

Table 9. Mobile phase proportions

| Time (min) | Mobile phase A% | Mobile phase B% | Mobile phase C% |
|---|---|---|---|
| 0 | 35 | 60 | 5 |
| 15 | 75 | 0 | 25 |
| 20 | 75 | 0 | 25 |
| 21 | 35 | 60 | 5 |
| 31 | 35 | 60 | 5 |

**Data analysis:**

Through comparison of the spectra of the samples and the naked antibody, DAR0, DAR2, DAR4, DAR6, and DAR8 were located. Then the spectra of the test samples were integrated, and the DAR values were calculated based on peak area. The calculation formula is as follows:

$$DAR = \Sigma(\text{number of linked drugs} \times \text{percent peak area})/\text{total peak area}.$$

Below, the activity of the antibodies and ADCs of the present disclosure was validated using biochemical tests.

**Test Example 1: Protein-Level ELISA Binding Assay**

Streptavidin (abcam, ab 136200, 1 μg/mL) was plated at 100 μL/well, and the plate was incubated overnight at 4 °C. The plate was washed 3 times with 250 μL of PBST solution (PBS containing 0.1% Tween 20) per well. The plate was blocked with 250 μL of 5% milk per well at 37 °C for 2 h. The plate was washed 3 times with 250 μL of PBST solution per well. Biotinylated DLL3 antigen (1 μg/mL) (SEQ ID NO: 9) was added, and the plate was incubated at 37

°C for 1 h. The plate was washed 3 times with 250 μL of PBST solution per well. Antibodies Hu6, Hu100, BI-764532, and C25 (negative antibody) (maximum concentration 100 nM, serially diluted 4-fold) were formulated, and incubation was performed at 37 °C for 1 h. The plate was washed 6 times with 250 μL of PBST solution per well. Working concentrations of human IgG (H+L)-HRP (Jackson, 109-035-003, 1:4000 dilution) were added at 100 μL/well, and the plate was incubated at 37 °C for 1 h. The plate was washed 6 times with 250 μL of PBST solution per well. TMB (KPL, 5120-0077) substrate solution was added at 100 μL/well, and color development was allowed at room temperature for 5-10 min. 1 M $H_2SO_4$ was added at 100 μL/well to stop the color development, and microplate reader (Molecular Devices, VERSA max) readings at 450 nm were taken.

Table 10. The protein binding activity of antibodies

| Antibody | Emax (OD value) | $EC_{50}$ (nM) |
|---|---|---|
| Hu 100 | 2.05 | 0.05 |
| Hu6 | 2.13 | 0.04 |

The results show that: both antibodies Hu6 and Hu100 have excellent capacities to bind to DLL3.

**Test Example 2: Cell-Level FACS Binding Assay**

Cells of the DLL3-expressing small cell lung cancer cell lines H1184 (ATCC, Cat. No. CRL-5858), DLL3/H82, cynoDLL3/CHO-s, and RatDLL3/CHO-s were suspended at 1 × $10^6$/mL in FACS buffer (1% BSA + pH 7.4 PBS), and the cell suspensions were added to a 96-well round-bottom plate (Corning, 3795) at 100 μL/well. The plate was centrifuged at 300 g for 5 min, and the supernatant was removed. Different concentrations of test antibodies were added at 100 μL/well. The plate was incubated in the dark in a refrigerator at 4 °C for 1 h. After 3 washes by centrifugation at 300 g, working concentrations of APC anti-human IgG Fc (BioLegend, 410712) or PE F(ab')2-goat anti-human IgG (invitrogen, H10104) were added. The plate was incubated in the dark in a refrigerator at 4 °C for 40 min. After 3 washes by centrifugation at 300 g, the geometric mean fluorescence intensity was measured on an Invitrogen flow cytometer, and the EC50 values of the antibodies in binding to DLL3-expressing cells were calculated. The results are shown in Table 11-1, Table 11-2, Table 11-3, and FIG. 1A to FIG. 1C.

Table 11-1. The activity of antibodies in binding to DLL3 expressed by cells

| Cell | H1184 | | cynoDLL3/CHO-s | | RatDLL3/CHO-s | |
|---|---|---|---|---|---|---|
| Antibody | Emax | $EC_{50}$ (nM) | Emax | $EC_{50}$ (nM) | Emax | $EC_{50}$ (nM) |
| Hu6 | 2073 | 0.06 | 1762 | 0.32 | 2112 | 1.42 |
| Hu 100 | 2112 | 0.15 | 2075 | 0.55 | No binding | |
| BI-764532 | 1625 | 3.94 | 963 | 3.42 | No binding | |

Table 11-2. The activity of antibodies in binding to DLL3/H82 cells expressing human DLL3

| Antibody | Emax | $EC_{50}$ (nM) |
|---|---|---|
| M100CHI | 13513 | 0.25 |
| hAb100L1H1 | 14315 | 0.24 |
| hAb100L1H2 | 14807 | 0.30 |
| hAb100L1H3 | 14865 | 0.25 |
| hAb100L1H4 | 14908 | 0.22 |
| hAb100L2H1 | 14409 | 0.29 |
| hAb100L2H2 | 14821 | 0.16 |
| hAb100L2H3 | 14785 | 0.30 |
| hAb100L2H4 | 13159 | 0.32 |

Table 11-3. The activity of antibodies in binding to H1184 cells

| Antibody | Emax | $EC_{50}$ (nM) |
|---|---|---|
| M6CHI | 3367 | 0.14 |
| hAb6L2H2 | 2193 | 0.06 |
| hAb6L4H4 | 2312 | 0.06 |
| hAb6L2H5 | 2486 | 0.06 |
| hAb6L2H6 | 2422 | 0.05 |
| hAb6L2H7 | 2196 | 0.07 |
| | | |
| hAb6L2H11 | 1959 | 0.04 |
| hAb6L2H12 | 2145 | 0.10 |
| hAb6L2H13 | 2105 | 0.10 |
| hAb6L4H6 | 2004 | 0.17 |
| hAb6L4H11 | 2188 | 0.07 |
| hAb6L4H12 | 2131 | 0.05 |
| hAb6L4H13 | 2118 | 0.09 |

The results show that: the antibodies in the present disclosure can all bind specifically to DLL3 expressed by cells, and among them, Hu6 exhibited relatively high capacities to bind to cells expressing DLL3 of different species. Hu100 exhibited excellent capacities to bind to cells expressing human and cynomolgus monkey DLL3, but Hu100 did not bind to cells expressing rat DLL3. BI-764532 only exhibited capacities to bind to cells expressing human and cynomolgus monkey DLL3, and its binding activity was weaker than that of Hu6 and Hu100.

**Test Example 3: DLL1 and DLL4 Binding Assays**

Stably transfected human DLL1/CHO-s and human DLL4/CHO-s cells were suspended at $1 \times 10^6$/mL in FACS buffer (containing 1% BSA and pH 7.4 PBS), and the cell suspensions were added to a 96-well round-bottom plate (Corning, 3795) at 100 μL/well. The plate was centrifuged at 300 g for 5 min, and the supernatant was removed. Test antibodies were added at 100 μL/well, and the plate was incubated in the dark in a refrigerator at 4 °C for 1 h. After 3 washes by centrifugation at 300 g, working concentrations of PE F(ab')2-goat anti-human IgG Fc secondary antibody (invitrogen, H10104) were added. The plate was incubated in the dark in a refrigerator at 4 °C for 40 min. After 3 washes by centrifugation at 300 g, the geometric mean fluorescence intensity was measured on an Invitrogen flow cytometer.

The results show that: neither antibody Hu6 nor Hu100 bound to human DLL1 or DLL4.

**Test Example 4: Biacore Antibody Affinity Assay**

Test antibodies were affinity-captured with a Protein A biosensor chip (Cat. # 29127556, Cytiva) for 18 seconds, and then the antigens human DLL3 (ACRO, DLL3-H52H4), cynomolgus monkey DLL3 (KACTUS, DLL-RM103), and mouse DLL3 (KACTUS, DLL-MM103) were allowed to flow over the surface of the chip for 180 seconds, followed by 600 seconds of dissociation. Reaction signals were detected in real time using a Biacore 8K (Cytiva) instrument to obtain association and dissociation curves. After the dissociation in each experimental cycle, the biosensor chip was washed and regenerated with a 10 mM glycine-hydrochloric acid solution (pH 1.5) (Cat. # BR-1003-54, Cytiva). A 1:1 model was fit to the data. The results are shown in Table 12-1, Table 12-2, and Table 12-3.

Table 12-1. Antibody affinity

| Antigen \ Antibody | Hu6 KD (M) | Hu100 KD (M) |
|---|---|---|
| Human DLL3 | 5.94E-10 | 4.71E-10 |
| Cynomolgus monkey DLL3 | 1.31E-09 | 1.11E-09 |
| Mouse DLL3 | 6.52E-09 | No binding |

Table 12-2. The affinity of antibodies for human DLL3

| Antibody | ka (1/Ms) | kd (1/s) | KD (M) |
|---|---|---|---|
| M100CHI | 1.03E+06 | 5.46E-04 | 5.28E-10 |
| hAb100L1H1 | 8.58E+05 | 3.74E-04 | 4.36E-10 |
| hAb100L1H2 | 7.81E+05 | 3.65E-04 | 4.67E-10 |
| hAb100L1H3 | 8.32E+05 | 3.52E-04 | 4.24E-10 |
| hAb100L1H4 | 3.48E+05 | 2.54E-04 | 7.29E-10 |
| hAb100L2H1 | 5.72E+05 | 6.29E-04 | 1.10E-09 |
| hAb100L2H2 | 8.48E+05 | 3.70E-04 | 4.37E-10 |
| hAb100L2H3 | 4.04E+05 | 2.68E-04 | 6.64E-10 |
| hAb100L2H4 | 6.52E+05 | 6.55E-04 | 1.00E-09 |

Table 12-3. The affinity of antibodies for human DLL3

| Antibody | ka (1/Ms) | kd (1/s) | KD (M) |
|---|---|---|---|
| M6CHI | 9.65E+05 | 6.57E-04 | 6.81E-10 |
| hAb6L1H1 | 7.98E+05 | 2.25E-03 | 2.81E-09 |
| hAb6L1H2 | 7.87E+05 | 1.73E-03 | 2.19E-09 |
| hAb6L1H3 | 8.12E+05 | 2.24E-03 | 2.76E-09 |
| hAb6L2H1 | 7.09E+05 | 1.55E-03 | 2.19E-09 |
| hAb6L2H2 | 7.04E+05 | 1.16E-03 | 1.64E-09 |
| hAb6L2H3 | 6.95E+05 | 1.39E-03 | 2.01E-09 |
| hAb6L3H1 | 7.15E+05 | 1.44E-03 | 2.02E-09 |
| hAb6L3H2 | 7.16E+05 | 1.14E-03 | 1.59E-09 |
| hAb6L3H3 | 7.18E+05 | 1.35E-03 | 1.88E-09 |
| hAb6L4H4 | 7.43E+05 | 1.08E-03 | 1.45E-09 |
| hAb6L2H5 | 8.10E+05 | 7.84E-04 | 9.69E-10 |
| hAb6L2H6 | 8.63E+05 | 7.56E-04 | 8.77E-10 |
| hAb6L2H7 | 6.70E+05 | 2.05E-03 | 3.06E-09 |
| hAb6L2H11 | 2.06E+06 | 7.49E-04 | 3.63E-10 |
| hAb6L2H12 | 1.98E+06 | 9.13E-04 | 4.61E-10 |
| hAb6L2H13 | 2.10E+06 | 8.03E-04 | 3.82E-10 |

(continued)

| Antibody | ka (1/Ms) | kd (1/s) | KD (M) |
|---|---|---|---|
| hAb6L4H6 | 1.91E+06 | 8.11E-04 | 4.24E-10 |
| hAb6L4H11 | 2.14E+06 | 6.72E-04 | 3.14E-10 |
| hAb6L4H12 | 2.06E+06 | 8.05E-04 | 3.91E-10 |
| hAb6L4H13 | 2.16E+06 | 6.95E-04 | 3.22E-10 |

The results show that the humanized and chimeric antibodies of mAb100 and mAb6 can bind specifically to human DLL3 with relatively high affinity. Antibody Hu6 exhibited relatively high affinity for human, cynomolgus monkey, and mouse DLL3; Hu100 exhibited relatively high affinity for human and cynomolgus monkey DLL3 but did not bind to mouse DLL3.

### Test Example 5: Competitive Epitope Binding Assay of Antibodies

The BI-764532 antibody (1 μg/mL) was plated at 100 μL/well, and the plate was incubated overnight at 4 °C. The plate was washed 3 times with 250 μL of PBST solution per well. The plate was blocked with 250 μL of 5% milk per well at 37 °C for 2 h. The plate was washed 3 times with 250 μL of PBST solution per well. Biotinylated DLL3-Strep (0.1 μg/mL, SEQ ID NO: 9) was added. Competitive antibodies BI-764532, Hu6, and Hu100 were formulated (maximum concentration 100 μg/mL, serially diluted 4-fold) and incubation was performed at 37 °C for 1 h. The plate was washed 6 times with 250 μL of PBST solution per well. Streptavidin-peroxidase (1:2000 dilution) (Jackson Immuno Research, 016-030-084) was added at 100 μL/well, and the plate was incubated at 37 °C for 1 h. The plate was washed 6 times with 250 μL of PBST solution per well. TMB (KPL, 5120-0077) substrate solution was added at 100 μL/well, and color development was allowed at room temperature for 5-10 min. 1 M $H_2SO_4$ was added at 100 μL/well to stop the color development, and microplate reader readings at 450 nm were taken. The results are shown in FIG. 2.

The results show that there was no competition between antibodies Hu6 and Hu100 and BI-764532, indicating that antibodies Hu6 and Hu100 bind to different epitopes than BI-764532 does.

### Test Example 6: Assay for Endocytic Activity of Anti-DLL3 Antibodies

DT3C is a recombinantly expressed fusion protein and is formed by fusing fragment A of diphtheria toxin (the toxin moiety only) and fragment 3C of group G streptococcus (the IgG binding moiety). The protein can have a high affinity for the IgG moiety of the antibody, enter cells when the antibody is endocytosed, and release toxic DT under the action of intracellular furin protease. DT can inhibit the activity of EF2-ADP ribosylation, blocking the protein translation process and finally causing cell death. DT3C that does not enter the cell has no cell-killing activity. The endocytosis activity of the antibodies was evaluated based on the killing of cells.

Experimental procedure

a. A suspension of DMS53/DLL3 cells was prepared in fresh cell culture medium RPMI1640 (GE, SH30809.01) containing 20% FBS and added to a 96-well cell culture plate at 2000 cells/50 μL/well, with columns 1 and 12 containing only 50 μL of medium and no cells. The plate was incubated at 37 °C with 5% carbon dioxide for 16 h.
b. A 4× solution of DT3C (9600 nM, expressed and purified by Shanghai Panchao Biotechnology Co., Ltd.) was prepared in serum-free medium and filtered using a 0.22 μm filter. A 4× antibody solution (1600 nM) was prepared in serum-free medium. 80 μL of the DT3C solution and 80 μL of the antibody solution were mixed in a 1:1 ratio by volume, and the mixture was incubated at room temperature for 30 min.
c. The mixture was serially diluted 5-fold with serum-free medium for 9 concentrations, with the 10th point being pure medium.
d. 50 μL of antibody dilution was added to cells, and the plate was incubated in an incubator for three days.
e. CTG (CellTiter-Glo® Luminescent Cell Viability Assay, Promega, G7573) was added at 50 μL/well. The plate was incubated in the dark at room temperature for 10 min, and chemiluminescence was measured on Victor3.

The results are shown in Table 13 below and FIG. 3.

Table 13. Cell killing caused by endocytosis of antibodies

| Antibody | IC50 (nM) | Imax |
|---|---|---|
| Hu6 | 0.56 | 87.16 |
| Hu100 | 1.47 | 86.13 |
| BI-764532 | 3.27 | 86.51 |

The results show that: both antibodies Hu6 and Hu100 can be endocytosed by cells.

**Test Example 7: Assay for Toxicity of ADCs to Cells with Different DLL3 Expression Levels**

The experimental procedure was as follows:

a. A cell suspension was prepared in fresh cell culture medium containing 10% FBS and added to a 96-well cell culture plate (Corning, 3903) at 135 μL/well, with columns 1 and 12 containing 135 μL of medium and no cells. The plate was incubated at 37 °C with 5% carbon dioxide for 16 h.
b. First-well working solutions of ADC samples (10×) were prepared in PBS, and with this concentration as the initial concentration, a serial dilution was performed with PBS by a corresponding factor. 10× ADC solutions were added at 15 μL/well, and the plate was incubated at 37 °C with 5% carbon dioxide for 6 days.
c. CTG (Promega, G7573) was added at 70 μL/well. The plate was incubated in the dark at room temperature for 10 min, and chemiluminescence was measured on Victor3. Data were processed using GraphPad Prism5, with the X-axis representing antibody or ADC concentration and the Y-axis representing light intensity.

The plating densities for different cells, first-well working solution concentrations (10×), and dilution factors are shown in Table 14.

Table 14. Cell plating densities and first-well working solution concentrations

| Cell | Medium | Number of cells per well | Concentration (nM) | Serial dilution |
|---|---|---|---|---|
| DMS53 | RPMI1640+20%FB S+1× GlutaMAX | 1000 | 6400 | 2.5-fold dilution, 14 concentrations |
| H1184 | RPMI1640+5%FB S | 1000 | 800 | 5-fold dilution, 9 concentrations |
| HT-29 | McCoy's 5A+10% FBS | 500 | 6400 | 2.5-fold dilution, 15 concentrations |
| U-2 OS | McCoy's 5A+10% FBS | 500 | 6400 | 2.5-fold dilution, 15 concentrations |
| CHO-K1 | DMEM/F12(1:1)+10 %FBS | 500 | 40000 | 2.5-fold dilution, 17 concentrations |

The cells used in the assay were as follows:

DMS53 (+) purchased from ATCC, CRL-2062;
H1184 (+++) purchased from ATCC, CRL-5858;
U-2OS (-) purchased from ATCC, HTB-96,
HT-29 (-) purchased from ATCC, HTB-38;
CHO-K1 (-) purchased from ATCC, CCL-61.
"+" indicates the expression level of DLL3, and "-" indicates that DLL3 is not expressed.

The results are shown in Table 15 and FIG. 4A to FIG. 4E.

Table 15. The killing activity of ADCs against cells with different DLL3 expression levels

| ADC | DMS53 (+) | | H1184 (+++) | |
|---|---|---|---|---|
| | IC50 (nM) | Imax (%) | IC50 (nM) | Imax (%) |
| ADC-1 | 8.27 | 99.37 | 0.11 | 99.56 |
| ADC-2 | 13.12 | 99.37 | 0.11 | 99.71 |

The results show that ADC-1 and ADC-2 have relatively strong target cell-killing activity: they can kill DLL3-expressing DMA53 and H1184 but cannot kill DLL3-negative U-2OS, HT-29, and CHO-K1 cells.

**Test Example 8: Assay for Bystander Killing Activity**

DMS53/DLL3high (DMS53 cells stably transfected with DLL3) and U-2OS (ATCC, HTB-96) cells were cultured with RPMI1640 + 20% FBS + 1 × Glutamax and McCoy's 5A + 10% FBS, respectively. The cells were trypsinized, neutralized with fresh medium, and centrifuged at 1000 rpm for 3 min. The supernatant was discarded, and the cells were resuspended in RPMI1640 + 20% FBS + 1× Glutamax. After cell counting, the density of DMS53/DLL3high cells was adjusted to $9 \times 10^4$ cells/mL, and the density of U-2OS cells was adjusted to $3 \times 10^4$ cells/mL. 500 μL of DMS53/DLL3high cells and 500 μL of U-2OS cells were added to each of the corresponding wells of a 12-well plate. 500 μL of U-2OS cells and 500 μL of RPMI1640 + 20% FBS + 1× Glutamax medium were added to the corresponding wells of a 12-well plate. The plates were incubated at 37 °C with 5% carbon dioxide for 24 h. 40× intermediate solutions (200 nM) of samples were prepared. 25 μL of each of the above ADC samples was taken and added to the corresponding wells of the 12-well plates. A solvent control group was set up. The plates were cultured at 37 °C with 5% carbon dioxide for 6 days. The cells in the 12-well plates were trypsinized and neutralized with fresh medium. 20 μL of cells were taken, and 20 μL of trypan blue was added. The cells were counted. The cells were centrifuged at 1000 rpm for 3 min, and the supernatant was discarded. The cells were washed once with 100 μL of FACS buffer and centrifuged at 1500 rpm for 3 min, and the supernatant was discarded. The cells were resuspended in 100 μL of FACS buffer, and 2 μg/mL anti-DLL3 positive antibody was added. The suspension was incubated on ice for 60 min. The cells were washed once with FACS buffer and centrifuged at 1500 rpm for 3 min, and the secondary antibody anti-APC anti-human IgG Fc (100×) was added. The cells were incubated for 30 min and washed once with FACS buffer. 200 μL of FACS buffer was added to resuspend the cells, and the suspension was assayed by FACS. The flow cytometry data were analyzed using FlowJo to obtain the proportion of DMS53/DLL3high, and the total quantity of DMS53/DLL3high and U-2OS was calculated. The data were plotted using GraphPad Prism5, with the X-axis representing different samples and the Y-axis representing the calculated cell number. The results are shown in FIG. 5.

The results show that ADC-1 and ADC-2 have significant bystander cytotoxic effects.

Biological Evaluations of ***In Vivo*** **Activity**

**Test Example 9: Evaluation *of In Vivo* Efficacy in CDX Mouse Model of DMS53 Cells**

200 μL of human small cell lung cancer DMS53 cells ($5 \times 10^6$ cells, containing 50% matrigel/mouse; ATCC, CRL-2062) was subcutaneously inoculated into the right flank of each Balb/c mouse. 21 days after inoculation, when the tumor volume was about 200 $mm^3$, mice that were overweight and had tumors that were either too big or too small were excluded. Mice were randomized into groups of 8 according to tumor volume, and administration was started that day. ADC was administered intraperitoneally at a dose of 1.8 mg/kg once a week, and a total of 2 administrations were performed. The tumor volume and body weight were measured twice a week, and data were recorded. Data were recorded using Excel statistical software: averages were calculated using avg; SD values were calculated using STDEV; SEM values were calculated using STDEV/SQRT (number of animals per group); graphs were generated using GraphPad Prism software, and statistical analysis of data was performed using Two-way ANOVA or One-way ANOVA.

Tumor volume (V) was calculated using the formula:

$$V = 1/2 \times L_{long} \times L_{short}^2$$

Relative tumor proliferation rate T/C (%) = $(T - T_0)/(C - C_0) \times 100\%$, where T and C represent the tumor volumes of the treatment group and the control group at the end of the experiment; $T_0$ and $C_0$ represent the tumor volumes at

the beginning of the experiment.

$$\text{Tumor growth inhibition rate TGI (\%)} = 1 - \text{T/C (\%)}.$$

The results are shown in Table 16 and FIG. 6.

Table 16. The efficacy of ADCs against DMS53 xenograft tumors in tumor-bearing nude mice

| ADC | Day-18 TGI (%) |
|---|---|
| ADC-1 | 56.71 |
| ADC-2 | 58.71 |

The results show that at the dose of 1.8 mpk, both ADC-1 and ADC-2 can significantly inhibit the growth of subcutaneous xenograft tumors of DMS53 cells.

**Test Example 10: Evaluation *of In Vivo* Efficacy in CDX Mouse Model of H1184 Cells**

200 μL of human small cell lung cancer NCI-H1184 cells ($5 \times 10^6$ cells, containing 50% matrigel; ATCC, CRL-5858) was subcutaneously inoculated into the right flank of each NDG mouse. 18 days after inoculation, when the tumor volume was 180 $mm^3$, mice that were overweight and had tumors that were either too big or too small were excluded. Mice were randomized into groups of 8 according to tumor volume, and administration was started that day. ADCs were injected intraperitoneally at a dose of 1.8 mg/kg. The ADCs were administered once a week, and 3 administrations were performed. The tumor volume and body weight were measured twice a week, and data were recorded.

Data were recorded using Excel statistical software: averages were calculated using avg; SD values were calculated using STDEV; SEM values were calculated using STDEV/SQRT (number of animals per group); graphs were generated using GraphPad Prism software, and statistical analysis of data was performed using Two-way ANOVA or One-way ANOVA.

Tumor volume (V) was calculated using the formula:

$$V = 1/2 \times L_{long} \times L_{short}^2$$

Relative tumor proliferation rate T/C (%) = $(T - T_0)/(C - C_0) \times 100\%$, where T and C represent the tumor volumes of the treatment group and the control group at the end of the experiment; $T_0$ and $C_0$ represent the tumor volumes at the beginning of the experiment.

$$\text{Tumor growth inhibition rate TGI (\%)} = 1 - \text{T/C (\%)}.$$

The results are shown in Table 17 and FIG. 7.

Table 17. The efficacy of ADCs against H1184 xenograft tumors in tumor-bearing nude mice

| ADC | Day-18 TGI (%) |
|---|---|
| ADC-1 | 109 |
| ADC-2 | 107 |

The results show that at the dose of 1.8 mpk, both ADC-1 and ADC-2 can significantly inhibit the growth of H1184 xenograft tumors.

## Claims

**1.** An anti-DLL3 antibody, comprising a heavy chain variable region and a light chain variable region, wherein:

i) the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 57; and

the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27;
wherein SEQ ID NO: 57 is represented by PLYX$_1$YGRSYNX$_2$VAY, wherein Xi is Y or H; X$_2$ is A or G; or ii) the heavy chain variable region comprises: a HCDR1 comprising the amino acid sequence of SEQ ID NO: 16; a HCDR2 comprising the amino acid sequence of SEQ ID NO: 17; and a HCDR3 comprising the amino acid sequence of SEQ ID NO: 18; and
the light chain variable region comprises: a LCDR1 comprising the amino acid sequence of SEQ ID NO: 19; a LCDR2 comprising the amino acid sequence of SEQ ID NO: 20; and a LCDR3 comprising the amino acid sequence of SEQ ID NO: 21;
preferably,
the heavy chain variable region comprises: a HCDR1 comprising the amino acid sequence of SEQ ID NO: 22; a HCDR2 comprising the amino acid sequence of SEQ ID NO: 23; and a HCDR3 comprising the amino acid sequence of SEQ ID NO: 24, 30, or 31; and
the light chain variable region comprises: a LCDR1 comprising the amino acid sequence of SEQ ID NO: 25; a LCDR2 comprising the amino acid sequence of SEQ ID NO: 26; and a LCDR3 comprising the amino acid sequence of SEQ ID NO: 27.

**2.** The anti-DLL3 antibody according to claim 1, being a murine antibody, a chimeric antibody, or a humanized antibody.

**3.** The anti-DLL3 antibody according to claim 1 or 2, wherein:

i) the heavy chain variable region comprises an amino acid sequence having at least 90% identity to SEQ ID NO: 50, 14, 51, 52, 53, or 54; and/or
the light chain variable region comprises an amino acid sequence having at least 90% identity to SEQ ID NO: 55, 15, or 56; or
ii) the heavy chain variable region comprises an amino acid sequence having at least 90% identity to SEQ ID NO: 43, 12, 32, 33, 34, 35, 36, 37, 38, 39, 40, 41, 42, or 44; and/or

the light chain variable region comprises an amino acid sequence having at least 90% identity to SEQ ID NO: 48, 13, 45, 46, 47, or 49;
preferably,

i) the heavy chain variable region comprises an amino acid sequence selected from the group consisting of any one of SEQ ID NOs: 50, 51, 52, 53, and 54, and/or the light chain variable region comprises the amino acid sequence of SEQ ID NO: 55 or 56; or
ii) the heavy chain variable region comprises an amino acid sequence selected from the group consisting of any one of SEQ ID NOs: 43, 32, 33, 34, 35, 36, 37, 38, 39, 40, 41, 42, and 44, and/or the light chain variable region comprises an amino acid sequence selected from the group consisting of any one of SEQ ID NOs: 48, 45, 46, 47, and 49; or
iii) the heavy chain variable region comprises the amino acid sequence of SEQ ID NO: 14, and/or the light chain variable region comprises the amino acid sequence of SEQ ID NO: 15; or
iv) the heavy chain variable region comprises the amino acid sequence of SEQ ID NO: 12, and/or the light chain variable region comprises the amino acid sequence of SEQ ID NO: 13;

more preferably,
the heavy chain variable region comprises the amino acid sequence of SEQ ID NO: 50, and the light chain variable region comprises the amino acid sequence of SEQ ID NO: 55; or
the heavy chain variable region comprises the amino acid sequence of SEQ ID NO: 43, and the light chain variable region comprises the amino acid sequence of SEQ ID NO: 48.

**4.** The anti-DLL3 antibody according to any one of claims 1 to 3, wherein the anti-DLL3 antibody is an antibody fragment; preferably, the antibody fragment is a Fab, Fab', F(ab')$_2$, Fab'-SH, Fd, Fv, scFv, dsFv, diabody, or domain antibody.

**5.** The anti-DLL3 antibody according to any one of claims 1 to 3, comprising a heavy chain constant region and a light chain constant region, wherein preferably, the heavy chain constant region comprises the amino acid sequence of SEQ ID NO: 28, and/or the light chain constant region comprises the amino acid sequence of SEQ ID NO: 29.

**6.** The anti-DLL3 antibody according to claim 5, comprising a heavy chain and a light chain, wherein:

the heavy chain comprises an amino acid sequence having at least 85% identity to SEQ ID NO: 60, and/or the light chain comprises an amino acid sequence having at least 85% identity to SEQ ID NO: 61; or
the heavy chain comprises an amino acid sequence having at least 85% identity to SEQ ID NO: 58, and/or the light chain comprises an amino acid sequence having at least 85% identity to SEQ ID NO: 59;
preferably,
the heavy chain comprises the amino acid sequence of SEQ ID NO: 60, and the light chain comprises the amino acid sequence of SEQ ID NO: 61; or
the heavy chain comprises the amino acid sequence of SEQ ID NO: 58, and the light chain comprises the amino acid sequence of SEQ ID NO: 59.

**7.** The anti-DLL3 antibody according to any one of claims 1 to 6, having at least one of the following properties:

a) the anti-DLL3 antibody binding to human DLL3 or an epitope thereof with a KD value of ≤3 nM, ≤2 nM, or ≤1 nM, as determined by Biacore;
b) the anti-DLL3 antibody binding to DLL3-expressing H1184 cells with an EC50 of ≤3 nM, as determined by FACS;
c) the anti-DLL3 antibody being capable of being endocytosed by a DLL3-expressing cell; and
d) the anti-DLL3 antibody binding to DLL3 or an epitope thereof with an $EC_{50}$ of ≤0.1 nM, as determined by ELISA.

**8.** An isolated nucleic acid, encoding the anti-DLL3 antibody according to any one of claims 1 to 7.

**9.** A host cell, comprising the isolated nucleic acid according to claim 8.

**10.** A method for preparing an anti-DLL3 antibody, comprising culturing the host cell according to claim 9 under conditions suitable for expression of the antibody.

**11.** An antibody-drug conjugate or a pharmaceutically acceptable salt thereof, comprising:

the anti-DLL3 antibody according to any one of claims 1 to 7, and
a drug;
wherein preferably, the drug is selected from the group consisting of: one or more of a cytotoxic agent, a radiolabel, a fluorophore, a chromophore, an imaging agent, an immunomodulator, an inhibitor of angiogenesis, an inhibitor of cell proliferation, a pro-apoptotic agent, and a lytic enzyme.

**12.** The antibody-drug conjugate or the pharmaceutically acceptable salt thereof according to claim 11, having a structure represented by general formula (Pc-L-Da):

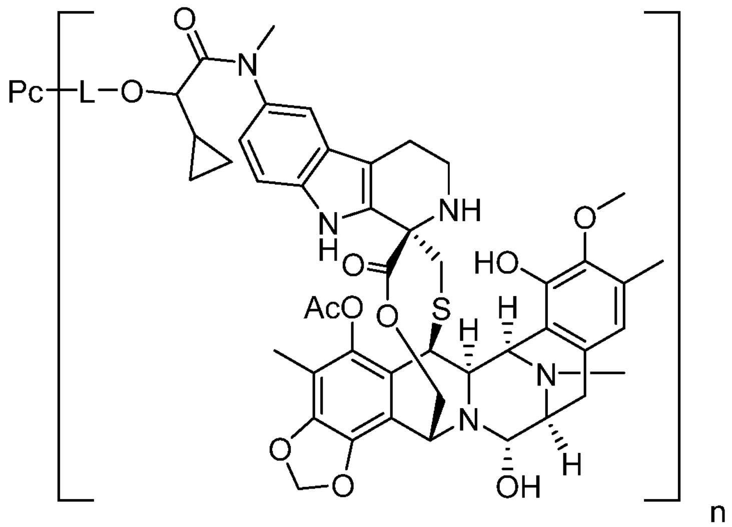

Pc-L-Da

wherein:

Pc is the anti-DLL3 antibody according to any one of claims 1 to 7;
L is a linker;
n is 1 to 10; preferably, n is 3 to 5.

**13.** The antibody-drug conjugate or the pharmaceutically acceptable salt thereof according to claim 11 or 12, having a structure represented by general formula (Pc-L-D):

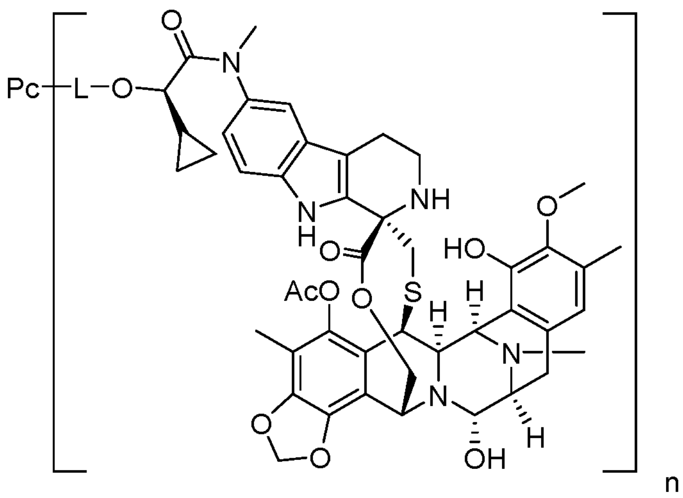

Pc-L-D

wherein:

Pc is the anti-DLL3 antibody according to any one of claims 1 to 7;
L is a linker;
n is 1 to 10; preferably, n is 3 to 5.

**14.** The antibody-drug conjugate or the pharmaceutically acceptable salt thereof according to claim 12 or 13, wherein the linker is $-L^1-L^2-L^3-L^4-$, wherein:

$L^1$ is selected from the group consisting of -(succinimid-3-yl-N)-W-C(O)-, $-CH_2-C(O)-NR^3-W-C(O)-$, and -C(O)-W-C(O)-, wherein W is selected from the group consisting of $C_{1-6}$ alkylene and $C_{1-6}$ alkylene-$C_{3-6}$ cy-

cloalkyl, wherein the $C_{1-6}$ alkylene and $C_{1-6}$ alkylene-$C_{3-6}$ cycloalkyl are each independently optionally further substituted with one or more substituents selected from the group consisting of halogen, hydroxy, cyano, amino, alkyl, chloroalkyl, deuterated alkyl, alkoxy, and cycloalkyl;

$L^2$ is selected from the group consisting of $-NR^4(CH_2CH_2O)_pCH_2CH_2C(O)-$, $-NR^4(CH_2CH_2O)_pCH_2C(O)-$, $-S(CH_2)_pC(O)-$, and a chemical bond, wherein p is an integer from 1 to 20;

$L^3$ is a peptide residue consisting of 2 to 7 amino acid residues, wherein the amino acids are selected from the group consisting of phenylalanine, glycine, valine, lysine, citrulline, serine, glutamic acid, and aspartic acid, and are optionally further substituted with one or more substituents selected from the group consisting of halogen, hydroxy, cyano, amino, alkyl, chloroalkyl, deuterated alkyl, alkoxy, and cycloalkyl;

$L^4$ is selected from the group consisting of $-NR^5(CR^6R^7)_t-$, $-C(O)NR^5$, $-C(O)NR^5(CH_2)_t-$, and a chemical bond, wherein t is an integer from 1 to 6;

$R^3$, $R^4$, and $R^5$ are identical or different and are each independently selected from the group consisting of a hydrogen atom, alkyl, haloalkyl, deuterated alkyl, and hydroxyalkyl;

$R^6$ and $R^7$ are identical or different and are each independently selected from the group consisting of a hydrogen atom, halogen, alkyl, haloalkyl, deuterated alkyl, and hydroxyalkyl;

preferably,

$L^1$ is

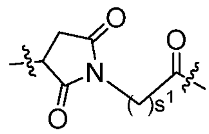

,

and $s^1$ is an integer from 2 to 8;

$L^2$ is a chemical bond;

$L^3$ is a tetrapeptide residue; preferably, $L^3$ is a tetrapeptide residue comprising glycine-glycine-phenylalanine-glycine;

$L^4$ is $-NH(CH_2)t-$, and t is 1 or 2;

wherein the $L^1$ end is attached to Pc;

more preferably, the linker is represented by the following structure:

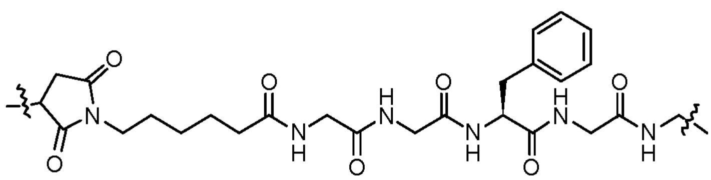

.

**15.** The antibody-drug conjugate or the pharmaceutically acceptable salt thereof according to any one of claims 11 to 14, wherein the antibody-drug conjugate has a structure selected from the group consisting of the following:

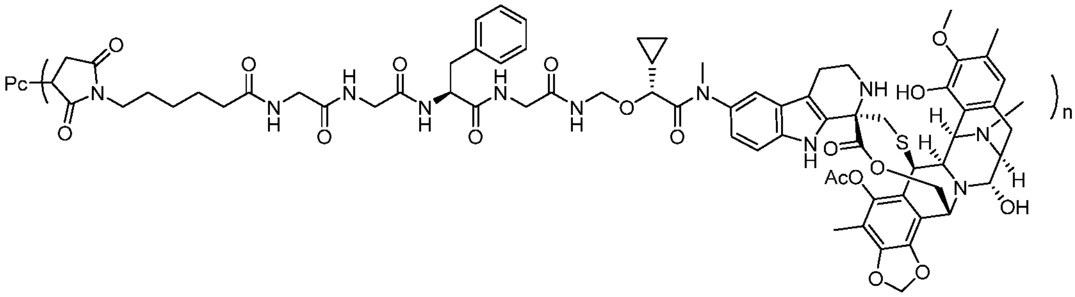

,

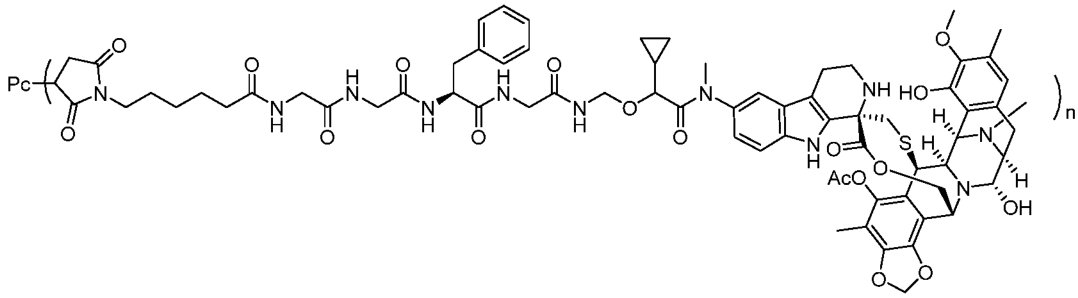

,

or

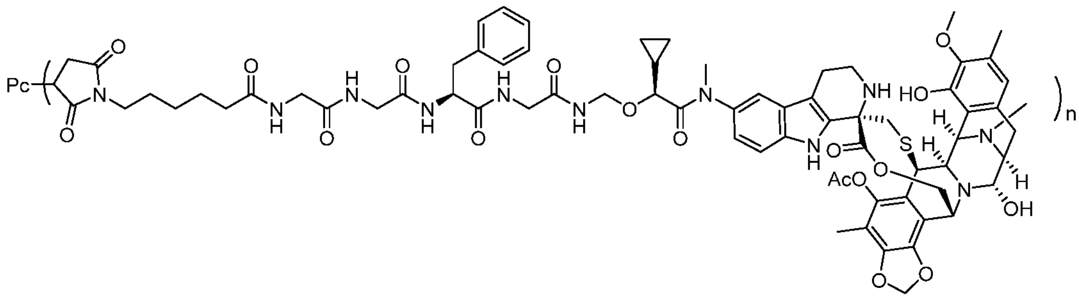

;

wherein:

Pc is the anti-DLL3 antibody according to any one of claims 1 to 7; n is 1 to 10;
preferably, the antibody-drug conjugate has the following structure:

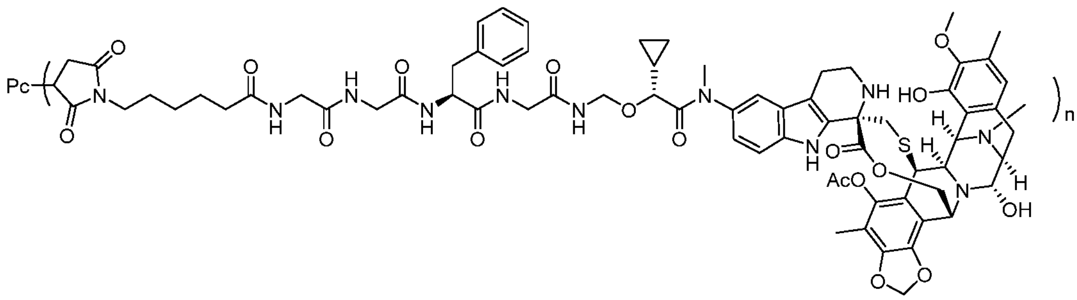

,

wherein:

Pc is the anti-DLL3 antibody according to claim 6;
wherein n is 1 to 8; more preferably, n is 3 to 5.

**16.** A pharmaceutical composition, comprising:

the anti-DLL3 antibody according to any one of claims 1 to 7, or the antibody-drug conjugate or the pharmaceutically acceptable salt thereof according to any one of claims 11 to 15, and
one or more pharmaceutically acceptable excipients, diluents, or carriers.

**17.** Use of the anti-DLL3 antibody according to any one of claims 1 to 7, or the antibody-drug conjugate or the pharmaceutically acceptable salt thereof according to any one of claims 11 to 15, or the pharmaceutical composition according to claim 16, in the preparation of a medicament for treating a tumor or cancer, wherein preferably, the tumor or cancer is selected from the group consisting of:
lung cancer, small cell lung cancer, large cell lung cancer, head and neck squamous cell carcinoma, head and neck cancer, brain cancer, neuroglioma, glioblastoma multiforme, neuroblastoma, central nervous system cancer, neuroendocrine tumor, throat cancer, pharyngeal squamous cell carcinoma, oral squamous cell carcinoma, nasopharyngeal cancer, esophageal cancer, thyroid cancer, medullary thyroid cancer, malignant pleural mesothelioma, breast cancer, triple-negative breast cancer, liver cancer, hepatobiliary cancer, pancreatic cancer, gastric cancer, gastrointestinal cancer, intestinal cancer, colorectal cancer, kidney cancer, clear cell renal cell carcinoma, ovarian cancer, endometrial cancer, cervical cancer, bladder cancer, prostate cancer, testicular cancer, adrenal cancer, glioblastoma, skin cancer, and melanoma; more preferably, the tumor or cancer is small cell lung cancer.

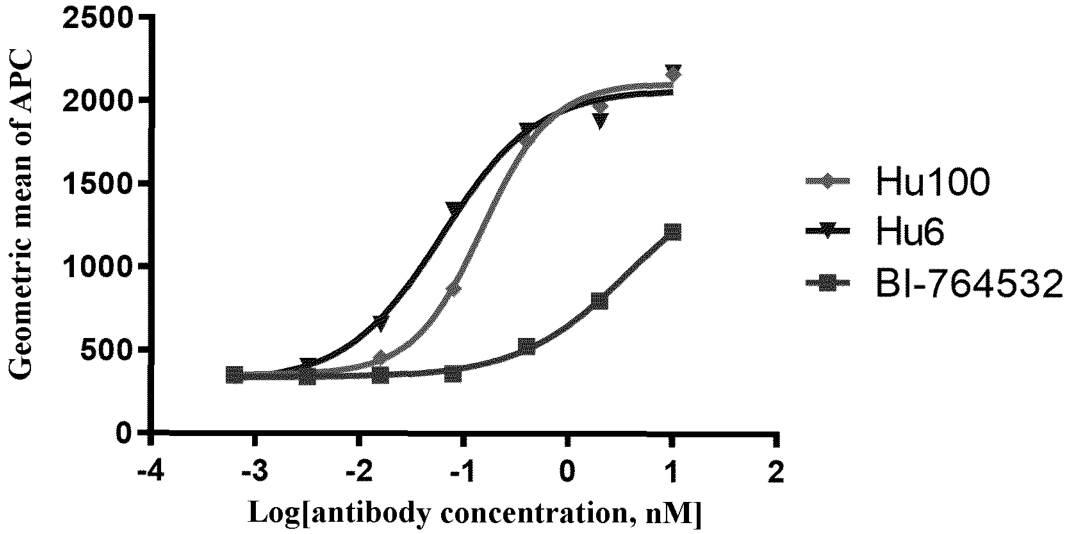
2500
2000
1500
1000
500
0
Geometric mean of APC
-4
-3
-2
-1
0
1
2
Log[antibody concentration, nM]
Hu100
Hu6
BI-764532

FIG. 1A

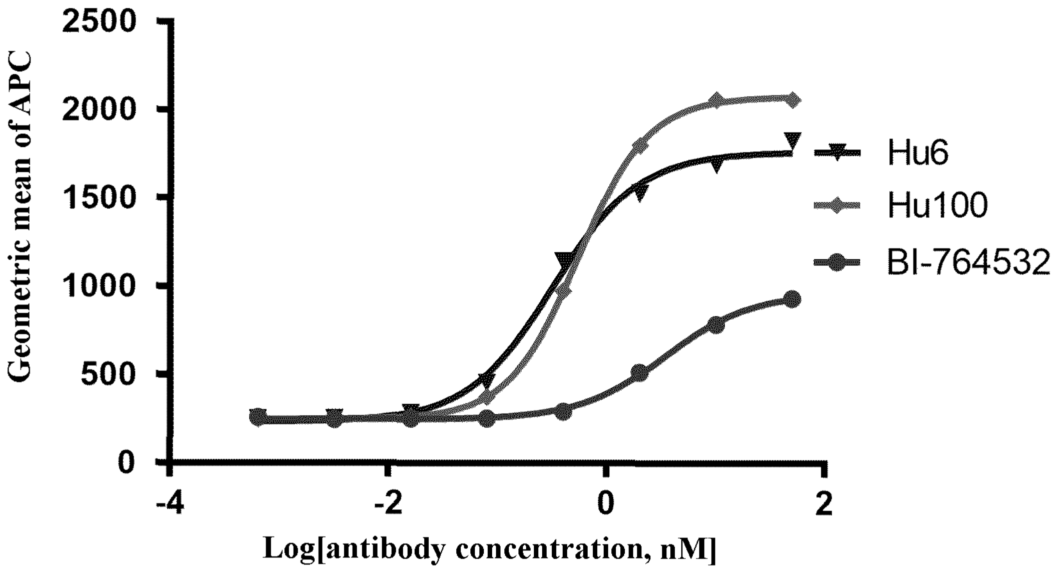
2500
2000
1500
1000
500
0
Geometric mean of APC
-4
-2
0
2
Log[antibody concentration, nM]
Hu6
Hu100
BI-764532

FIG. 1B

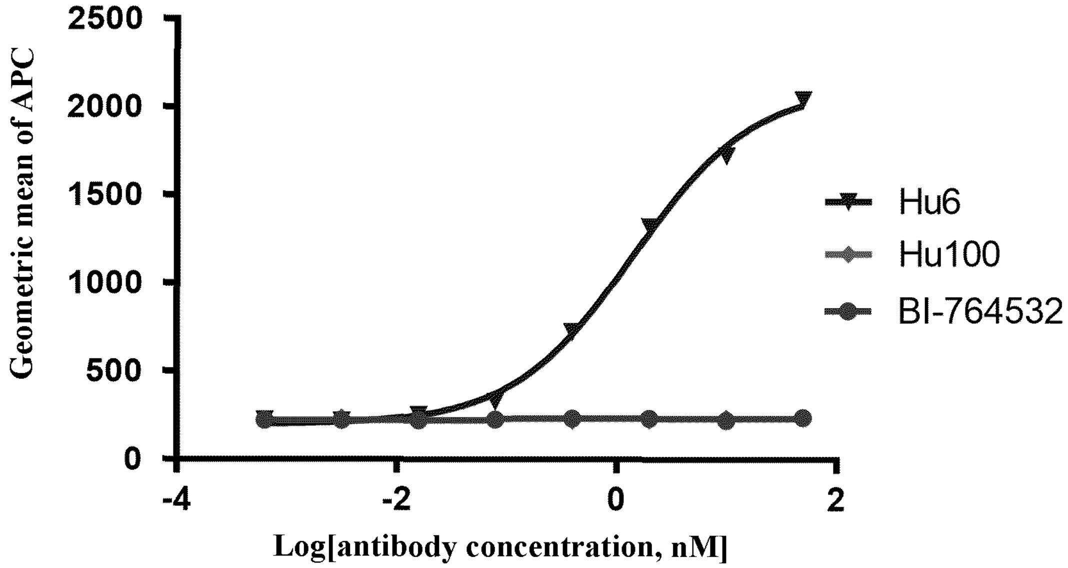
2500
2000
1500
1000
500
0
Geometric mean of APC
-4
-2
0
2
Log[antibody concentration, nM]
Hu6
Hu100
BI-764532

FIG. 1C

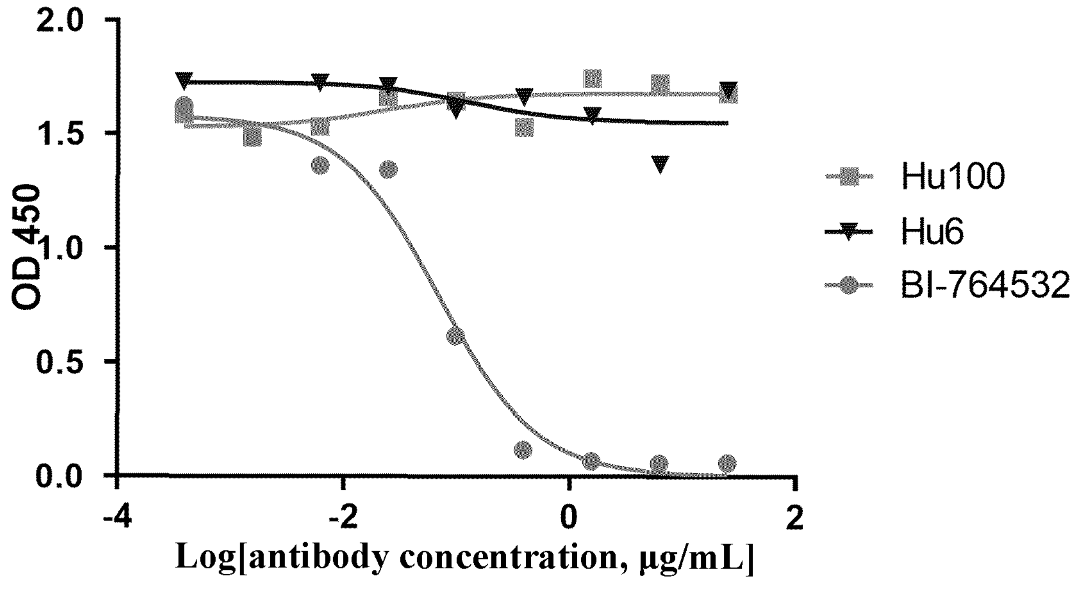
2.0
1.5
1.0
0.5
0.0
OD 450
-4
-2
0
2
Log[antibody concentration, µg/mL]
Hu100
Hu6
BI-764532

FIG. 2

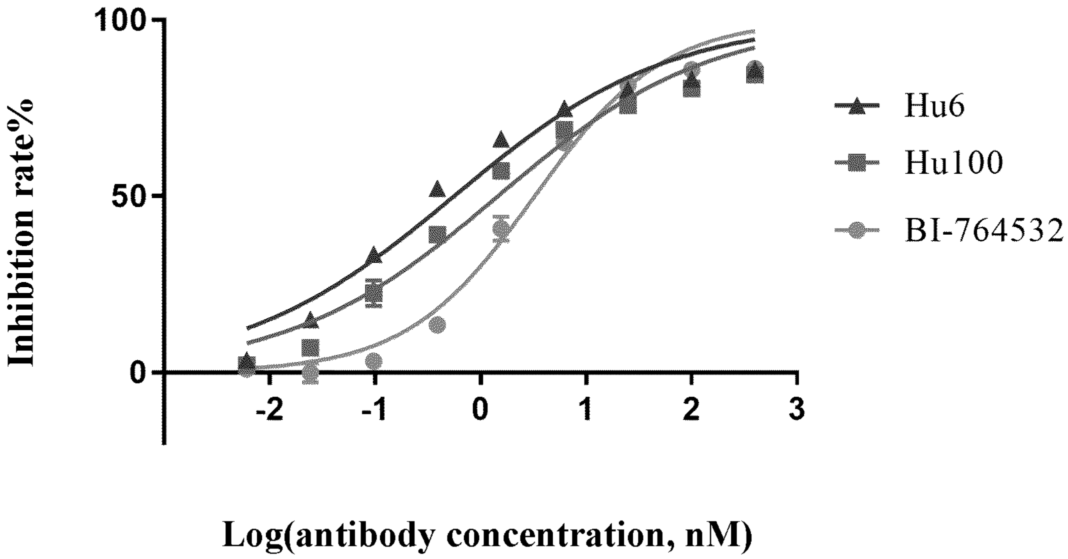
100
50
0
Inhibition rate%
-2
-1
0
1
2
3
Log(antibody concentration, nM)
Hu6
Hu100
BI-764532

FIG. 3

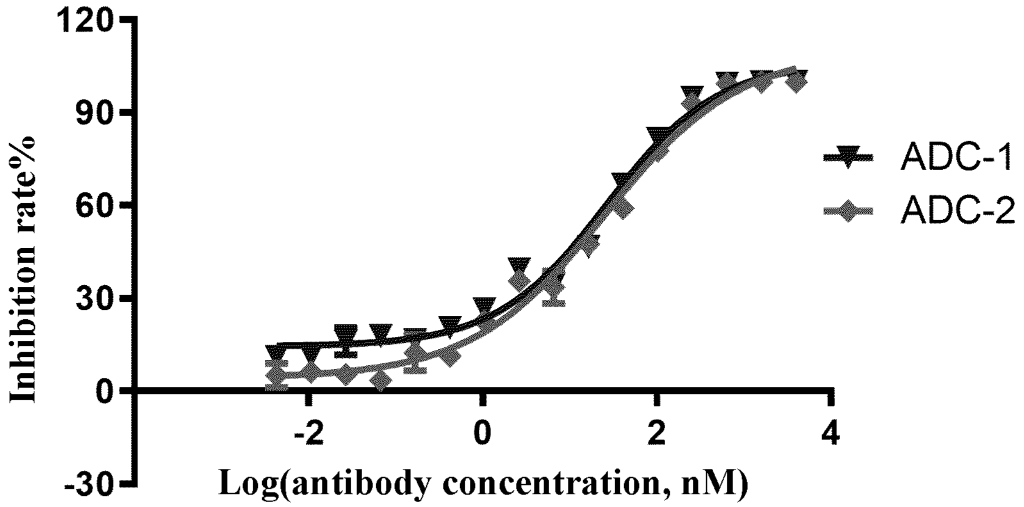
120
90
60
30
0
-30
Inhibition rate%
-2
0
2
4
Log(antibody concentration, nM)
ADC-1
ADC-2

FIG. 4A

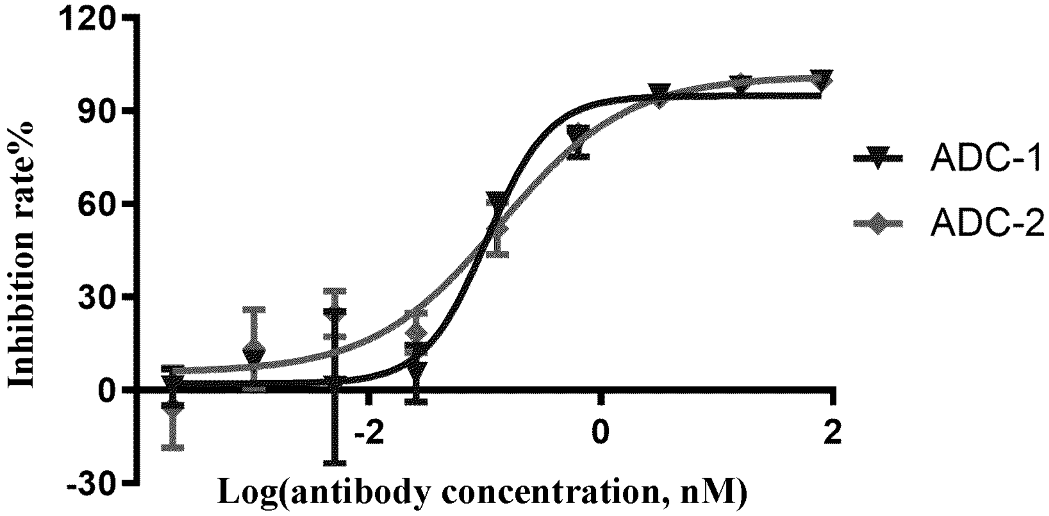
120
90
60
30
0
-30
Inhibition rate%
-2
0
2
Log(antibody concentration, nM)
ADC-1
ADC-2

FIG. 4B

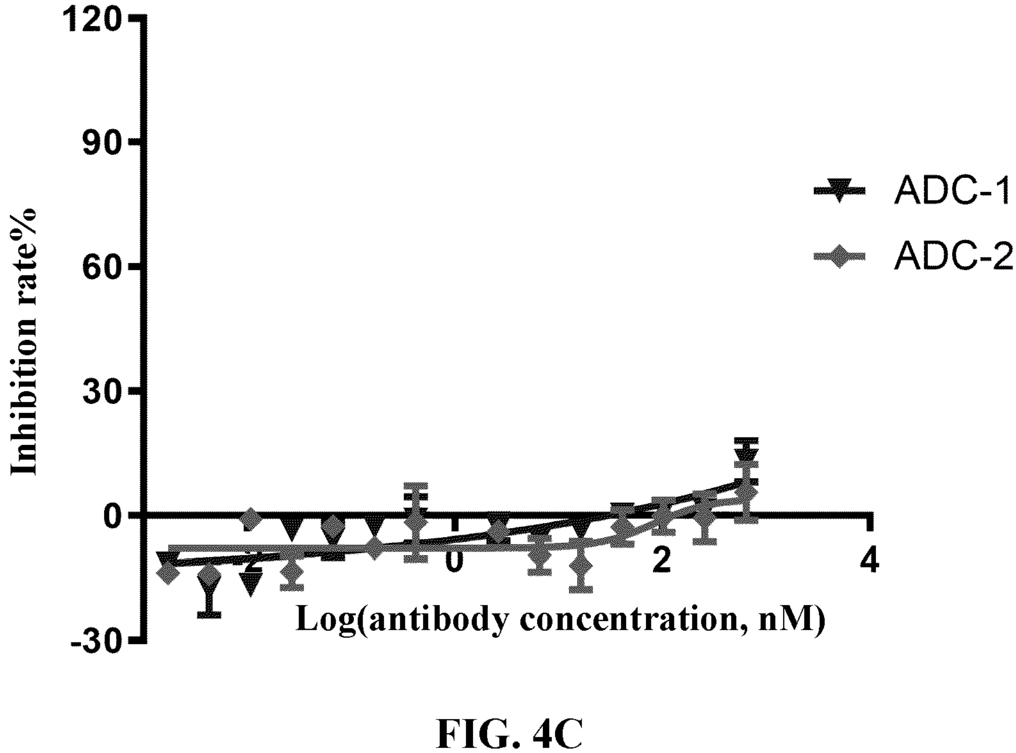
120
90
60
30
0
-30
Inhibition rate%
-2
0
2
4
Log(antibody concentration, nM)
ADC-1
ADC-2

FIG. 4C

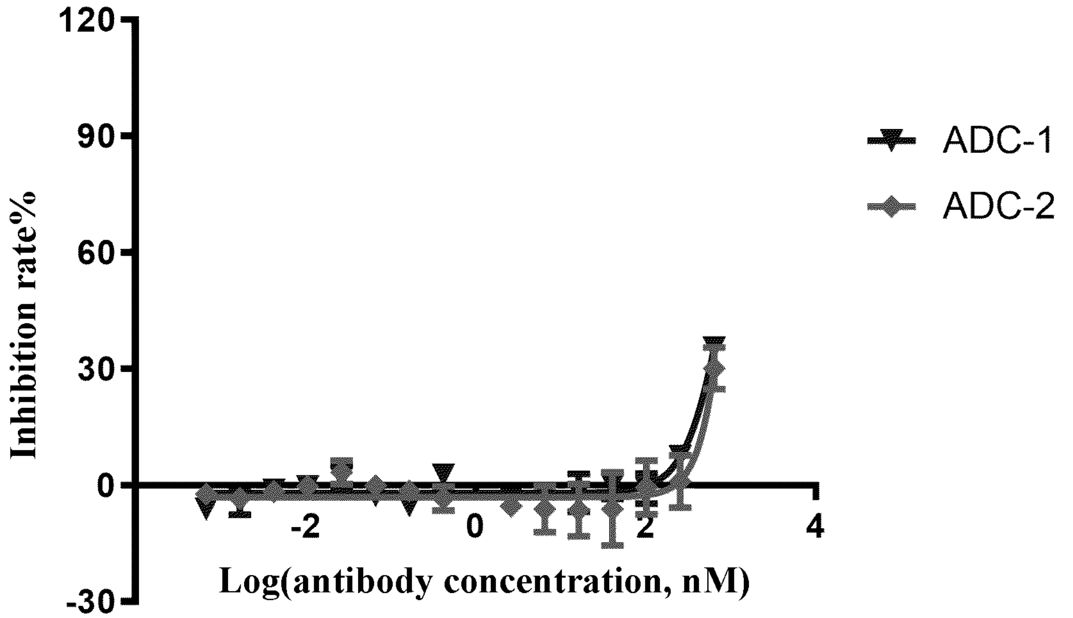
120
90
60
30
0
-30
Inhibition rate%
-2
0
2
4
Log(antibody concentration, nM)
ADC-1
ADC-2

FIG. 4D

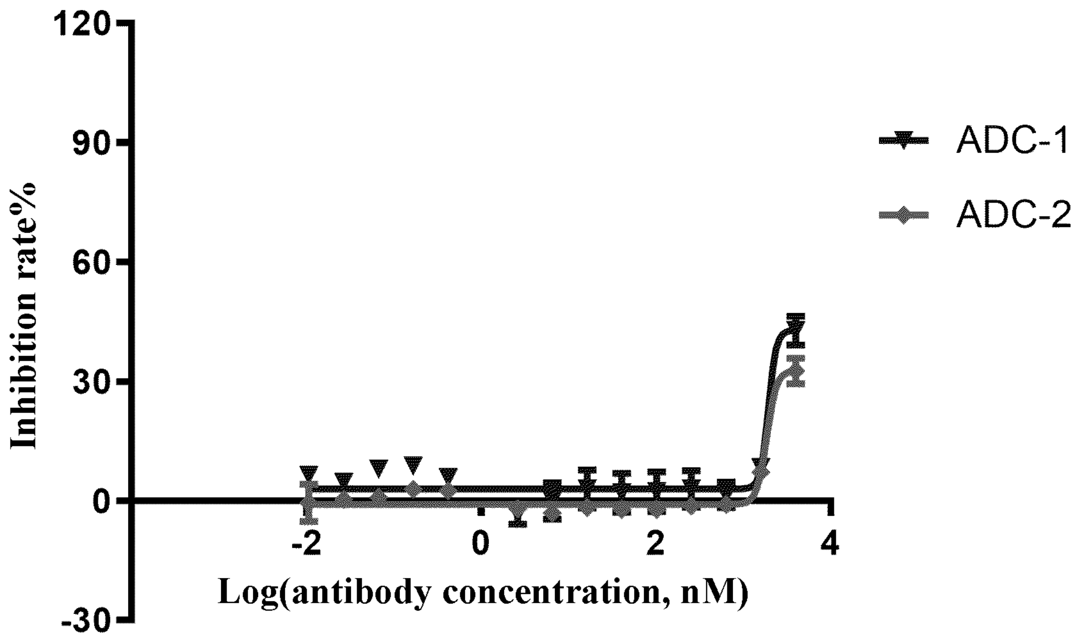
120
90
60
30
0
-30
Inhibition rate%
-2
0
2
4
Log(antibody concentration, nM)
ADC-1
ADC-2

FIG. 4E

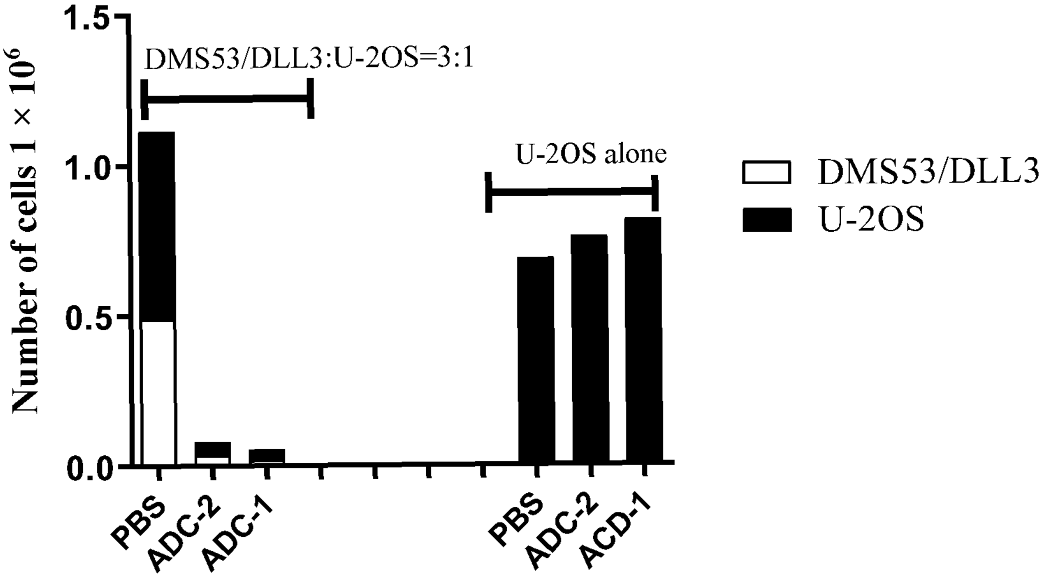
1.5
1.0
0.5
0.0
Number of cells 1 × 10^6
DMS53/DLL3:U-2OS=3:1
U-2OS alone
DMS53/DLL3
U-2OS
PBS
ADC-2
ADC-1
PBS
ADC-2
ACD-1

FIG. 5

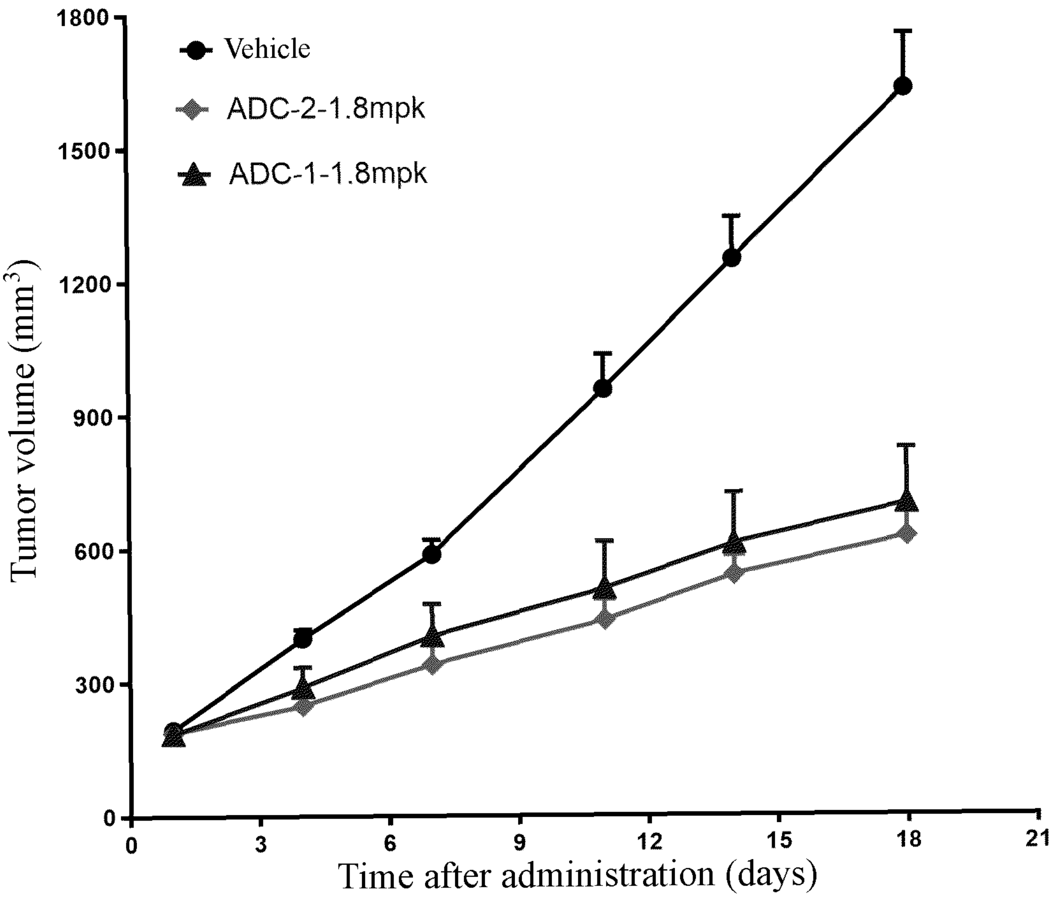
1800
1500
1200
900
600
300
0
Tumor volume (mm^3)
Vehicle
ADC-2-1.8mpk
ADC-1-1.8mpk
0
3
6
9
12
15
18
21
Time after administration (days)

FIG. 6

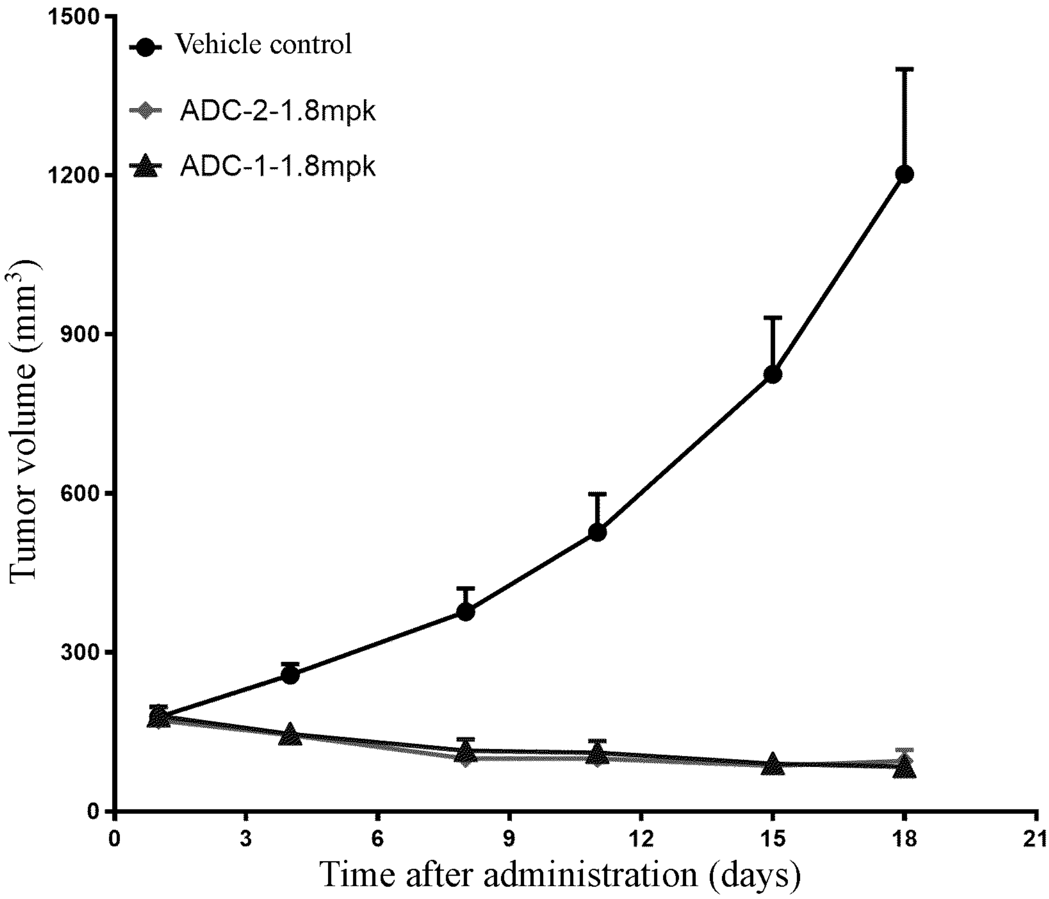
1500
1200
900
600
300
0
Tumor volume (mm3)
Vehicle control
ADC-2-1.8mpk
ADC-1-1.8mpk
0
3
6
9
12
15
18
21
Time after administration (days)

FIG. 7

## INTERNATIONAL SEARCH REPORT

International application No.
**PCT/CN2022/141269**

**A. CLASSIFICATION OF SUBJECT MATTER**

A61K39/395(2006.01)i;C07K16/28(2006.01)i

According to International Patent Classification (IPC) or to both national classification and IPC

**B. FIELDS SEARCHED**

Minimum documentation searched (classification system followed by classification symbols)

IPC:A61K; C07K

Documentation searched other than minimum documentation to the extent that such documents are included in the fields searched

Electronic data base consulted during the international search (name of data base and, where practicable, search terms used)

CNKI, CNTXT, DWPI, ENTXT, ELSEVIER, ISI Web of Knowledge, PubMed: 抗体, 抗原, 抗原结合片段, 单克隆抗体, 单抗, 嵌合抗体人源化抗体, antibody, McAb, monoclonal antibody, chimeric antibody, humanized antibody, CDR, DLL3, 海鞘素衍生物, 抗体-药物偶联物, antibody drug conjugate, ADC; 中国专利生物序列检索系统, Chinese Patent Biological Sequence Retrieval System, Genbank, EMBL, STN

**C. DOCUMENTS CONSIDERED TO BE RELEVANT**

| Category* | Citation of document, with indication, where appropriate, of the relevant passages | Relevant to claim No. |
|---|---|---|
| A | CN 112584860 A (PHANES THERAPEUTICS INC.) 30 March 2021 (2021-03-30)<br>claims 1-9, and specific embodiments, sequence listing | 1-17 |
| A | CN 108136015 A (ABBVIE STEMCENTRX LLC.) 08 June 2018 (2018-06-08)<br>description, paragraph 9, and specific embodiments, sequence listing | 1-17 |
| A | US 2019225685 A1 (ABBVIE STEMCENTRX LLC.) 25 July 2019 (2019-07-25)<br>specific embodiments, sequence listing | 1-17 |
| A | US 2021047399 A1 (PHANES THERAPEUTICS INC.) 18 February 2021 (2021-02-18)<br>entire document | 1-17 |
| A | CN 113521299 A (JIANGSU HENGRUI MEDICINE CO., LTD. et al.) 22 October 2021 (2021-10-22)<br>entire document | 1-17 |
| A | CN 113631191 A (DAIICHI SANKYO CO., LTD.) 09 November 2021 (2021-11-09)<br>entire document | 1-17 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **10 March 2023** | **15 March 2023** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| Facsimile No. **(86-10)62019451** | Telephone No. |

Form PCT/ISA/210 (second sheet) (July 2022)

**INTERNATIONAL SEARCH REPORT**

International application No.
**PCT/CN2022/141269**

**C. DOCUMENTS CONSIDERED TO BE RELEVANT**

| Category* | Citation of document, with indication, where appropriate, of the relevant passages | Relevant to claim No. |
|---|---|---|
| A | CN 105873612 A (STEMCENTRX INC.) 17 August 2016 (2016-08-17)<br>entire document | 1-17 |
| A | CN 106550593 A (ABBVIE STEMCENTRX LLC) 29 March 2017 (2017-03-29)<br>entire document | 1-17 |
| A | WO 2021218896 A1 (JIANGSU HENGRUI MEDICINE CO., LTD.) 04 November 2021 (2021-11-04)<br>entire document | 1-17 |

Form PCT/ISA/210 (second sheet) (July 2022)

**INTERNATIONAL SEARCH REPORT**

International application No.
**PCT/CN2022/141269**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   a. ☑ forming part of the international application as filed.
   b. ☐ furnished subsequent to the international filing date for the purposes of international search (Rule 13*ter*.1(a)),
      ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**
**Information on patent family members**

International application No.
**PCT/CN2022/141269**

| Patent document cited in search report | Publication date (day/month/year) | Patent family member(s) | Publication date (day/month/year) |
|---|---|---|---|
| CN 112584860 A | 30 March 2021 | WO 2019217145 A1 | 14 November 2019 |
| | | WO 2019217145 A8 | 23 January 2020 |
| | | SG 11202009772 PA | 27 November 2020 |
| | | KR 20210008367 A | 21 January 2021 |
| | | AU 2019267349 A1 | 29 October 2020 |
| | | US 2021047399 A1 | 18 February 2021 |
| | | EP 3790586 A1 | 17 March 2021 |
| | | EP 3790586 A4 | 19 January 2022 |
| | | BR 112020021280 A2 | 26 January 2021 |
| | | CA 3097193 A1 | 14 November 2019 |
| | | JP 2021528047 A | 21 October 2021 |
| CN 108136015 A | 08 June 2018 | PE 20181292 A1 | 07 August 2018 |
| | | PH 12018500380 A1 | 03 September 2018 |
| | | EA 201890530 A1 | 28 September 2018 |
| | | KR 20180041717 A | 24 April 2018 |
| | | EP 3337517 A2 | 27 June 2018 |
| | | EP 3337517 A4 | 17 April 2019 |
| | | TW 201718026 A | 01 June 2017 |
| | | CL 2018000458 A1 | 13 July 2018 |
| | | AU 2016308365 A1 | 15 March 2018 |
| | | IL 257645 A | 30 April 2018 |
| | | JP 2018529656 A | 11 October 2018 |
| | | US 2018243435 A1 | 30 August 2018 |
| | | CO 2018001624 A2 | 19 July 2018 |
| | | ZA 201801401 B | 28 August 2019 |
| | | CA 2996165 A1 | 23 February 2017 |
| | | UY 36862 A | 31 March 2017 |
| | | MX 2018002166 A | 12 September 2018 |
| | | WO 2017031458 A2 | 23 February 2017 |
| | | WO 2017031458 A3 | 06 April 2017 |
| | | CL 2018003758 A1 | 15 March 2019 |
| | | BR 112018003269 A2 | 25 September 2018 |
| | | HK 1257056 A1 | 11 October 2019 |
| US 2019225685 A1 | 25 July 2019 | None | |
| US 2021047399 A1 | 18 February 2021 | None | |
| CN 113521299 A | 22 October 2021 | None | |
| CN 113631191 A | 09 November 2021 | CA 3139180 A1 | 01 October 2020 |
| | | WO 2020196712 A1 | 01 October 2020 |
| | | US 2022168438 A1 | 02 June 2022 |
| | | JPWO 2020196712 A1 | 01 October 2020 |
| | | KR 20210143839 A | 29 November 2021 |
| | | EP 3949988 A1 | 09 February 2022 |
| | | EP 3949988 A4 | 16 November 2022 |
| | | TW 202102226 A | 16 January 2021 |
| CN 105873612 A | 17 August 2016 | IL 244254 A0 | 21 April 2016 |
| | | PH 12016500375 A1 | 02 May 2016 |
| | | CA 2922544 A1 | 05 March 2015 |
| | | CL 2016000468 A1 | 09 December 2016 |
| | | KR 20160047567 A | 02 May 2016 |

Form PCT/ISA/210 (patent family annex) (July 2022)

**INTERNATIONAL SEARCH REPORT**
**Information on patent family members**

International application No.
**PCT/CN2022/141269**

| Patent document cited in search report | Publication date (day/month/year) | Patent family member(s) | Publication date (day/month/year) |
|---|---|---|---|
| | | JP 2016531914 A | 13 October 2016 |
| | | US 2016175460 A1 | 23 June 2016 |
| | | CL 2017001916 A1 | 20 April 2018 |
| | | PE 20160209 A1 | 09 May 2016 |
| | | SG 11201601375 VA | 30 March 2016 |
| | | BR 112016004073 A2 | 17 October 2017 |
| | | BR 112016004073 A8 | 12 June 2018 |
| | | WO 2015031693 A1 | 05 March 2015 |
| | | RU 2016111137 A | 03 October 2017 |
| | | RU 2016111137 A3 | 12 July 2018 |
| | | CL 2018002620 A1 | 14 December 2018 |
| | | AU 2014312210 A1 | 07 April 2016 |
| | | MX 2016002545 A | 17 June 2016 |
| | | EP 3038659 A1 | 06 July 2016 |
| | | EP 3038659 A4 | 26 July 2017 |
| CN 106550593 A | 29 March 2017 | ECSP 16075472 A | 31 March 2017 |
| | | GT 201600168 A | 20 December 2018 |
| | | PH 12016501618 A1 | 06 February 2017 |
| | | WO 2015127407 A1 | 27 August 2015 |
| | | CA 2939941 A1 | 27 August 2015 |
| | | JP 2017514143 A | 01 June 2017 |
| | | EP 3107576 A1 | 28 December 2016 |
| | | EP 3107576 A4 | 06 September 2017 |
| | | MX 2016010677 A | 10 April 2017 |
| | | CR 20160437 A | 20 February 2017 |
| | | SG 11201606898 TA | 29 September 2016 |
| | | CL 2018001646 A1 | 05 October 2018 |
| | | US 2017137533 A1 | 18 May 2017 |
| | | US 10308721 B2 | 04 June 2019 |
| | | CL 2016002105 A1 | 31 March 2017 |
| | | PE 20161209 A1 | 10 November 2016 |
| | | AU 2015218633 A1 | 01 September 2016 |
| | | BR 112016018891 A2 | 10 October 2017 |
| | | IL 247248 A0 | 29 September 2016 |
| | | DOP 2016000214 A | 30 November 2016 |
| | | KR 20170008202 A | 23 January 2017 |
| | | EA 201691683 A1 | 28 April 2017 |
| WO 2021218896 A1 | 04 November 2021 | TW 202144369 A | 01 December 2021 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202111592539
- US 20050238649 A1
- US 5208020 A
- US 6248516 B
- US 6291158 B
- US 6582915 B
- US 6593081 B
- US 6172197 B
- US 20040110941 A
- EP 0368684 A
- US 6696245 B
- WO 04058821 A
- WO 04003019 A
- WO 03002609 A
- US 5821337 A
- US 7527791 B
- US 6982321 B
- US 7087409 B
- US 5731168 A
- US 7695936 B
- WO 2009089004 A
- US 20090182127 A
- WO 2019234220 A1
- WO 2021218896 A1
- CN 2021089838 W
- WO 2013106717 A1
- EP 2907824 B1


### Non-patent literature cited in the description


- *J. Biol. Chem.,* 1968, vol. 243, 3558
- **KABAT et al.** Sequences of Proteins of Immunological Interest. Public Health Service, National Institutes of Health, 1991
- **MARTIN.** Protein Sequence and Structure Analysis of Antibody Variable Domains [J. *ACR.,* 2001
- **LEFRANC, M.P. et al.** *Dev. Comp. Immunol.,* 2003, vol. 27, 55-77
- *Front Immunol.,* 16 October 2018, vol. 9, 2278
- *Prot. Sci.,* 2000, vol. 9, 487-496
- **CHARI et al.** *Cancer Research,* 1992, vol. 52, 127-131
- *Protein Engineering,* 1994, vol. 7, 697
- **ALMAGRO ; FRANSSON.** *Front. Biosci.,* 2008, vol. 13, 1619-1633
- **RIECHMANN et al.** *Nature,* 1988, vol. 332, 323-329
- **QUEEN et al.** *Proc. Nat'l Acad. Sci. USA,* 1989, vol. 86, 10029-10033
- **KASHMIRI et al.** *Methods,* 2005, vol. 36, 25-34
- **PADLAN.** *Mol. Immunol.,* 1991, vol. 28, 489-498
- **DALL' ACQUA et al.** *Methods,* 2005, vol. 36, 43-60
- **OSBOURN et al.** *Methods,* 2005, vol. 36, 61-68
- **KLIMKA et al.** *Br. J. Cancer,* 2000, vol. 83, 252-260
- **SIMS et al.** *J. Immunol.,* 1993, vol. 151, 2296
- **CARTER et al.** *Proc. Natl. Acad. Sci. USA,* 1992, vol. 89, 4285
- **PRESTA et al.** *J. Immunol,* 1993, vol. 151, 2623
- **BACA et al.** *J. Biol. Chem.,* 1997, vol. 272, 10678-10684
- **ROSOK et al.** *J. Biol. Chem.,* 1996, vol. 271, 22611-22618
- **CUNNINGHAM ; WELLS.** *Science,* 1989, vol. 244, 1081-1085
- **CARTER.** *J. Immunol. Methods,* 2001, vol. 248, 7-15
- **MARVIN ; ZHU.** *Acta Pharmacologica Sincia,* 2005, vol. 26 (6), 649-658
- **KONTERMANN.** *Acta Pharmacologica Sincia,* 2005, vol. 26, 1-9
- **RIDGWAY et al.** *Prot Eng,* 1996, vol. 9, 617-621
- **CARTER.** *J Immunol Meth,* 2001, vol. 248, 7-15
- **SAMBROOK et al.** Molecular Cloning: A Laboratory Manual. Cold Spring Harbor Laboratory Press
- **AUSUBEL et al.** Current Protocols in Molecular Biology. Greene Publishing Association, Wiley Interscience